# EUROPEAN PATENT SPECIFICATION

(11) **EP 0 648 409 B1**
(45) Date of publication and mention of the grant of the patent: **20.07.2005**
(21) Application number: 94201765.8
(22) Date of filing: 08.02.1991
(51) Int. Cl.: A01H 5/10, A01H 1/02

(54) **Safflower products**
Safflorprodukte
Produits de carthame

(30) Priority: 09.02.1990 US 478077; 09.02.1990 US 478079
(43) Date of publication of application: 19.04.1995
(62) Divisional of application: 91906095.4
(73) Proprietor: Bergman, Jerald W., Sydney, MT 59270 (US)
(72) Inventor: Bergman, Jerald W., Sydney, MT 59270 (US)
(74) Representative: Mallalieu, Catherine Louise

(56) References cited:
- EP-A- 0 044 723
- EP-A- 0 323 753
- FR-A- 2 617 675
- US-A- 3 186 854
- US-A- 3 529 974
- US-A- 4 627 192
- US-A- 5 436 386
- JOURN. AMERICAN OIL CHEMISTS SOC., vol.49, no.1, January 1972 pages 27 - 29, XP002004244 P.F.KNOWLES 'The plant genetist's contribution towards changing lipid and amino acid composition of safflower'
- R.W.ALLARD 'Principles of plant breeding' , JOHN WILEY & SONS , NEW YORK , XP0002004247 "Transgressive segregation" * page 472, line 28 - line 29 * * page 67, paragraph 2 - page 72, paragraph 1 *
- H.KUCKUCK ET AL 'Grundzüge der Pflanzenzüchtung' , WALTHER DE GRUYTER , BERLIN , XP0002004248 Paragraph 1.2 pages 20-49 : "Kombinationszüchtung" * page 21, line 1 - page 40, line 10 *
- J.M.POEHLMAN 'Breeding field crops' , VAN NOSTRAND REINHOLD , NEW YORK , XP0002004249 Chapter 4, pages 66-86 :"Quantitative inheritance in plant breeding:" * the whole document *
- PLANT BREEDING, vol.98, 1987, BERLIN pages 89 - 96, XP002004245 N.N.ROY & A.W.TARR 'Prospects for the development of rapeseed (B.napus L.) with improved linoleic and linolenic acid content'
- CANADIAN JOURNAL OF BOTANY, vol.43, 1965 pages 63 - 69, XP002004246 D.T.CANVIN 'The effect of temperature on the oil content and fatty acid composition of the oils from several oil seed crops'
- KIRK-OTHMER 'Encyclopedia of chemical technology' , JOHN WILEY & SONS , NEW YORK , XP0002004250 pages 795-831, chapter "Fats and fatty oils" * page 798; table 1 * * page 804 - page 805; table 3 * * page 812, paragraph 5 * * page 816, paragraph 4 - page 818, paragraph 2 *
- J.BOYELDIEU 'Produire des grains olagineux et protéagineux' , LAVOISIER TEC & DOC , PARIS FR , XP0002004251 chapter 3, "Le colza" pages 37-45 par.3.3 "Recherches en cours et perspectives" par.3.3.2 "Les nouveaux objectifs poursuivis en matière de composition de la graine de colza" * table 22 * Chapter VII, paragraph 1.6 "Le carthame (ou safran bâtard) Carthamus tinctorius" * page 210, line 32 - line 40 *
- CROP SCIENCE, vol.7, 1967 pages 417 - 422, XP000569026 D.M.YERMANOS ET AL 'Inheritance of quality and quantity of seed-oil in safflower (Carthamus tinctorius L.)'
- INDIAN JOURNAL OF GENETICS, vol.43, 1983 pages 68 - 75, XP000568495 V.RANGA RAO 'Combining ability for yield, percent oil and related components in safflower'
- THEORETICAL AND APPLIED GENETICS, vol.50, 1977 pages 185 - 191, XP000569021 V.R.RAO & M.RAMACHANDRAM 'An analysis of components in of yield and oil in Safflower (Carthamus tinctorius L.)'
- PROCEEDINGS 2ND INT. SAFFLOWER CONFERENCE 1989, vol.2, 1989, HYDERABAD INDIA pages 169 - 172, XP000568609 A.B.HILL 'Hybrid Safflower breeding'

## Description

Safflower, Carthamus tinctorius L. , is a highly branched, herbaceous, thistle-like annual varying in height from 30 to 150 cm. Safflower has been grown for centuries in small plots over a vast area from China to the Mediterranean region, and along the Nile Valley as far as Ethiopia. Since 1945 commercial production has taken place in the United States, Russia, Australia and Mexico. Safflower has remained a minor oilseed in terms of total production and world trade. The crop can be grown under full mechanization so that the production costs compare favorably with other oilseeds. In addition, safflower is suitable for growth in drier regions and thus provides a profitable alternative in such areas. Altering the oil content, increasing protein content or increasing seed yield are current objectives in safflower breeding programs.

Safflower oil is comprised primarily of palmitic, stearic, oleic and linoleic acids, with the vast majority comprised of oleic and linoleic acids. Two different types of oil can be produced, depending on the genetics of the particular safflower line. One type is high in linoleic acid and the other is high in oleic acid. A third experimental type of safflower oil contains about equal amounts of oleic and linoleic acids. A high oleic acid concentration of safflower oil is desirable when its end use is in the food industry. A high linoleic acid safflower oil is desirable when its end use is in resin and paint formulations. In all instances, it is desirable to produce a safflower oil which is low in the saturated fatty acids, i.e., palmitic and stearic acids.

Three allelic genes, Ol, ol' and ol, are the primary genetic determinants of the proportions of oleic and linoleic acids. The genotype olol has 72-80% oleic acid in the oil, and the genotype OlOl has 72-80% linoleic acid. The genotype ol'ol' has about 45% of each fatty acid. It has been discovered that additional minor genes may be involved in determining the fatty acid content of a particular safflower variety. Knowles, P.F., J. Am. Oil Chem. Soc. 49, 27 (1972) noted that such modifying genes may make it possible to raise the oleic acid contant above 80%, but probably not above 85%.

Knowles, supra, further noted that although high oleic and high linoleic safflower varieties are temperature stable, there is a slight variation in fatty acid content depending on the growth temperature of the variety. This variation is illustrated in Table 1.

**TABLE 1**

| Fatty Acid Composition of Safflower Types Grown Under Different Temperatures | | | | |
|---|---|---|---|---|
| | | | Fatty Acid (%) | |
| Oil Type | Temp.¹ | Sat.² | Oleic | Linoleic |
| high linoleic | Low | 8.4 | 9.1 | 82.4 |
| | Int. | 7.8 | 14.0 | 78.2 |
| | High | 9.3 | 15.2 | 75.5 |
| high oleic | Low | 6.3 | 69.7 | 24.0 |
| | Int. | 7.0 | 71.6 | 21.4 |
| | High | 8.3 | 77.4 | 14.3 |

| | | | | |
|---|---|---|---|---|
| ¹ Day and night temperatures, respectively: low = 18.4°C and 25.6°C; intermediate = 26.7°C and 23.9°C; and high = 29.4°C and 26.1°C. | | | | |
| ² Saturates = palmitic and stearic acids. | | | | |

Thus, a high oleic acid safflower variety grown in Montana, for example, having a mean growing temperature of about 70°F, would be expected to have a slightly lower production of oleic acid than the same variety grown in California. Conversely, the variety grown in California would be expected to have a higher level of oleic acid than the value reported for the variety in Montana.

### SUMMARY OF THE INVENTION

According to one aspect of the present invention there is a safflower seed or oil obtained therefrom, said seed or oil containing 3.7 to 6.2 wt.% saturated fatty acid content, and an oleic acid content of at least 81.7%.

According to another aspect of the present invention there is provided a safflower plant which produces a seed according to the present invention.

Preferably the oleic acid content of the oil ranges from 83.4% to 90%.

Preferably the linoleic acid content ranges from 86% to 91%.

Preferably the linoleic acid content is at least 83%.

Preferably the ratio of the amount of saturated fatty acid in said oil to the amount of oleic acid is less than 0.08.

Preferably the oleic acid content is at least 81.9 %.

Preferably the ratio of saturated fatty acids to oleic acid is less than about 0.07.

Preferably the safflower seed or oil contains 4.7 to 5.8% of saturated fatty acids, and an oleic acid content of 83.4% to 86.1%.

Preferably said linoleic acid content is between 80% and 91 %.

Preferably the safflower oil has been deodorized.

Preferably the deodorized safflower oil is bleached.

Preferably the safflower oil has an AOM value of greater than about 60 hours.

Preferably said oil contains an oleic acid content of 83.4% to 86.1% and a linoleic acid content of 7.4% to 10.4%.

### DETAILED DESCRIPTION OF THE INVENTION

In order to provide an understanding of several of the terms used in the specification and claims, the following definitions are provided.

Homogeneous assemblage - The term homogeneous assemblage is intended to refer a group of seeds which are homogeneous for a given characteristic. This term is intended to include any seeds which are homogeneous as determined by any 1000 seeds from one acre of safflower.

Mean temperature - The mean temperature is the mean of the recorded daily temperatures during a specified period, such as a month or a developmental stage of safflower growth. The mean temperature (F) for the months of May through October from 1949 through the 1988-89 crop year is shown in Table 2.

**TABLE 2**

| Mean Temperatures (°F) for 1949-89 at the Eastern Agricultural Research Center, Sidney Montana | | | |
|---|---|---|---|
| Month | Mean Temp. | Month | Mean Temp. |
| May | 55.5 | Aug. | 67.8 |
| June | 64.2 | Sept. | 56.4 |
| July | 69.7 | Oct. | 45.6 |

Saturated Fatty acids - As used herein, the term saturated fatty acids is intended to include only palmitic acid and stearic acid.

Seed development or production - For the purposes of this invention, the seed development, seed production or production of seed is the time during safflower growth from pollination through seed maturation. In Montana, this time is usually from about the third week of July through the middle of August.

Series of crosses - A series of crosses is used herein to refer to the breeding technique of transgressive segregation in which multiple, single crosses are made to produce female and male parents.

As noted above, the content of various fatty acids such as oleic and linoleic, which is characteristic of oil from a given safflower seed sample, is commonly expressed as a percentage of the total fatty acid fraction in the oil. This convention will be followed in the description given below unless otherwise indicated. Dimensionless ratios of saturated fatty acid content to either oleic or linoleic acid content, which are also mentioned below, are calculated by dividing the saturated fatty acid percentage by either the oleic or the linoleic acid percentage, as applicable. As also noted above, the specific level of oleic acid, linoleic acid and saturated fatty acids is dependent upon the temperature during seed development. Unless indicated otherwise, the percentages shown for the contents of these fatty acids are for safflower grown in Montana and at a mean temperature of about 70°F (21°C) for seed development or production. As used herein, about 70°F is intended to include mean temperatures of 70°F, plus or minus 2°F.

The present invention has a low level of saturated fatty acid in the oil. The fatty acid content in a ratio to the oleic acid is expressed as the combined weight of the two saturated fatty acids divided by the weight of the oleic acid. The measurement of the ratio is done using the extracted fatty acids, however, the oils may also be used because there is little difference in the methods and little difference in the weight of the oil versus the fatty acids.

The ratios for the saturated fatty acids to the oleic acid are thus preferably less than about 0.08, more preferably less than about 0.07, yet more preferably less than about 0.06.

For purposes of the present description, the terms "variety", "cultivar" and "line" are used synonymously to refer to a group of plants which are uniform in their traits such that there is relatively minor variation within the group and such variation can be characterized. The decreased variation within this group has generally (although not exclusively) resulted from several generations of self-pollination or selfing. A variety is considered true breeding for a particular trait if it is genetically homozygous for that trait to the extent that when the variety is self-pollinated, a significant amount of independent segregation of the trait among progeny is not observed.

Safflower is primarily self-pollinating, with some cross-pollination possible through the interaction of insects, primarily bees. Safflower pollen is too heavy for wind-aided cross-pollination to occur. Consequently, in arid areas, such as in Montana, there is little cross-pollination that occurs because of a low bee population. Safflower breeding, as described herein, is performed using conventional techniques to develop the high oleic acid and high linoleic acid varieties described and claimed.

The safflower of the present invention reproducibly expresses a high oleic acid trait or a high linoleic acid trait, and conversely, a low saturated fatty acid trait, against a phenotypic background of agronomical traits which is sufficiently consistent for commercial applications. In contrast, the original breeding material produced seeds having either a lower oleic acid content or a lower linoleic acid content, and conversely, a higher saturated fatty acid content against a similar phenotypic background of agronomic traits. In accordance with one embodiment of the present invention, safflower seed is produced which contains in excess of 80%, preferably in excess of 81.7%, preferably 81.9%, and most preferably in excess of 83.4%, up to 90%, oleic acid when produced at a mean seed development temperature of about 70°F. In a second embodiment of the present invention, safflower seed is produced which contains in excess of 80%, preferably in excess of 83.0%, and most preferably in excess of 86.0%, up to 91%, linoleic acid when produced at a mean seed development temperature of about 70°F.

To produce the safflower varieties of the present invention, an extensive breeding program is used in which many parental varieties are utilized to pursue transgressive segregation. In order to accumulate all minor genes for various traits, it is necessary to keep the selection pressure low. In this breeding program varieties having the major ol gene (for high oleic) or the major Ol gene (for high linoleic) are utilized and many crosses are made to accumulate minor, favorable genes into one genotype. Any genotype having desirable characteristics may be utilized in this breeding program. The intermediate lines which are produced are not crossed with any of the original breeding material. The genotypes are then selfed and selected for the specific level of oleic acid or linoleic acid desired.

This general breeding procedure has given attention to the modifications in fatty acid composition and other desirable traits produced by genes having small effects on such traits. Since 1973, cultivars have been crossed and intercrossed on an annual basis to permit selection for transgressive segregation of superior lines for the desired traits, including oil quality, Alternaria leaf spot resistance, Pseudomonas bacterial blight resistance, oil content, drought resistance and other quantitative traits. Each year, selections have been made for traits or characteristics that are expressed. Approximately the top 50% of the breeding material is saved from each cycle of selection for intercrossing for the next cycle of selection and intercrossing. In this manner genes with small effects for desirable traits such as fatty acid composition, disease resistance, drought tolerance, etc., are incorporated into the continually improving germplasm.

Newly developed cultivars with the additional favorable genes are incorporated into the germplasm base by intercrossing with superior breeding lines. During the period from 1973 to 1982, selection was practiced to improve seed oil content, Alternaria leaf spot resistance, Pseudomonas bacterial blight resistance, Sclerotina head rot resistance, posthoused seed dormancy, drought resistance and other expressed and desirable traits. For example, seed oil content of the breeding material was improved approximately 1% each year, from 33% to 42% during the period from 1974 to 1982. Selection for improved fatty acid composition, either oleic acid content or linoleic acid content, began in 1985. Selection for transgressive segregation of superior lines for fatty acid composition has been practiced since 1985. Oil types have been obtained which are lower in saturated fatty acids with, corresponding higher levels of oleic acid or linoleic acid than previously reported.

The top 302 lines for oleic acid productions were selected from a group of 6000 crosses which were made in 1985. The 1985 lines produced from about 70.9% to 80.8% oleic acid in the seed. The linoleic acid content ranged from 12.4% to 22.1% and the saturated fatty acid content ranged from 4.7% to 6.8%. The ratio of saturated fatty acids to oleic acid ranged from 0.059 to 0.088, with two lines at 0.093 and 0.095. Only one of the 302 lines was above 80%.

These 302 lines were selfed in 1986 and the seed analyzed for oleic acid production. A total of 215 lines were selected after the 1986 season which produced seeds having an oleic acid content greater than 80%. The range in oleic acid content of these lines was 80% to 84.7%. The linoleic acid content ranged from 8.4% to 13.6% and the saturated fatty acid content ranged from 3.9% to 7.5%. The ratio of saturated fatty acids to oleic acid ranged from 0.047 to 0.086, with one line at 0.093. Twenty-four of these lines produced seeds having an oleic acid content of greater than 83%.

These 215 lines were selfed in 1987 and the seed analyzed for oleic acid production. A total of 303 lines were selected after the 1987 season which produced seeds having oleic acid content greater than 80%. The range in oleic acid content of these lines was 81.9% to 85.9%. The linoleic acid content ranged from 7.2% to 11.8% and the saturated fatty acid content ranged from 3.7% to 6.2%. The ratio of saturated fatty acids to oleic acid ranged from 0.044 to 0.076. Forty-eight of these lines produced seeds having an oleic acid content of greater than 84%.

These 303 lines were selfed in 1988 and the seed analyzed for oleic acid production. A total of 352 lines were selected after the 1988 season which produced seeds having oleic acid content greater than 80%. The range in oleic acid content of these lines was 83.4% to 86.1%. The linoleic acid content ranged from 7.4% to 10.4% and the saturated fatty acid content ranged from 4.7% to 5.8%, with six lines up to 6.5%. The ratio of saturated fatty acids to oleic acid ranged from 0.055 to 0.070, with three lines up to 0.075. Twenty-two of these lines produced seeds having an oleic acid content of greater than 85%.

These 352 lines were selfed in 1989 and the seed analyzed for oleic acid production. Slight changes were seen in the oleic acid content of the progeny, with some lines approaching 87%. In order to increase the oleic acid content to greater than 87%, and up to 90%, the previously obtained lines are intercrossed to derive new lines. These new lines produce seed having an oleic acid content of up to 90%.

The safflower varieties produced in accordance with the present invention share characteristics common among commercial safflower varieties. Several of these characteristics will be set forth in more detail below.

### EXAMPLES

The following examples are provided to further illustrate the present invention and are not intended to limit the invention beyond the limitations set forth in the appended claims.

### EXAMPLE 1

### Determination of Fatty Acid Content

The fatty acid composition of safflower seeds developed in the breeding program was determined by gas-liquid chromatography (GLC) in accordance with the procedures described in Hougen, F.W. and Bodo, V., J. Am. Oil Chem. Soc. 50, 230 (1973) and Daun, J.K. and Mazur, P.B.,. J. Am. Oil Chem. Soc. 60, 1751 (1983); and, with techniques developed by D.I. McGregor, Agriculture Canada Research Station, Saskatoon, Saskatchewan, Canada for rapeseed and mustard.

### A) Analysis of 12 Gram Samples

A 12 gram sample of the safflower seed of interest was dried at 45-50°C for at least 4 hours. The sample was then ground in the presence of 40 ml of hexane using a tissue homogenizer for approximately 45-60 seconds. The container of the homogenate was then capped and allowed to settle for approximately 15 minutes. A 0.5 ml aliquot was then pipetted in a 13x100 mm glass test tube. To this tube was added 0.5 ml of benzene followed by 2 ml of sodium methoxide in methanol at a concentration of 2 g sodium methoxide in 100 ml methanol. The tube was capped and allowed to stand for 10 minutes. After standing, 3 drops of Bromophenol Blue in ethanol was added followed by drops of 1N HCl until the blue solution turned yellow. The excess HCl was then neutralized with 1N sodium carbonate until one drop turned the solution blue. Deionized water was then added to make sure separation of the two phases was complete. Approximately 1 ml of the upper phase was then placed in a septum sealed vial and analyzed by GLC.

### B) Analysis of 1 to 5 Seed Samples

The individual seeds were cut in half with a razor blade. The halves were then placed in a glass test tube with a small amount of hexane, approximately 1-2 ml. The pieces were then mashed and stirred for around 30 seconds. with a glass rod and allowed to sit with the tube capped for 15 minutes. A 0.5 ml sample of the hexane extractant was then treated in the manner described above. The fatty acid analysis of half a seed enabled the planting of the remaining half for further breeding.

### C) Gas-Liquid Chromatography

The GLC analyses were accomplished using a 5890 Hewlett-Packard instrument equipped with a flame ionization detector and a Hewlett-Packard 3392 integrator. The column used was a J&W Scientific fused silica capillary column coated with DB-225+ (film thickness of 0.25 microns, column dimensions of 15 meters x 0.318 mm, catalog no. 1232212. The operating conditions of the GLC included an injector temperature of 225°C and a detector temperature of 300°C. Column flow was 1.6 cc/min. of helium. Each chromatographic run was temperature programmed to begin at 170°C and remain at that temperature for 2.0 minutes. The temperature was then increased to 190°C at a rate of 3°C/min. The temperature was then held at 190°C for 1.5 minutes. The column temperature was increased to 220°C at a rate of 2°C/min. The temperature was held at 220°C for 3 minutes. After this period of time, the chromatograph was completed and the column prepared for the next run.

### Example 2

### Characterization of Parent Lines

The following is a description of several of the parent safflower lines utilized in the breeding program. Any parent material could be utilized as long as it met the criteria described above, namely the presence of desirable traits such as oil content, blight resistance and the like. These parent materials are readily available from public institutions or for sale by commercial organizations.

'Frio' safflower developed by the Arizona Agricultural Experiment Station (AES) and the Agricultural Research Service - U.S. Department of Agriculture (ARS-USDA) was released in 1965 and has the genotype OlOl that produces seed high in linoleic acid.

'Sidney Selection 87-14-6' is a 1965 selection for Alternaria leaf spot resistance made from the 1964 bulk composite of 555 safflower introductions of the 1960 world safflower collection.

'79AZ9543-1' is an experimental white flowered oleic safflower line obtained in 1980 from the Arizona AES.

'S-304' was developed by Seedtec International, Woodland California and has the genotype ol'ol' that produces an oil with approximately equal amount of linoleic and oleic acids, but the relative amounts of each fatty acid are greatly influenced by growing temperature.

'S-208' was developed by Seedtec International and is a normal white hull seed variety and has the genotype OlOl that produces seed high in linoleic acid. A white flowered individual plant from S-208 was used as one of the parental lines.

'UC-1' was developed in 1966 by the California AES and was the first commercial oleic safflower cultivar having the genotype olol that produces seed higher in oleic acid content.

'Oleic Leed' is a high oleic acid content cultivar released in 1974 by the ARS-USDA and the California AES. The oleic acid olol gene was obtained from 'UC-l' crossed to 'Leed' and backcrossed to 'Leed' two times.

'Th-5' is a parental line developed by the ARS-USDA and the Utah AES in 1970 and carries the thth gene pair which governs reduced pericarp. The line was released for use as a female parent in hybrid development programs.

'Carthamus nitidus' is a related species to C. tinctorius L. with 12 chromosome pairs.

'ol41-1' is an individual plant selection with extremely reduced hull or partial hull, with an iodine value of 141 obtained from A.L. Urie in 1974. This plant was obtained from a line segregating for linoleic gene Ol and oleic gene ol.

'US-10' was developed by the ARS-USDA and the California AES in 1959 and has the genotype OlOl that produces seed high in linoleic acid. A white flowered individual plant from 'US-10' was used as one of the parental lines.

Examples 3-9 are directed to the development of high oleic acid varieties.

### Example 3

### 1985 Breeding Program and Results

The top 302 lines for oleic acid production were selected from a group of 6000 crosses which were made in 1985. The 1985 lines produced from about 70.9% to 80.8% oleic acid in the seed. The linoleic acid content ranged from 12.4% to 22.1% and the saturated fatty acid content ranged from 4.7% to 6.8%. The ratio of saturated fatty acids to oleic acid ranged from 0.059 to 0.088, with two lines at 0.093 and 0.095. Only one of the 302 lines was above 80%. The mean temperatures for July and August were 71.4°F and 66.3°F, respectively. The results are shown in Table 3.

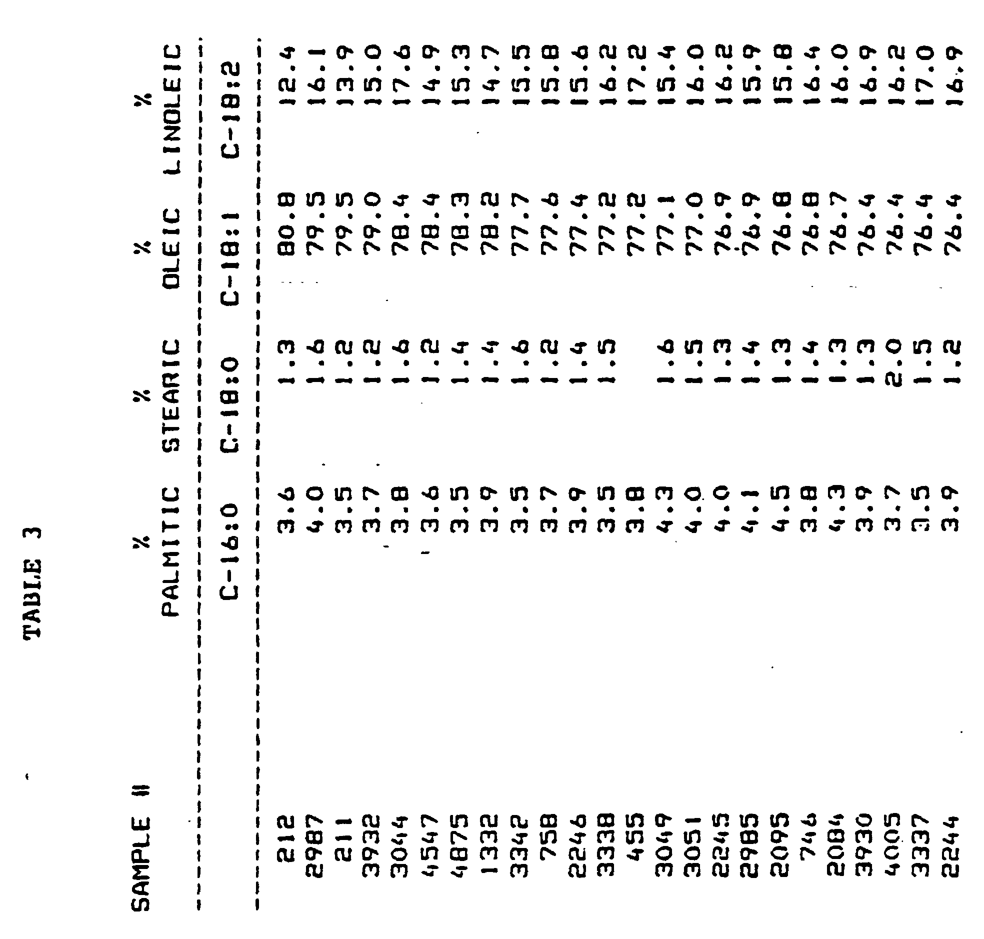

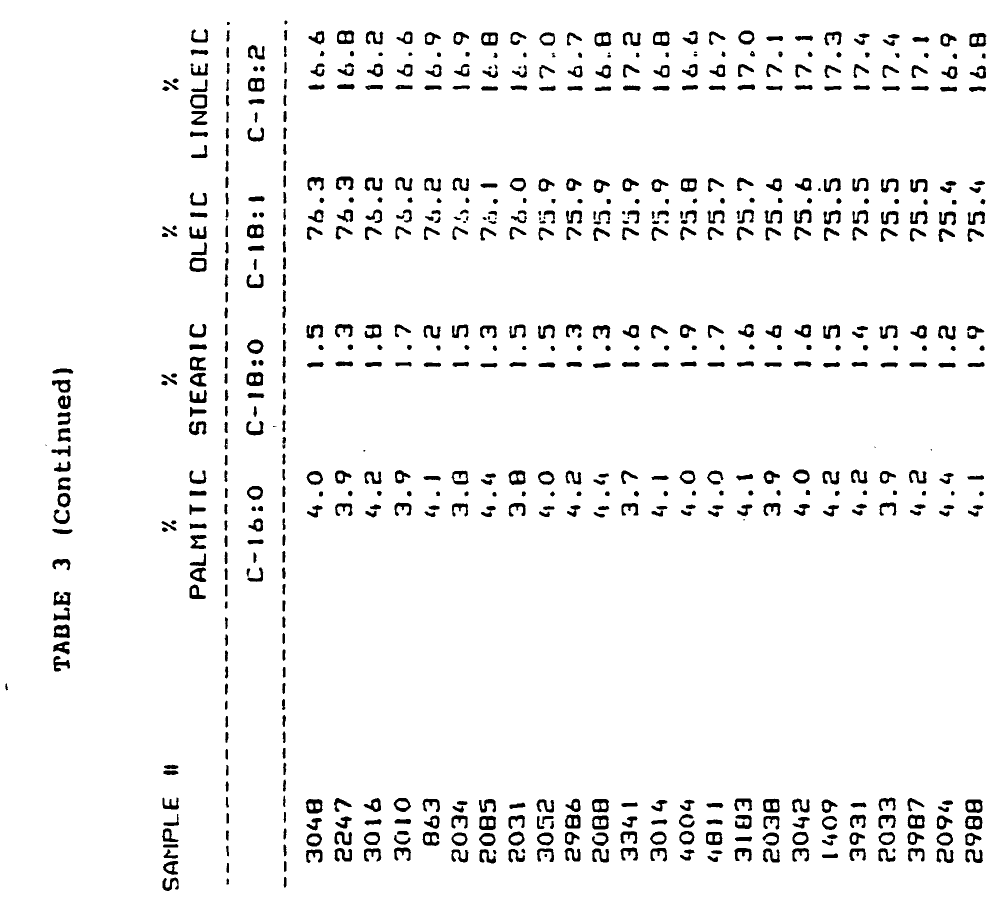

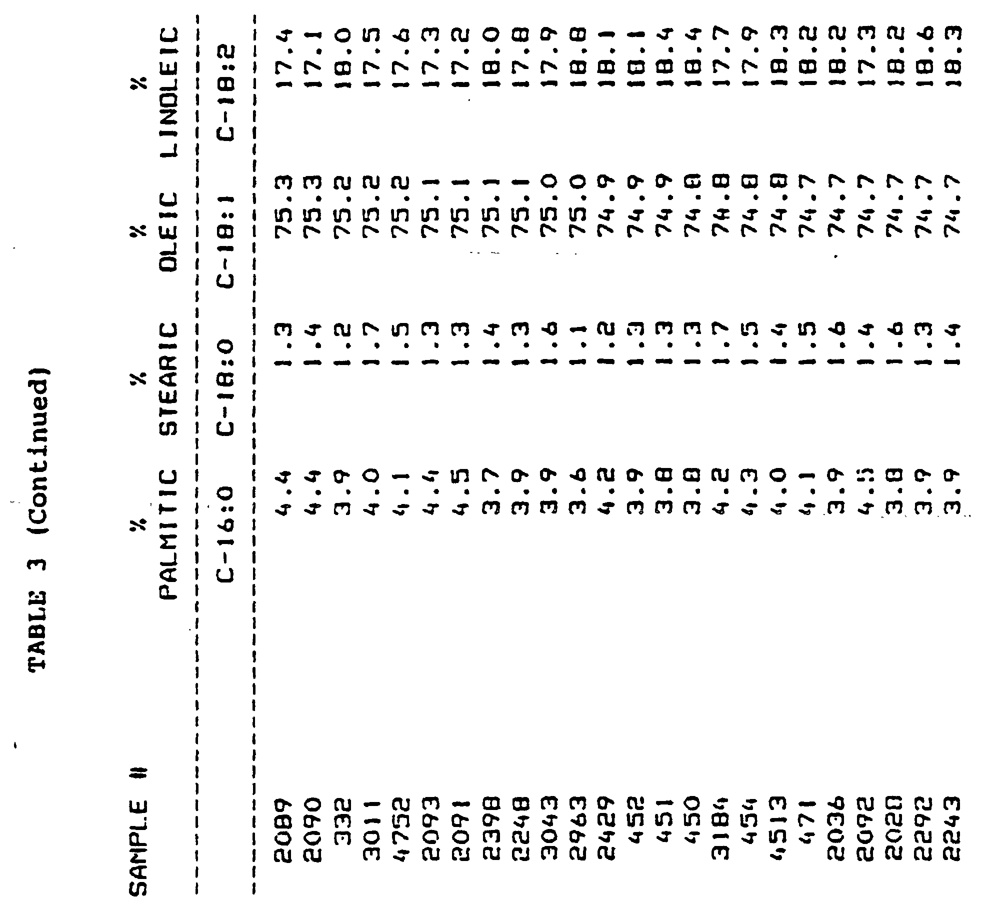

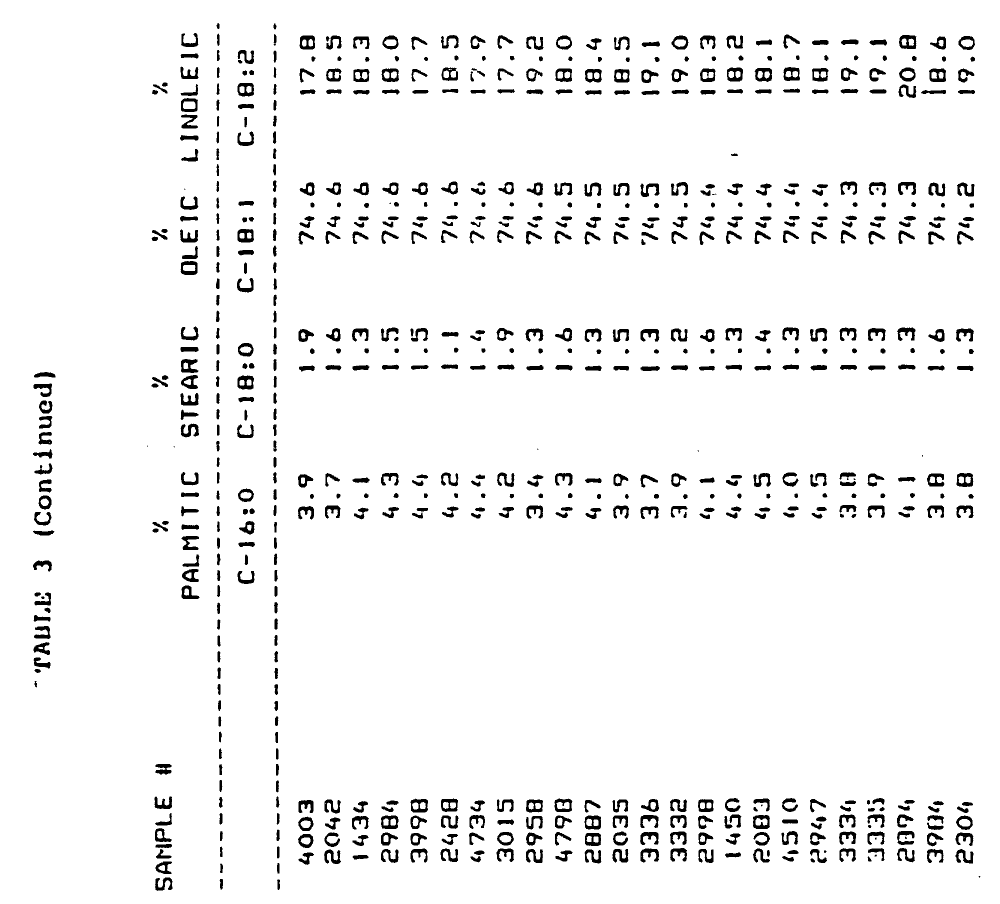

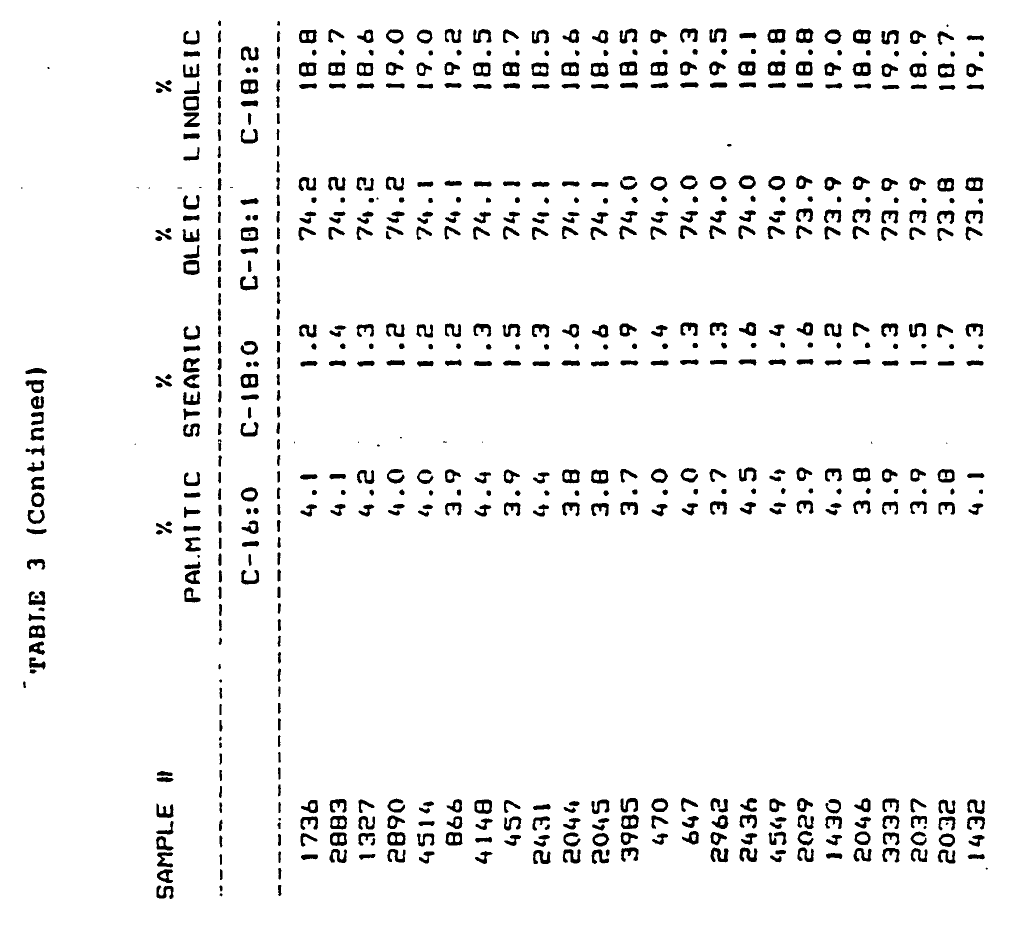

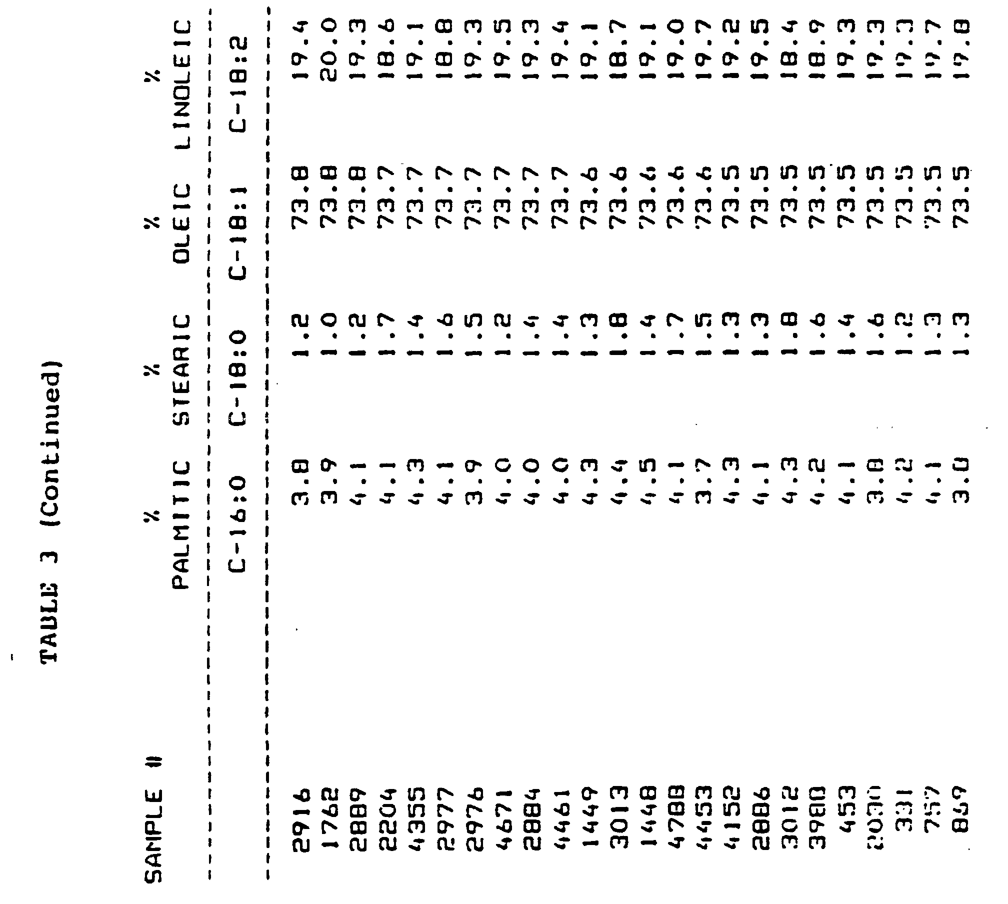

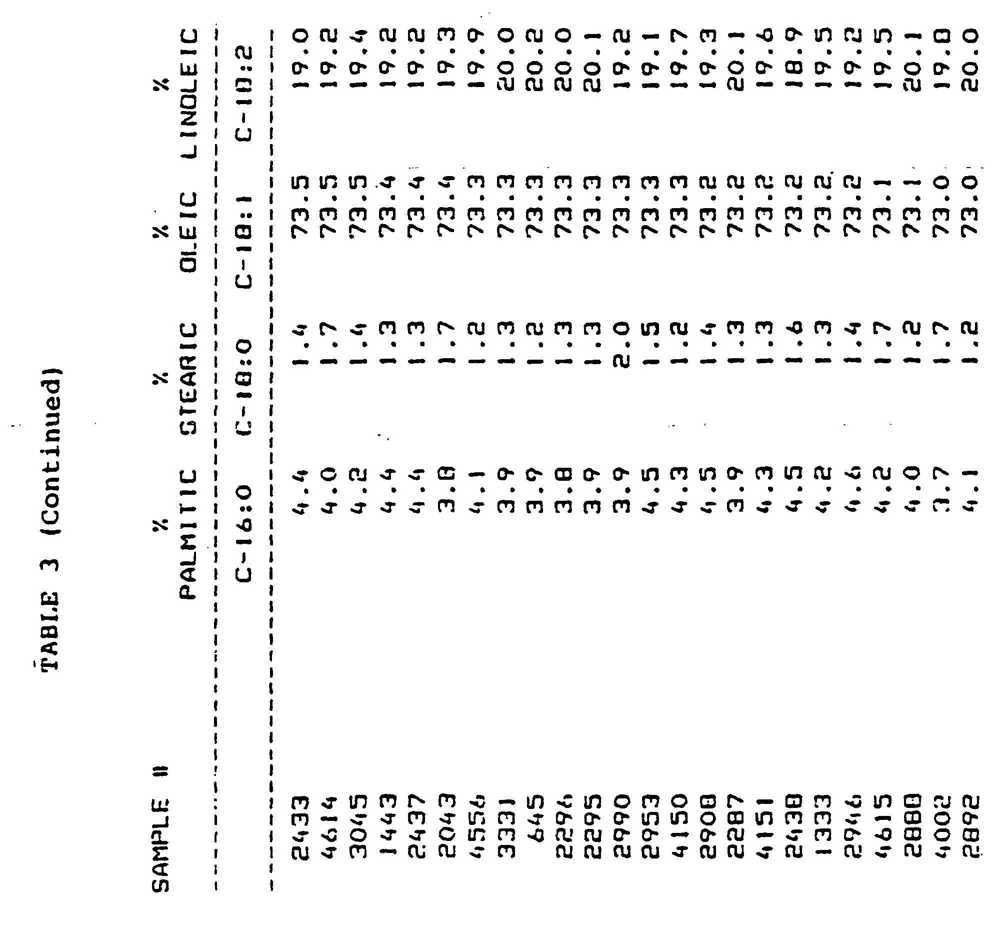

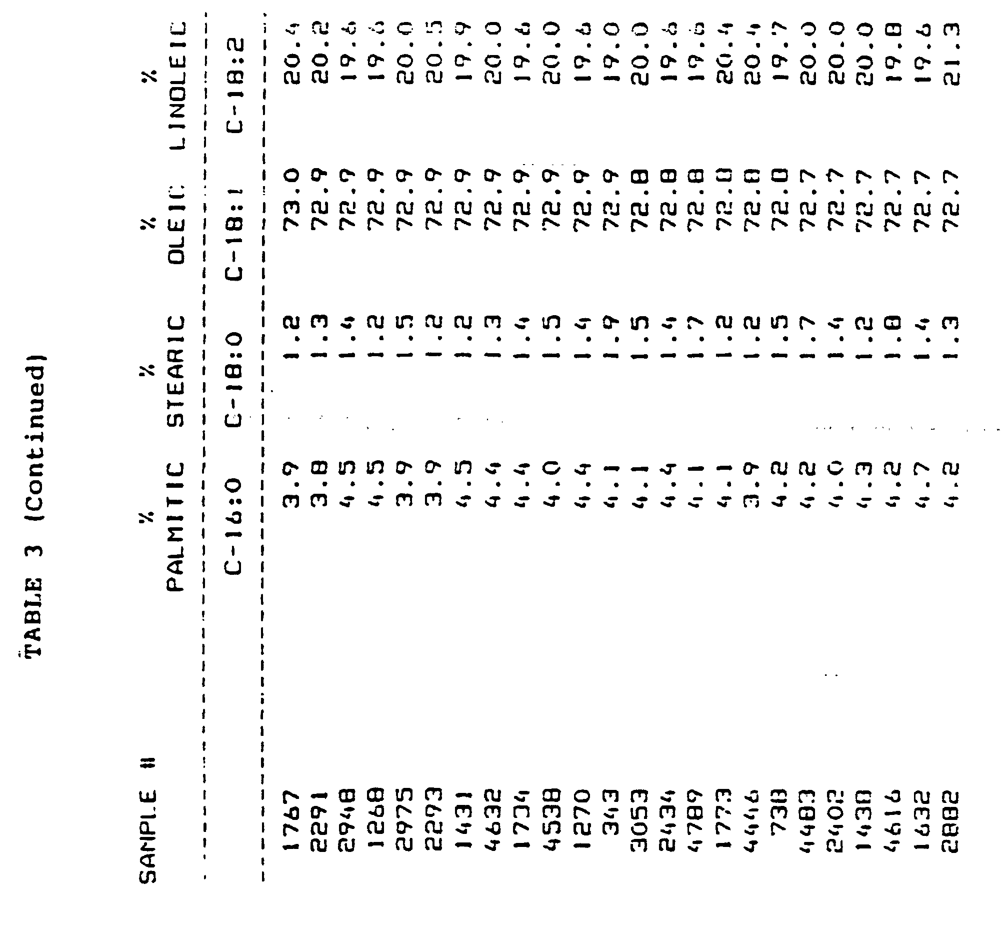

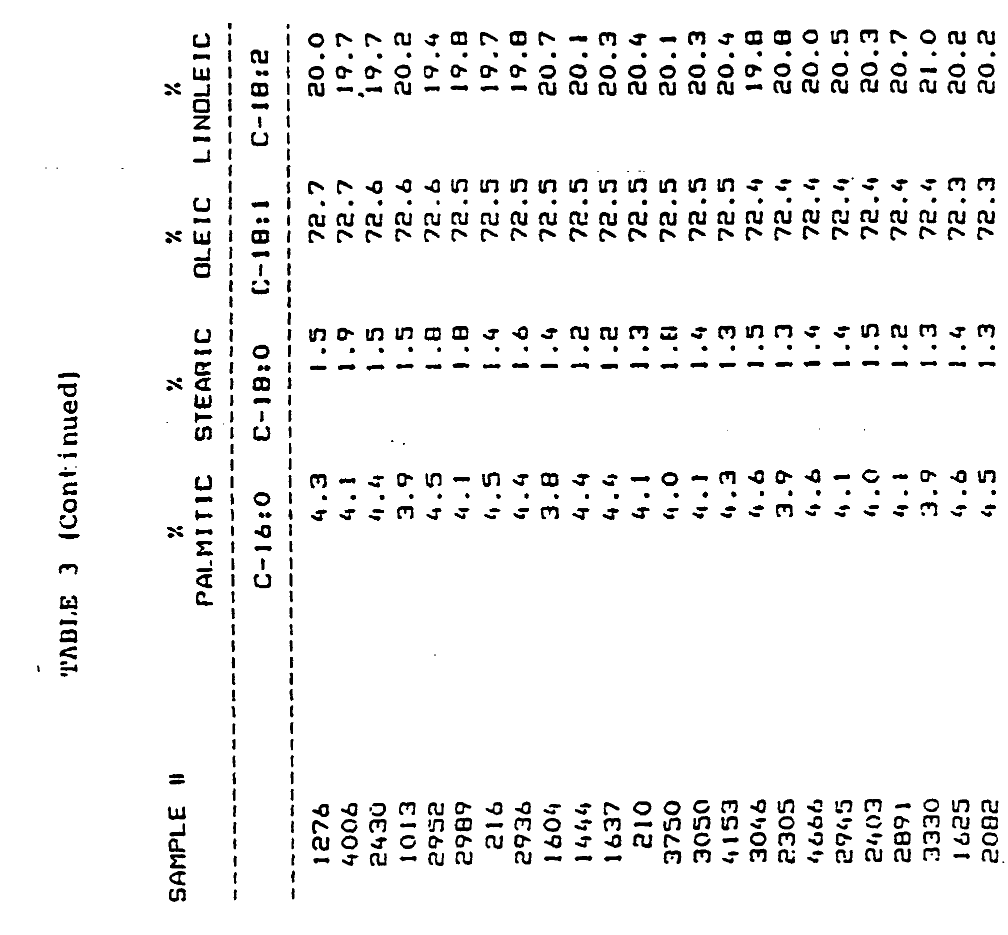

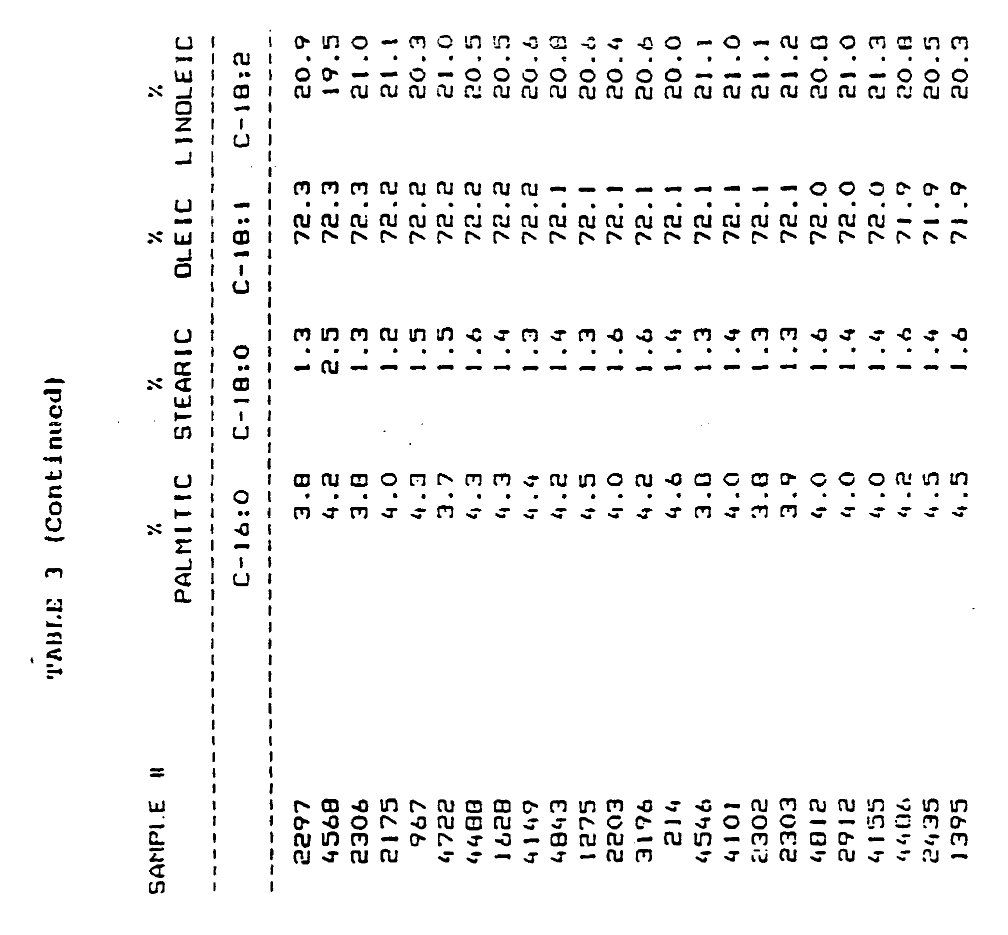

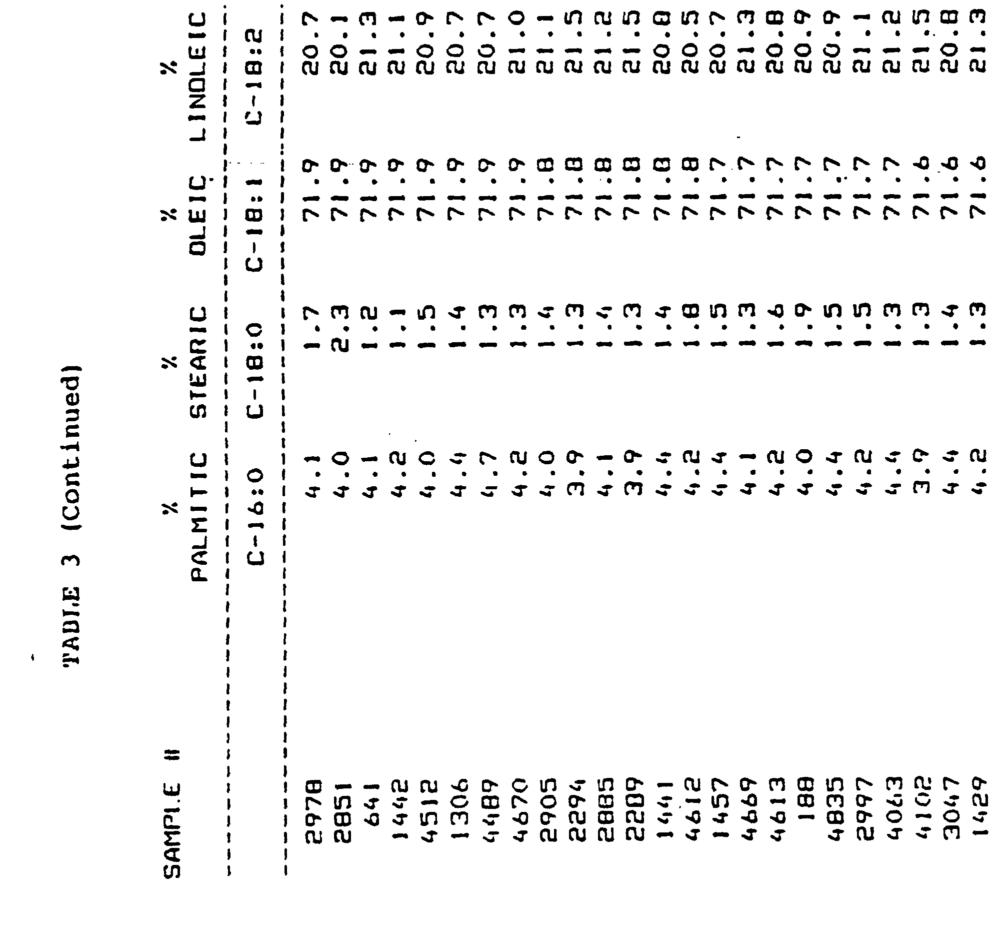

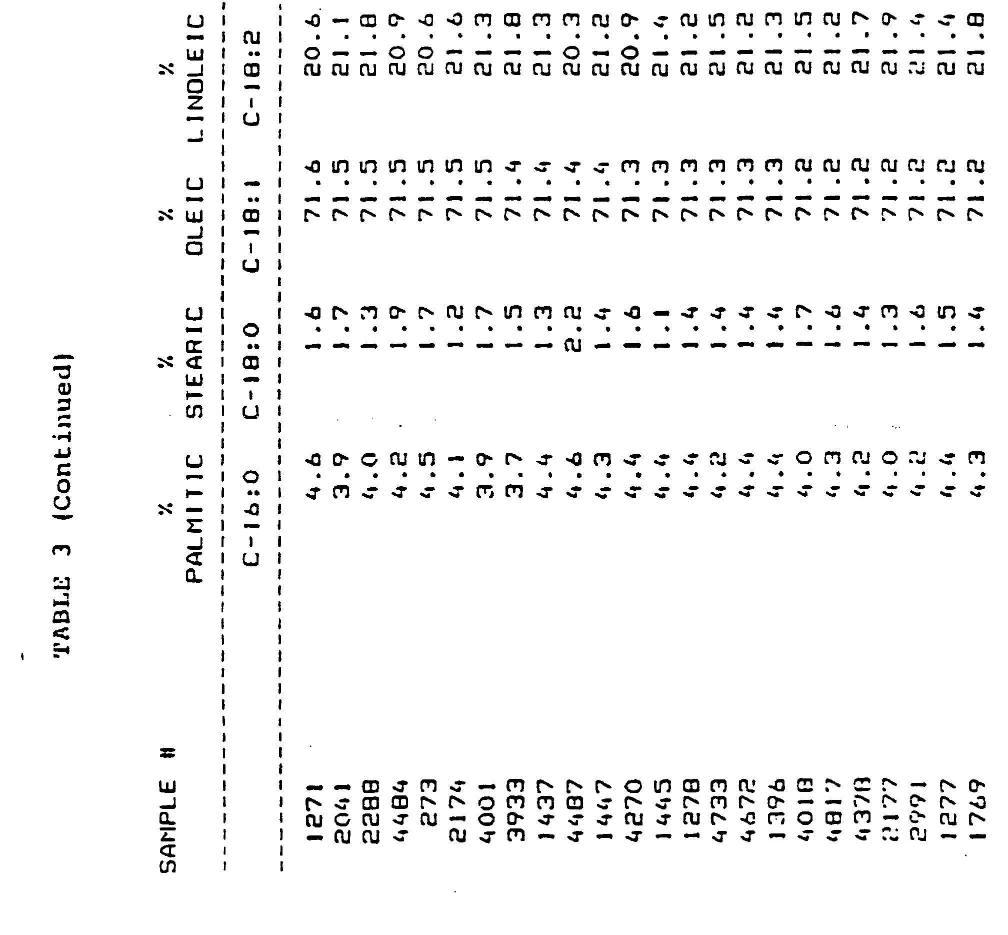

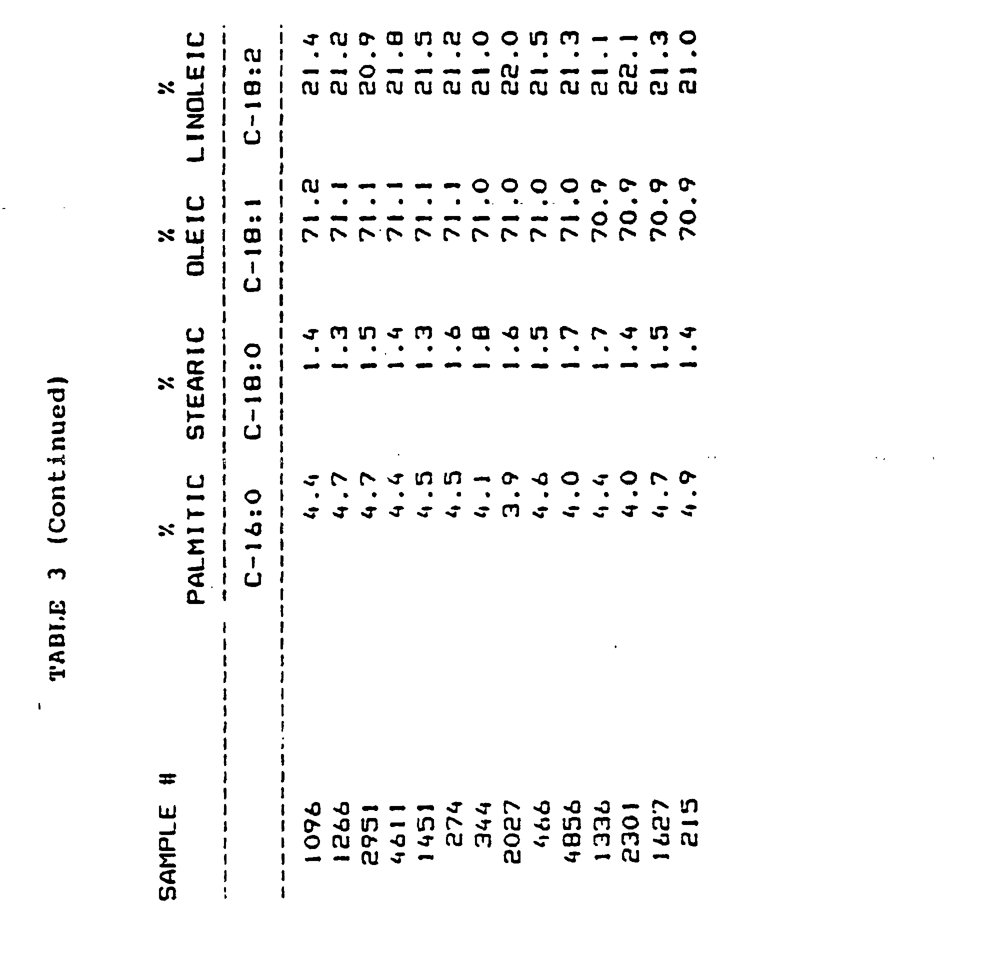

### Example 4

### 1986 Breeding Program and Results

These 302 lines were selfed in 1986 and the seed analyzed for oleic acid production. A total of 215 lines were selected after the 1986 season which produced seeds having an oleic acid content greater than 80%. The range in oleic acid content of these lines was 80% to 84.7%. The linoleic acid content ranged from 8.4% to 13.6% and the saturated fatty acid content ranged from 3.9% to 7.5%. The ratio of saturated fatty acids to oleic acid ranged from 0.047 to 0.086, with one line at 0.093. Twenty-four of these lines produced seeds having an oleic acid content of greater than 83%. The mean temperatures for July and August were 68.9°F and 68.0°F, respectively. The results are shown in Table 4.

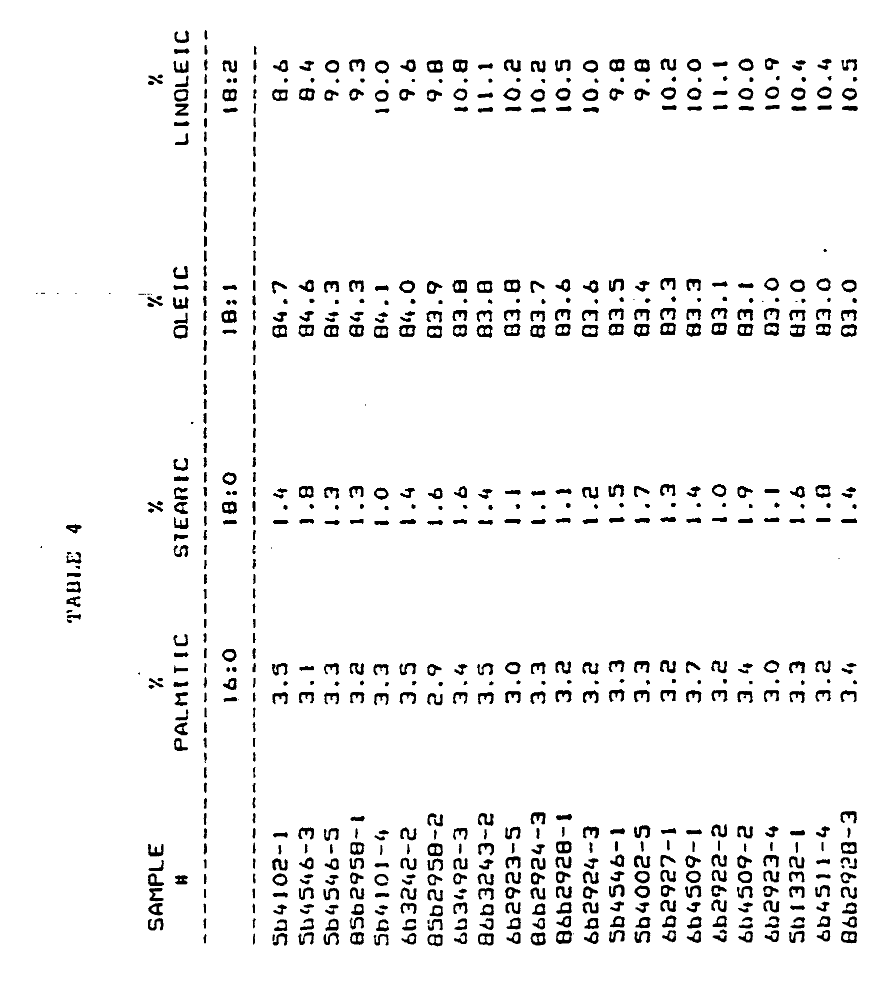

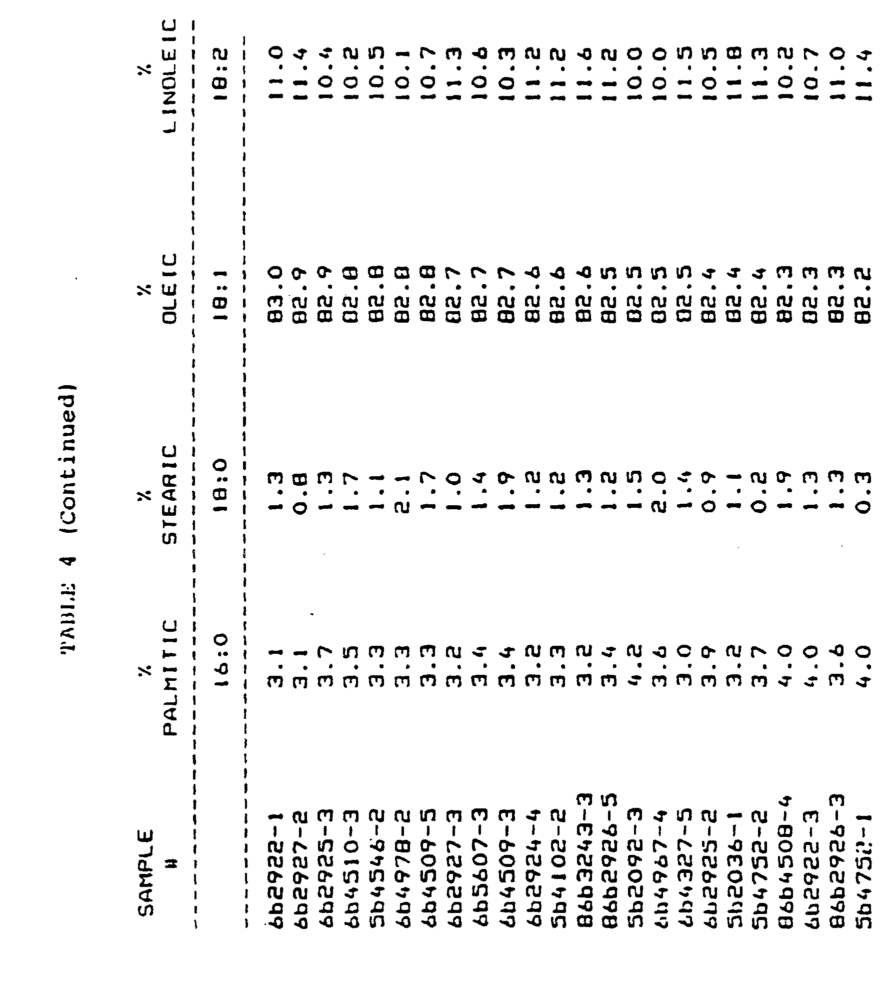

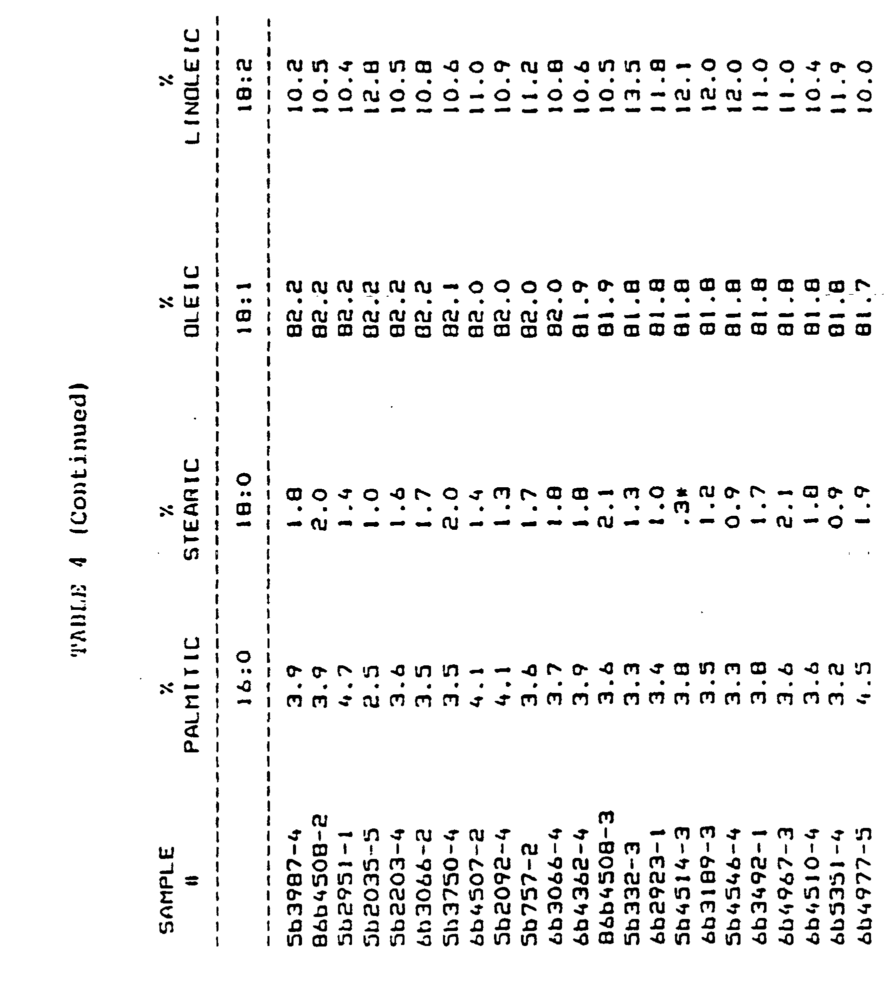

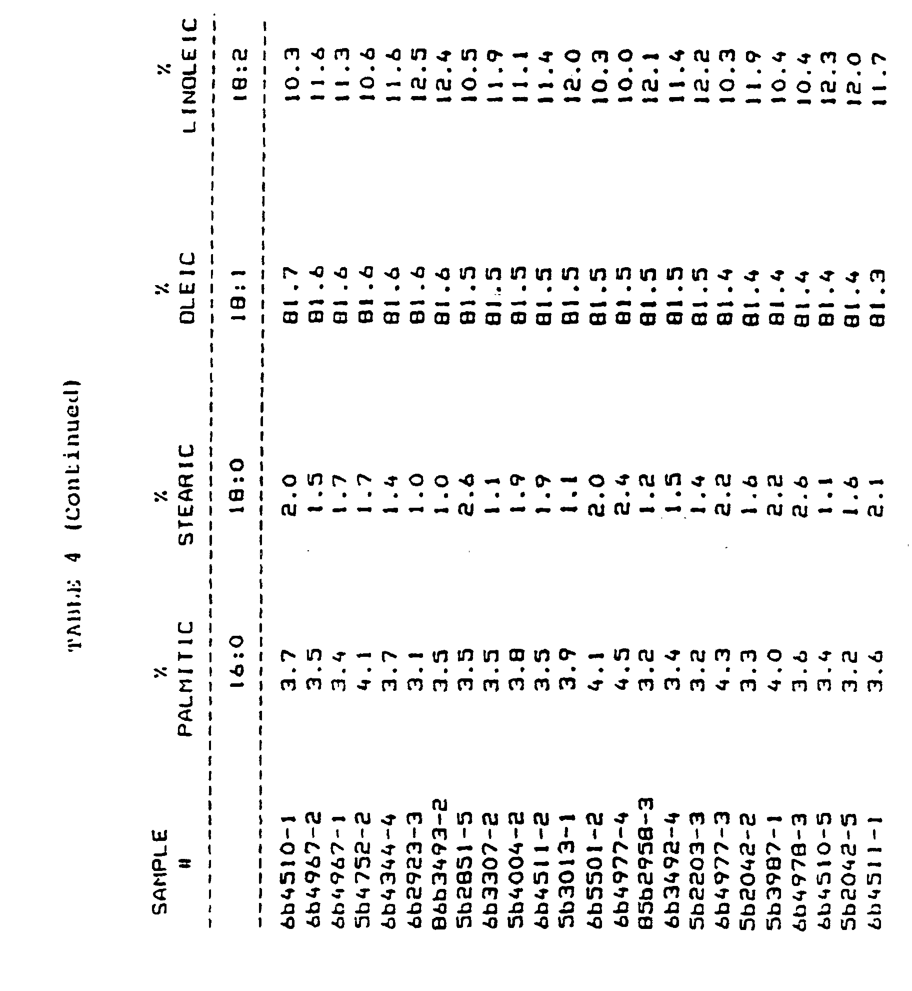

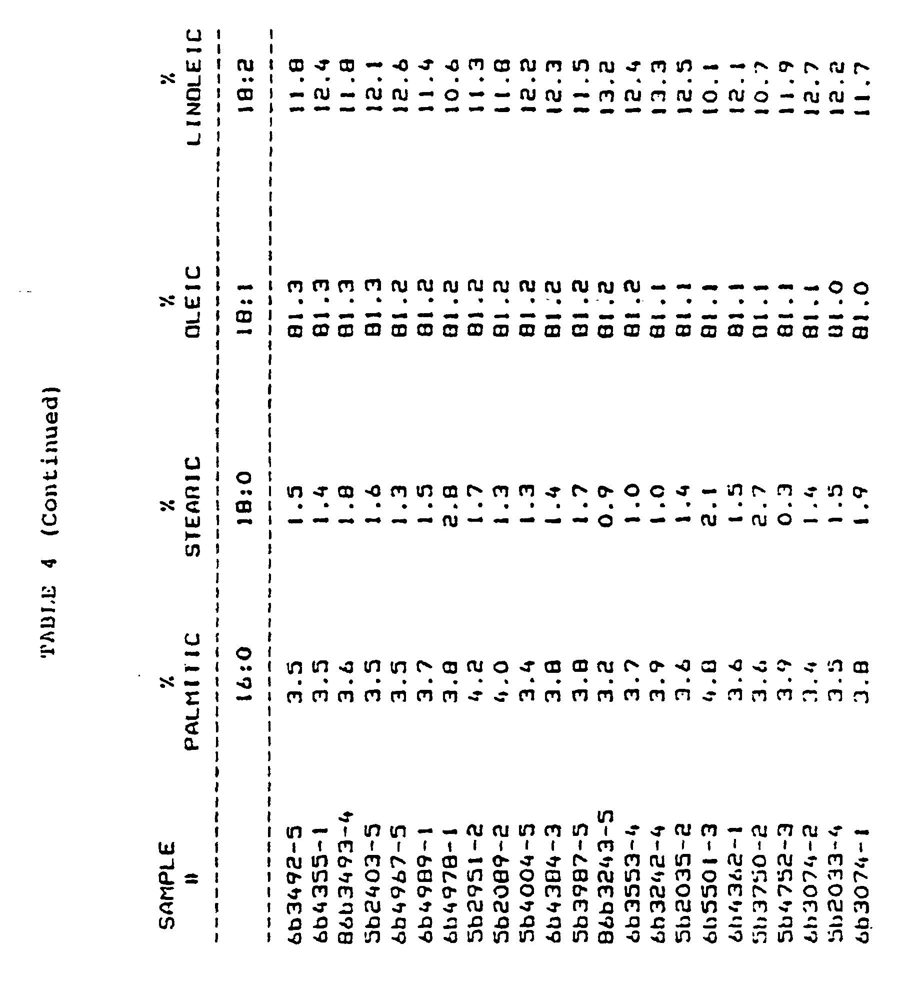

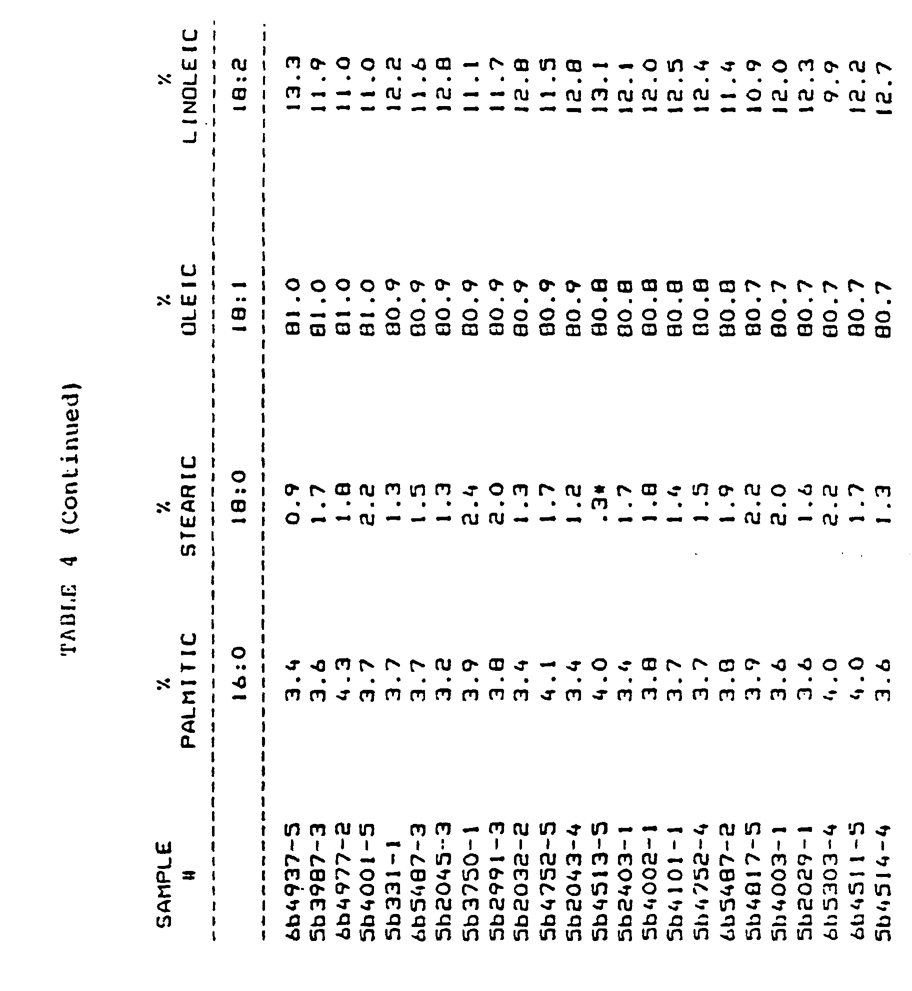

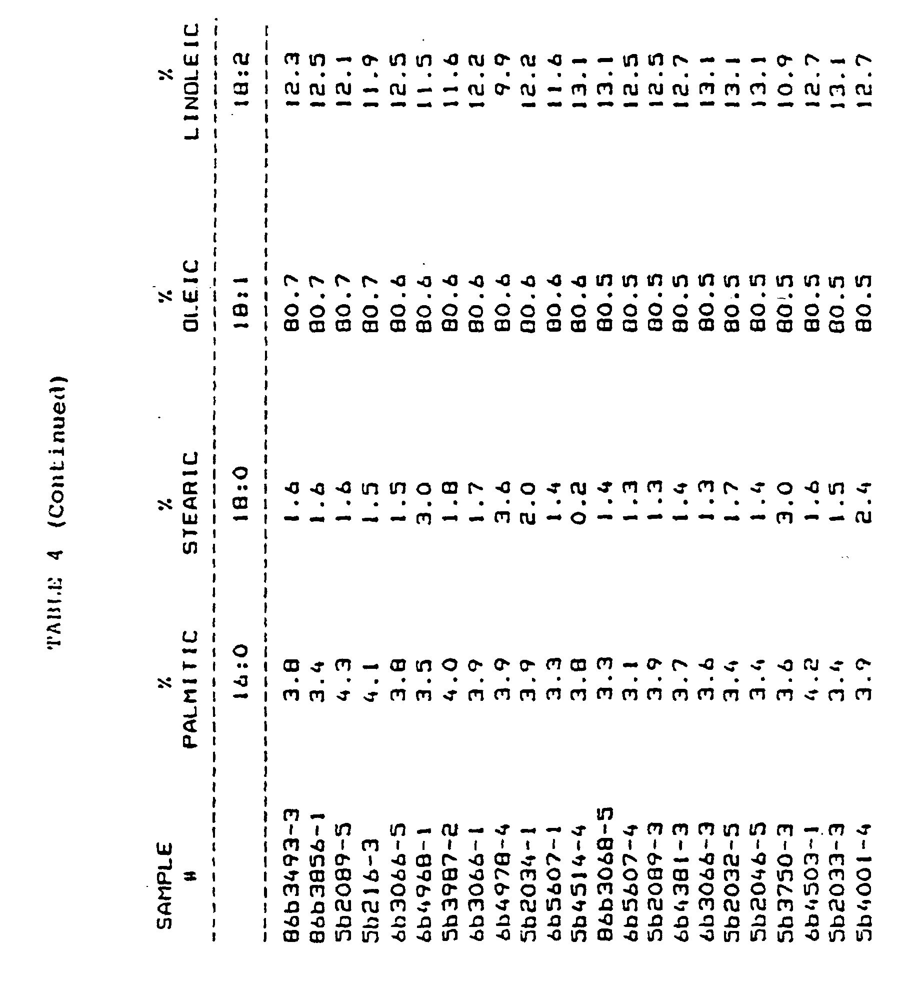

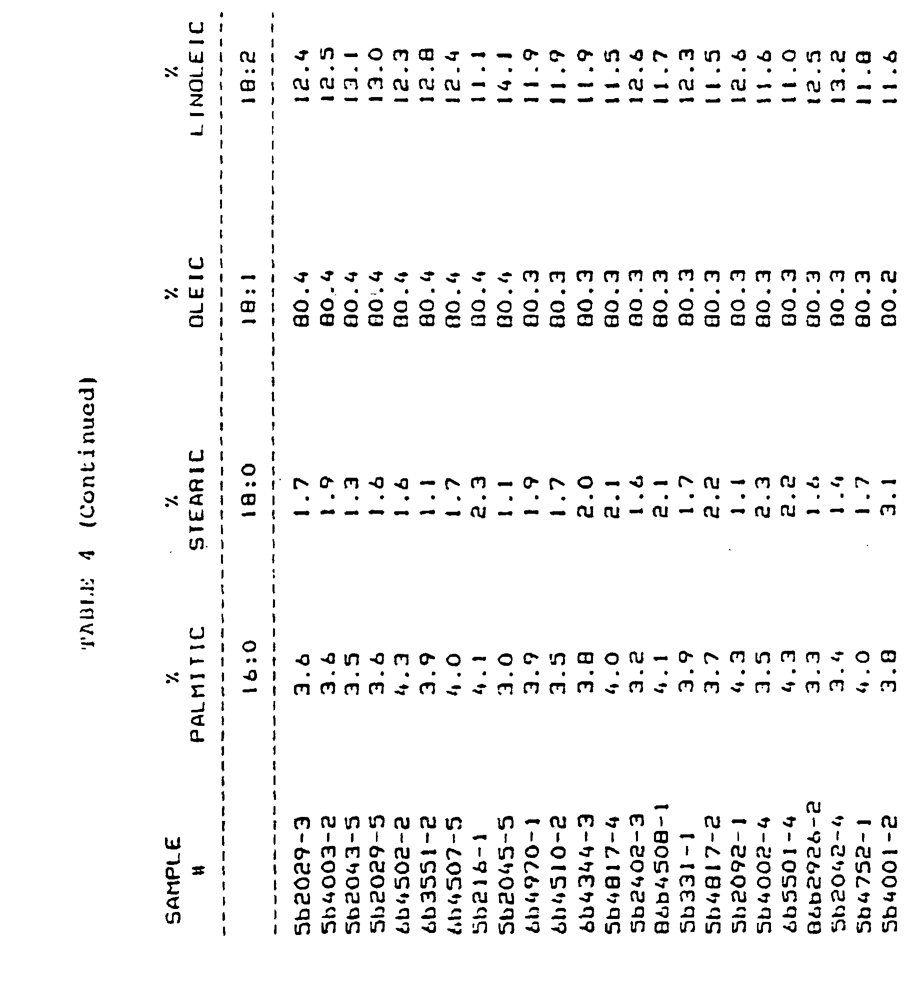

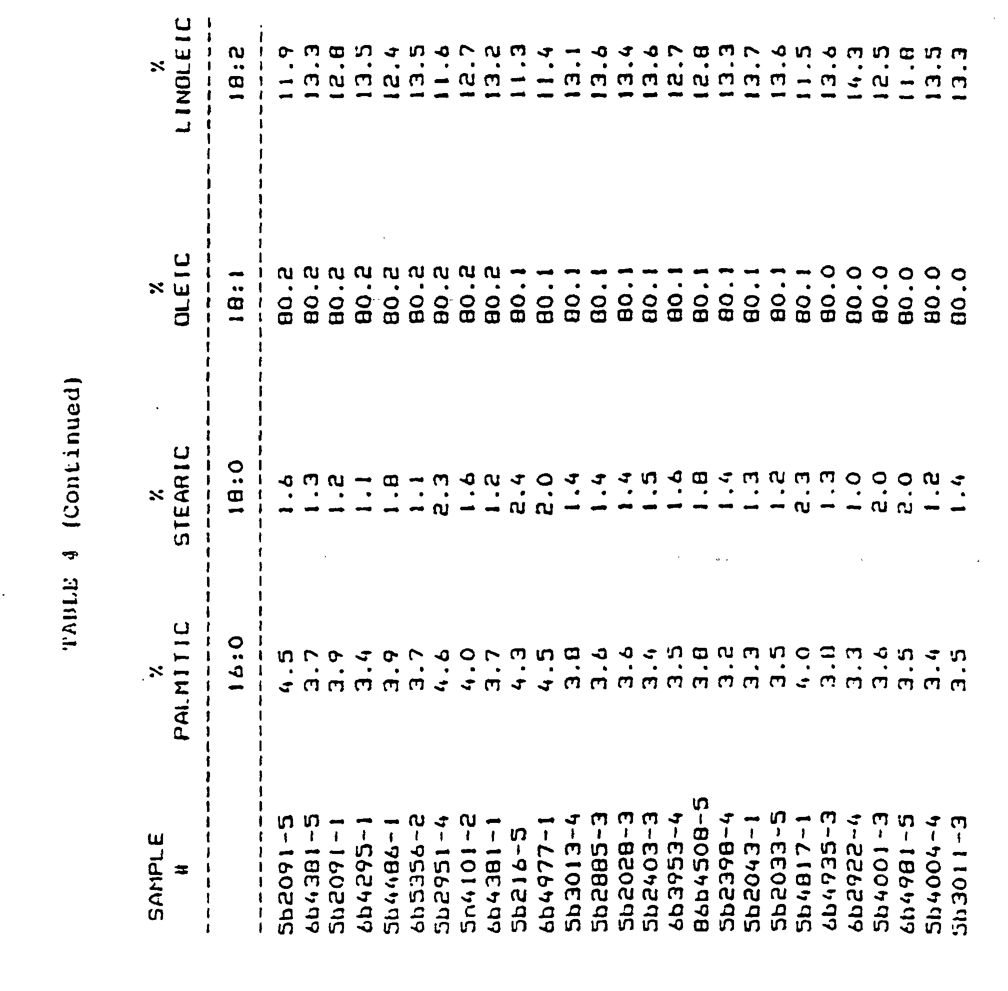

### Example 5

### 1987 Breeding Program and Results

These 215 lines were selfed in 1987 and the seed analyzed for oleic acid production. A total of 303 lines were selected after the 1987 season which produced seeds having oleic acid content greater than 80%. The range in oleic acid content of these lines was 81.9% to 85.9%. The linoleic acid content ranged from 7.2% to 11.8% and the saturated fatty acid content ranged from 3.7% to 6.2%. The ratio of saturated fatty acids to oleic acid ranged from 0.044 to 0.076. Forty-eight of these lines produced seeds having an oleic acid content of greater than 84%. The mean temperatures for July and August were 71.3°F and 66.0°F, respectively. The results are shown in Table 5.

| FIELD ENTRY | % PALMITIC | % STEARIC | % OLEIC | % LINOLEIC |
|---|---|---|---|---|
| # 16:0 | 16.0 | 18:0 | 18:1 | 18:2 |
| 7b3074-1 | 3.5 | 1.4 | 85.9 | 7.2 |
| 7b2377-2 | 3.2 | 1.1 | 85.3 | 8.6 |
| 7b3151-1 | 3.2 | 1.2 | 85.0 | 8.6 |
| 7b3144-5 | 3.3 | 1.1 | 84.9 | 8.6 |
| 7b3155-2 | 3.3 | 1.1 | 84.9 | 8.8 |
| 7b3361-2 | 3.4 | 1.4 | 84.8 | 8.5 |
| 7b3083-2 | 3.8 | 1.7 | 84.8 | 7.4 |
| 7b2377-1 | 3.2 | 1.2 | 84.8 | 8.8 |
| 7b3203-2 | 3.0 | 0.7 | 84.7 | 10.2 |
| 7b3081-2 | 3.8 | 1.9 | 84.7 | 7.4 |
| 7b3424-2 | 3.1 | 0.9 | 84.7 | 9.3 |
| 7b3523-1 | 3.2 | 0.9 | 84.7 | 9.3 |
| 7b3154-1 | 3.3 | 1.1 | 84.7 | 9.1 |
| 7b2382-2 | 3.1 | 1.1 | 84.6 | 9.5 |
| 7b2381-3 | 3.4 | 1.1 | 84.6 | 8.9 |
| 7b3075-2 | 2.9 | 1.2 | 84.5 | 9.4 |
| 7b2382-1 | 3.1 | 1.2 | 84.5 | 9.2 |
| 7b3078-1 | 3.2 | 1.2 | 84.5 | 9.0 |
| 7b3074-4 | 3.6 | 1.7 | 84.5 | 8.1 |
| 7b2376-2 | 3.1 | 1.1 | 84.4 | 9.4 |
| 7b3157-1 | 3.1 | 1.1 | 84.4 | 9.2 |
| 7b3497-2 | 3.4 | 1.1 | 84.4 | 9.6 |
| 7b3144-2 | 3.6 | 1.1 | 84.4 | 8.8 |
| 7b3151-2 | 3.1 | 1.0 | 84.4 | 9.4 |
| 7b3496-2 | 3.2 | 1.0 | 84.3 | 9.5 |
| 7b532-2 | 3.3 | 0.8 | 84.3 | 10.2 |
| 7b2382-4 | 3.2 | 1.0 | 84.3 | 9.3 |
| 7b3151-2 | 3.1 | 1.0 | 84.3 | 9.7 |
| 7b3354-2 | 3.6 | 1.3 | 84.3 | 8.9 |
| 7b3497-3 | 3.5 | 1.0 | 84.2 | 9.1 |
| 7b3498-2 | 3.5 | 1.1 | 84.2 | 9.3 |
| 7b3524-2 | 3.2 | 1.1 | 84.2 | 9.5 |
| 7b3151-4 | 3.0 | 1.0 | 84.2 | 9.8 |
| 7b2381-1 | 3.4 | 0.9 | 84.1 | 9.4 |
| 7b3148-2 | 3.2 | 1.1 | 84.1 | 9.7 |
| 7b3168-2 | 3.2 | 1.3 | 84.1 | 9.4 |
| 7b3764-1 | 3.9 | 1.0 | 84.1 | 8.8 |
| 7b3156.1 | 3.1 | 1.1 | 84.1 | 9.5 |
| 7b3159-1 | 3.1 | 1.0 | 84.1 | 9.9 |
| 7b3SB9-1 | 3.6 | 1.0 | 84.1 | 9.4 |
| 7b3076-2 | 3.2 | 1.8 | 84.0 | 8.8 |
| 7b3082-4 | 3.6 | 1.5 | 84.0 | 8.5 |
| 7b3589-1 | 3.2 | 1.1 | 84.0 | 11.2 |
| 7b3523-1 | 3.5 | 1.1 | 84.0 | 9.4 |
| 7b3211-1 | 3.3 | 1.3 | 84.0 | 9.5 |
| 7b3333-2 | 3.4 | 1.5 | 84.0 | 8.9 |
| 7b3155-1 | 3.6 | 1.0 | 83.9 | 9.3 |
| 7b3325-1 | 3.4 | 1.6 | 83.9 | 8.9 |
| 7b3498-1 | 3.4 | 1.1 | 83.9 | 9.8 |
| 7b3496-1 | 3.3 | 1.1 | 83.9 | 10.0 |
| 7b3361-1 | 3.7 | 1.8 | 83.9 | 8.4 |
| 7b3166-1 | 3.1 | 1.0 | 83.9 | 10.1 |
| 7b2377-1 | 3.2 | 1.3 | 83.9 | 9.4 |
| 7b532-1 | 3.3 | 1.2 | 83.9 | 9.7 |
| 7b2376-5 | 3.2 | 1.0 | 83.8 | 9.9 |
| 7b2381-2 | 3.4 | 0.9 | 83.8 | 9.8 |
| 7b3179-1 | 3.5 | 0.6 | 83.8 | 10.6 |
| 7b2382-2 | 3.4 | 1.0 | 83.8 | 9.5 |
| 7b3449-1 | 3.4 | 1.8 | 83.8 | 9.0 |
| 7b3424-5 | 3.1 | 1.1 | 83.8 | 10.0 |
| 7b3764-2 | 3.4 | 0.8 | 83.7 | 10.0 |
| 7b3157-2 | 3.2 | 1.1 | 83.7 | 9.9 |
| 7b2382-1 | 3.2 | 1.2 | 83.7 | 9.7 |
| 7b2205-2 | 3.4 | 1.1 | 83.7 | 9.8 |
| 7b3158-2 | 3.2 | 1.3 | 83.7 | 9.7 |
| 7b3588-1 | 3.3 | 1.2 | 83.7 | 9.9 |
| 7b3201-9 | 3.3 | 1.0 | 83.7 | 9.9 |
| 7b3362-2 | 3.4 | 2.0 | 83.7 | 8.7 |
| 7b532-5 | 3.5 | 0.9 | 83.7 | 9.9 |
| 7b3152-2 | 3.1 | 1.1 | 83.7 | 10.1 |
| 7b3523-3 | 3.7 | 0.9 | 83.7 | 9.7 |
| 7b3217-1 | 3.3 | 1.4 | 83.6 | 9.5 |
| 7b3589-2 | 3.3 | 1.2 | 83.6 | 9.9 |
| 7b3159-5 | 3.3 | 1.1 | 83.6 | 10.0 |
| 7b1111-2 | 3.1 | 0.8 | 83.6 | 10.7 |
| 7b3502-1 | 3.3 | 1.0 | 83.6 | 10.0 |
| 7b3336-2 | 3.4 | 1.5 | 83.6 | 9.4 |
| 7b2376-4 | 3.1 | 1.0 | 83.6 | 10.2 |
| 7b2381-1 | 3.2 | 0.9 | 83.6 | 10.2 |
| 7b3217-2 | 3.5 | 1.2 | 83.5 | 10.0 |
| 7b3302-2 | 3.5 | 1.5 | 83.5 | 9.3 |
| 7b3082-2 | 3.6 | 1.9 | 83.5 | 8.6 |
| 7b3501-1 | 3.4 | 1.1 | 83.5 | 10.3 |
| 7b3164-1 | 3.3 | 1.0 | 83.5 | 10.1 |
| 7b3149-1 | 3.3 | 1.1 | 83.5 | 9.9 |
| 7b3261-1 | 3.5 | 2.1 | 83.5 | 8.7 |
| 7b2378-1 | 3.3 | 0.9 | 83.5 | 10.7 |
| 7b3493-2 | 3.6 | 1.0 | 83.4 | 9.9 |
| 7b3502-2 | 3.4 | 1.1 | 83.4 | 10.2 |
| 7b3076-7 | 3.2 | 1.7 | 83.4 | 9.7 |
| 7b3912-2 | 3.4 | 1.4 | 83.4 | 9.9 |
| 7b3246-1 | 4.2 | 1.0 | 83.4 | 9.5 |
| 7b3201-2 | 3.2 | 1.0 | 83.4 | 10.2 |
| 7b3349-1 | 3.3 | 1.5 | 83.4 | 9.9 |
| 7b3588-3 | 3.4 | 1.2 | 83.4 | 9.9 |
| 7b3226-1 | 3.6 | 2.0 | 83.3 | 9.2 |
| 7b494-1 | 3.9 | 1.4 | 83.3 | 9.9 |
| 7b3166-2 | 3.2 | 1.1 | 83.3 | 10.3 |
| 7b2378-1 | 3.3 | 0.9 | 83.3 | 10.5 |
| 7b3311-1 | 3.2 | 1.6 | 83.3 | 10.1 |
| 7b3412-1 | 3.2 | 1.2 | 83.3 | 10.5 |
| 7b2382-3 | 3.4 | 1.2 | 83.3 | 9.8 |
| 7b3332-2 | 3.4 | 1.5 | 83.3 | 9.7 |
| 7b3236-1 | 3.4 | 1.7 | 83.3 | 9.3 |
| 7b2378-2 | 3.2 | 0.9 | 83.3 | 10.4 |
| 7b3326-1 | 3.4 | 1.6 | 83.3 | 9.8 |
| 7b3521-3 | 3.4 | 1.0 | 83.3 | 10.2 |
| 7b3238-1 | 3.7 | 2.0 | 83.2 | 9.1 |
| 7b3449-2 | 3.5 | 1.6 | 83.2 | 9.7 |
| 7b3881-2 | 3.3 | 0.7 | 83.2 | 10.7 |
| 7b3409-2 | 3.7 | 1.8 | 83.2 | 9.4 |
| 7b3268-4 | 4.2 | 2.8 | 83.2 | 8.3 |
| 7b3766-2 | 3.3 | 1.0 | 83.2 | 10.5 |
| 7b3333-1 | 3.4 | 1.4 | 83.2 | 9.8 |
| 7b3588-5 | 3.3 | 1.0 | 83.2 | 10.4 |
| 7b3544-2 | | | 83.2 | 16.8 |
| 7b3588-4 | 3.4 | 1.2 | 83.2 | 10.2 |
| 7b3349-2 | 3.2 | 1.7 | 83.2 | 9.9 |
| 7b3408-3 | 3.6 | 1.4 | 83.1 | 10.1 |
| 7b2381-5 | 3.4 | 1.0 | 83.1 | 10.3 |
| 7b3235-3 | 4.0 | 1.5 | 83.1 | 9.2 |
| 7b3206-2 | 3.4 | 1.2 | 83.1 | 10.1 |
| 7b3341-1 | 3.6 | 2.1 | 83.1 | 9.1 |
| 7b3212-2 | 3.2 | 1.1 | 83.1 | 10.4 |
| 7b3348-2 | 3.3 | 1.6 | 83.1 | 10.0 |
| 7b3581-1 | 3.7 | 1.1 | 83.1 | 10.0 |
| 7b2378-2 | 3.3 | 1.0 | 83.1 | 10.8 |
| 7b3211-2 | 3.2 | 1.1 | 83.1 | 10.5 |
| 7b3407-4 | 3.8 | 1.8 | 83.1 | 9.3 |
| 7b3293-2 | 3.0 | 1.4 | 83.0 | 11.2 |
| 7b3298-1 | 3.2 | 1.4 | 83.0 | 11.0 |
| 7b3165-1 | 3.2 | 1.1 | 83.0 | 10.5 |
| 7b3583-1 | 3.4 | 1.1 | 83.0 | 10.7 |
| 7b2381-2 | 3.5 | 0.8 | 83.0 | 10.6 |
| 7b3206-1 | 3.3 | 1.1 | 83.0 | 10.5 |
| 7b3346-1 | 3.3 | 1.5 | 83.0 | 10.3 |
| 7b3503-2 | 3.3 | 1.0 | 83.0 | 10.9 |
| 7b3526-1 | 3.2 | 1.0 | 83.0 | 10.7 |
| 7b3168-1 | 3.1 | 1.0 | 83.0 | 10.7 |
| 7b3429-5 | 3.4 | 1.4 | 82.9 | 10.2 |
| 7b3159-2 | 3.1 | 1.1 | 82.9 | 10.9 |
| 7b3309-2 | 3.0 | 1.3 | 82.9 | 11.0 |
| 7b3294-2 | 3.3 | 1.5 | 82.9 | 10.5 |
| 7b3335-2 | 3.4 | 1.4 | 82.9 | 10.4 |
| 7b3497-1 | 3.5 | 1.1 | 82.9 | 10.6 |
| 7b3406-2 | 3.9 | 2.0 | 82.9 | 9.2 |
| 7b3499-1 | 3.3 | 1.1 | 82.9 | 10.7 |
| 7b3317-1 | 3.4 | 1.6 | 82.9 | 10.0 |
| 7b2193-4 | 3.5 | 1.2 | 82.9 | 10.3 |
| 7b3357-4 | 3.6 | 1.8 | 82.9 | 9.5 |
| 7b3164-2 | 3.3 | 1.0 | 82.9 | 10.6 |
| 7b3235-5 | 4.0 | 1.5 | 82.8 | 9.6 |
| 7b3412-2 | 3.3 | 1.3 | 82.8 | 10.8 |
| 7b3221-5 | 3.6 | 1.5 | 82.8 | 9.8 |
| 7b3424-4 | 3.5 | 1.1 | 82.8 | 10.8 |
| 7b3322-1 | 3.4 | 1.7 | 82.8 | 10.2 |
| 7b3499-2 | 3.4 | 1.0 | 82.8 | 10.8 |
| 7b3334-1 | 3.4 | 1.4 | 82.8 | 10.1 |
| 7b3504-1 | 3.4 | 1.1 | 82.8 | 10.5 |
| 7b3311-2 | 3.2 | 1.6 | 82.8 | 10.7 |
| 7b3347-1 | 3.3 | 1.6 | 82.8 | 10.4 |
| 7b3331-2 | 3.3 | 1.5 | 82.8 | 10.1 |
| 7b3148-1 | 3.2 | 1.1 | 82.8 | 10.8 |
| 7b3228-2 | 3.8 | 2.0 | 82.7 | 9.6 |
| 7b3424-1 | 3.4 | 1.0 | 82.7 | 10.9 |
| 7b3295-1 | 3.2 | 1.5 | 82.7 | 10.7 |
| 7b3408-2 | 3.5 | 1.3 | 82.7 | 10.6 |
| 7b3081-1 | 3.5 | 1.6 | 82.7 | 10.2 |
| 7b3345-1 | 3.5 | 1.5 | 82.7 | 10.2 |
| 7b3089-2 | 3.9 | 1.7 | 82.7 | 9.8 |
| 7b3335-1 | 3.5 | 1.6 | 82.7 | 10.1 |
| 7b3153-2 | 3.1 | 1.1 | 82.7 | 10.8 |
| 7b3334-2 | 3.4 | 1.6 | 82.7 | 10.3 |
| 7b3179-2 | 3.6 | 0.9 | 82.7 | 10.6 |
| 7b2382-5 | 3.2 | 1.1 | 82.7 | 10.8 |
| 7b3187-1 | 3.3 | 1.0 | 82.7 | 10.6 |
| 7b3214-1 | 3.5 | 1.0 | 82.7 | 10.1 |
| 7b3574-1 | 3.3 | 1.5 | 82.7 | 10.7 |
| 7b3218-1 | 3.6 | 1.0 | 82.7 | 10.8 |
| 7b3147-1 | 3.1 | 1.1 | 82.7 | 11.0 |
| 7b3323-2 | 3.2 | 1.3 | 82.7 | 10.9 |
| 7b3182-1 | 3.2 | 1.0 | 82.7 | 11.0 |
| 7b3312-2 | 3.2 | 1.5 | 82.7 | 10.7 |
| 7b3911-1 | 3.2 | 1.6 | 82.7 | 10.5 |
| 7b3203-1 | 3.2 | 0.9 | 82.7 | 10.9 |
| 7b3158-1 | 3.2 | 1.1 | 82.7 | 10.9 |
| 7b3303-2 | 3.3 | 1.5 | 82.7 | 11.0 |
| 7b2204-1 | 3.4 | 1.1 | 82.6 | 10.8 |
| 7b3293-1 | 3.1 | 1.4 | 82.6 | 11.0 |
| 7b2258-2 | 3.3 | 1.0 | 82.6 | 11.3 |
| 7b3322-1 | 3.3 | 1.4 | 82.6 | 10.7 |
| 7b3149-2 | 3.2 | 1.1 | 82.6 | 10.9 |
| 7b3338-1 | 3.4 | 2.0 | 82.6 | 9.8 |
| 7b3218-2 | 3.3 | 0.6 | 82.6 | 11.4 |
| 7b17-4 | 3.2 | 1.4 | 82.6 | 10.9 |
| 7b1970-1 | 3.6 | 1.5 | 82.6 | 10.1 |
| 7b3248-1 | 4.0 | 1.3 | 82.6 | 10.2 |
| 7b3167-2 | 3.2 | 1.1 | 82.6 | 10.8 |
| 7b3766-1 | 3.5 | 1.1 | 82.6 | 10.6 |
| 7b3359-1 | 3.8 | 1.4 | 82.5 | 10.4 |
| 7b3219-6 | 3.7 | 1.6 | 82.5 | 10.1 |
| 7b3212-1 | 3.3 | 1.1 | 82.5 | 11.0 |
| 7b3411-2 | 3.3 | 1.2 | 82.5 | 11.2 |
| 7b3208-2 | 3.3 | 1.1 | 82.5 | 11.0 |
| 7b3589-2 | 3.5 | 1.3 | 82.5 | 10.7 |
| 7b3173-1 | 3.3 | 0.9 | 82.5 | 11.2 |
| 7b3329-3 | 3.3 | 1.3 | 82.5 | 10.8 |
| 7b3153-1 | 3.0 | 1.0 | 82.5 | 11.1 |
| 7b3314-1 | 3.0 | 1.4 | 82.5 | 11.2 |
| 7b3588-1 | 3.5 | 1.2 | 82.5 | 10.7 |
| 7b1462-3 | 3.7 | 1.1 | 82.5 | 10.9 |
| 7b3777-2 | 3.2 | 1.0 | 82.5 | 11.2 |
| 7b3334-1 | 3.4 | 1.2 | 82.5 | 11.3 |
| 7b2204-2 | 3.4 | 1.1 | 82.5 | 10.6 |
| 7b3242-2 | 3.8 | 1.7 | 82.5 | 10.0 |
| 7b3323-1 | 3.3 | 1.4 | 82.5 | 10.8 |
| 7b3797-1 | 3.7 | 1.1 | 82.5 | 11.0 |
| 7b2376-2 | 3.2 | 1.1 | 82.5 | 11.0 |
| 7b3169-1 | 3.2 | 1.1 | 82.4 | 11.3 |
| 7b3147-2 | 3.3 | 0.9 | 82.4 | 11.2 |
| 7b3319-2 | 3.3 | 1.5 | 82.4 | 10.9 |
| 7b2381-4 | 3.3 | 0.9 | 82.4 | 11.1 |
| 7b3315-2 | 3.3 | 1.8 | 82.4 | 10.6 |
| 7b2378-3 | 3.2 | 0.8 | 82.4 | 11.5 |
| 7b3296-2 | 3.2 | 1.4 | 82.4 | 11.1 |
| 7b3340-1 | 3.7 | 1.9 | 82.4 | 9.7 |
| 7b3178-1 | 3.4 | 0.9 | 82.4 | 11.8 |
| 7b3356-1 | 4.0 | 1.5 | 82.4 | 10.2 |
| 7b3316-1 | 3.3 | 1.6 | 82.4 | 10.7 |
| 7b3496-1 | 3.3 | 0.9 | 82.4 | 11.4 |
| 7b3245-1 | 3.7 | 1.9 | 82.4 | 10.0 |
| 7b3297-2 | 3.2 | 1.5 | 82.4 | 11.0 |
| 7b2386-2 | 4.1 | 1.5 | 82.4 | 9.7 |
| 7b3592-2 | 3.6 | 1.9 | 82.4 | 10.2 |
| 7b3391-2 | 3.3 | 2.0 | 82.3 | 10.5 |
| 7b3298-2 | 3.2 | 1.5 | 82.3 | 11.2 |
| 7b3307-1 | 3.2 | 1.4 | 82.3 | 11.2 |
| 7b3261-2 | 3.4 | 1.6 | 82.3 | 10.7 |
| 7b3340-2 | 3.3 | 2.1 | 82.3 | 9.8 |
| 7b3234-2 | 4.2 | 1.6 | 82.3 | 9.6 |
| 7b3585-1 | 3.3 | 1.3 | 82.3 | 11.1 |
| 7b3178-1 | 3.2 | 0.9 | 82.3 | 11.5 |
| 7b3321-2 | 3.2 | 1.5 | 82.3 | 11.3 |
| 7b2387-2 | 3.5 | 1.4 | 82.3 | 10.9 |
| 7b3397-2 | 3.6 | 2.1 | 82.3 | 10.0 |
| 7b3079-8 | 3.5 | 1.6 | 82.3 | 10.6 |
| 7b3322-2 | 3.3 | 1.2 | 82.3 | 11.2 |
| 7b3799-2 | 3.9 | 1.6 | 82.3 | 10.2 |
| 7b17-1 | 3.1 | 1.4 | 82.3 | 11.5 |
| 7b3075-1 | 3.2 | 1.2 | 82.3 | 11.5 |
| 7b3221-7 | 3.4 | 1.6 | 82.2 | 10.5 |
| 7b3199-1 | 3.6 | 1.2 | 82.2 | 11.0 |
| 7b2385-1 | 3.7 | 1.9 | 82.2 | 10.2 |
| 7b3436-1 | 3.7 | 1.4 | 82.2 | 10.7 |
| 7b3161-1 | 3.2 | 1.0 | 82.2 | 11.3 |
| 7b3346-2 | 3.5 | 1.3 | 82.2 | 11.2 |
| 7b3117-1 | 3.9 | 2.0 | 82.2 | 10.2 |
| 7b3309-1 | 3.2 | 1.4 | 82.2 | 11.1 |
| 7b3108-2 | 3.4 | 1.0 | 82.2 | 11.3 |
| 7b3881-1 | 3.6 | 1.0 | 82.2 | 11.0 |
| 7b3091-2 | 3.9 | 1.3 | 82.2 | 10.9 |
| 7b3368-8 | 4.1 | 1.9 | 82.2 | 9.7 |
| 7b3792-2 | 3.1 | 1.3 | 82.2 | 11.4 |
| 7b3247-2 | 3.5 | 1.4 | 82.2 | 11.0 |
| 7b3324-1 | 3.4 | 1.4 | 82.2 | 11.0 |
| 7b3795-2 | 3.8 | 1.2 | 82.2 | 11.0 |
| 7b3524-1 | 3.4 | 1.1 | 82.2 | 11.4 |
| 7b3306-2 | 3.4 | 1.4 | 82.1 | 11.2 |
| 7b3506-1 | 3.6 | 0.9 | 82.1 | 11.3 |
| 7b3563-4 | 3.8 | 1.2 | 82.1 | 11.0 |
| 7b2385-4 | 3.6 | 1.5 | 82.1 | 10.6 |
| 7b3526-2 | 3.3 | 1.1 | 82.1 | 11.3 |
| 7b3357-3 | 3.9 | 1.5 | 82.1 | 10.5 |
| 7b3092-2 | 4.3 | 1.9 | 82.1 | 9.9 |
| 7b3424-3 | 3.2 | 0.8 | 82.1 | 11.7 |
| 7b3318-2 | 3.0 | 1.0 | 82.1 | 11.9 |
| 7b3313-1 | 3.3 | 1.8 | 82.1 | 10.9 |
| 7b3317-2 | 3.3 | 1.6 | 82.0 | 11.2 |
| 7b3511-2 | 4.3 | 1.8 | 82.0 | 10.0 |
| 7b3359-2 | 3.5 | 1.5 | 82.0 | 10.8 |
| 7b3324-2 | 3.3 | 1.5 | 82.0 | 11.3 |
| 7b3511-2 | 4.3 | 1.8 | 82.0 | 10.0 |
| 7b3764-1 | 3.7 | 1.1 | 82.0 | 11.1 |
| 7b3165-2 | 3.3 | 0.9 | 82.0 | 11.6 |
| 7b2001-4 | 3.4 | 1.9 | 82.0 | 10.5 |
| 7b3241-2 | 4.0 | 2.0 | 82.0 | 10.5 |
| 7b3308-2 | 3.3 | 1.3 | 82.0 | 12.0 |
| 7b3527-2 | 3.9 | 1.2 | 82.0 | 10.9 |
| 7b3883-3 | 4.6 | 1.7 | 82.0 | 9.7 |
| 7b3308-1 | 3.2 | 1.3 | 82.0 | 12.0 |
| 7b3076-10 | 3.2 | 1.8 | 81.9 | 11.3 |
| 7b3348-1 | 3.5 | 1.6 | 81.9 | 10.9 |
| 7b3341-2 | 3.4 | 2.1 | 81.9 | 9.9 |
| 7b3619-2 | 3.5 | 1.3 | 81.9 | 11.6 |
| 7b3316-2 | 3.4 | 1.7 | 81.9 | 11.2 |
| 7b2386-1 | 3.8 | 1.9 | 81.9 | 10.4 |
| 7b3315-1 | 3.0 | 1.5 | 81.9 | 11.6 |
| 7b3409-3 | 3.6 | 1.6 | 81.9 | 10.5 |
| 7b3313-2 | 3.4 | 1.6 | 81.9 | 11.4 |
| 7b2385-1 | 3.8 | 1.9 | 81.9 | 10.2 |
| 7b3539-1 | 4.1 | 1.3 | 81.9 | 10.5 |
| 7b3414-1 | 3.7 | 1.5 | 81.9 | 10.8 |
| 7b3175-1 | 3.3 | 1.0 | 81.9 | 11.4 |

### Example 6

### 1988 Breeding Program and Results

These 303 lines were selfed in 1988 and the seed analyzed for oleic acid production. A total of 352 lines were selected after the 1988 season which produced seeds having oleic acid content greater than 80%. The range in oleic acid content of these lines was 83.4% to 86.1%. The linoleic acid content ranged from 7.4% to 10.4% and the saturated fatty acid content ranged from 4.7% to 5.8%, with six lines up to 6.5%. The ratio of saturated fatty acids to oleic acid ranged from 0.055 to 0.070, with three lines up to 0.075. Twenty-two of these lines produced seeds having an oleic acid content of greater than 85%. The mean temperatures for July and August were 73.7°F and about 70.6°F, respectively. The results are shown in Table 6.

| FIELD ENTRY | % PALMITIC | % STEARIC | % OLEIC | % LINOLEIC |
|---|---|---|---|---|
| # | 16:0 | 18:0 | 18:1 | 18:2 |
| MB8-5293-1m | 3.5 | 1.3 | 86.1 | 7.6 |
| MB8-5360-1m | 3.6 | 1.3 | 85.4 | 7.9 |
| MB8-5486-1m | 3.6 | 1.3 | 85.4 | 8.0 |
| MB8-5294-1m | 3.6 | 1.2 | 85.3 | 8.4 |
| MB8-5485-1m | 3.7 | 1.2 | 85.3 | 8.0 |
| MB8-5383-1m | 3.8 | 1.3 | 85.3 | 7.9 |
| MB8-5359-1m | 3.6 | 1.2 | 85.2 | 8.2 |
| MB8-5318-1m | 3.9 | 1.5 | 85.2 | 7.6 |
| MB8-5314-1m | 3.9 | 1.4 | 85.2 | 7.7 |
| MB8-5183-1m | 3.8 | 1.3 | 85.2 | 7.8 |
| MB8-5360-1m | 3.6 | 1.2 | 85.2 | 8.2 |
| MB8-5271-1m | 3.8 | 1.2 | 85.2 | 7.9 |
| MB8-5297-1m | 3.9 | 1.6 | 85.1 | 7.4 |
| MB8-5297-1m | 3.8 | 1.6 | 85.1 | 7.4 |
| MB8-5198-1m | 4.0 | 1.4 | 85.0 | 7.9 |
| MB8-4249-1m | 3.5 | 1.2 | 85.0 | 8.8 |
| MB8-5268-1m | 3.9 | 1.5 | 85.0 | 7.8 |
| MB8-4341-1m | 3.7 | 1.2 | 85.0 | 8.4 |
| MB8-5359-1m | 3.7 | 1.2 | 85.0 | 8.6 |
| MB8-5197-1m | 3.8 | 1.3 | 85.0 | 8.3 |
| MB8-5374-1m | 3.8 | 1.3 | 85.0 | 8.1 |
| MB8-4426-1m | 3.5 | 1.2 | 85.0 | 8.8 |
| MB8-5317-1m | 3.8 | 1.2 | 84.9 | 8.5 |
| MB8-4431-1m | 3.7 | 1.2 | 84.9 | 8.5 |
| 88b-5514-1 | 3.8 | 1.2 | 84.9 | 8.3 |
| 88b-5646-1 | 3.7 | 1.1 | 84.8 | 8.8 |
| 88b-5588-1 | 3.8 | 1.5 | 84.8 | 8.2 |
| MB8-5309-2m | 3.6 | 1.5 | 84.8 | 8.5 |
| MB8-5190-1m | 3.8 | 1.3 | 84.8 | 8.3 |
| MB8-4412-1m | 3.7 | 1.2 | 84.8 | 8.5 |
| MB8-5387-1m | 4.0 | 1.5 | 84.8 | 8.2 |
| MB8-5273-1m | 3.7 | 1.3 | 84.8 | 8.5 |
| MB8-5308-2m | 3.6 | 1.2 | 84.8 | 9.0 |
| MB8-5310-1m | 3.8 | 1.2 | 84.8 | 8.6 |
| MB8-5360-4m | 3.6 | 1.3 | 84.8 | 8.9 |
| MB8-5217-1m | 3.7 | 1.3 | 84.8 | 8.5 |
| MB8-5370-1m | 3.9 | 1.4 | 84.8 | 7.7 |
| MB8-5298-1m | 3.8 | 1.2 | 84.8 | 8.9 |
| MB8-5372-1m | 3.9 | 1.6 | 84.7 | 7.7 |
| MB8-4732-1m | 3.7 | 1.4 | 84.7 | 8.2 |
| MB8-5288-1m | 3.6 | 1.6 | 84.7 | 8.6 |
| 88b-5603-1 | 3.8 | 1.2 | 84.7 | 8.5 |
| MB8-5189-1m | 3.8 | 1.2 | 84.7 | 8.8 |
| 88b-5529-1 | 3.8 | 1.5 | 84.7 | 8.4 |
| MB8-5401-1m | 3.8 | 1.8 | 84.7 | 7.9 |
| MB8-4993-1m | 3.9 | 1.4 | 84.7 | 8.6 |
| MB8-5392-1m | 3.8 | 1.6 | 84.7 | 8.3 |
| MB8-5373-1m | 3.7 | 1.3 | 84.7 | 8.1 |
| MB8-4823-1m | 4.2 | 1.6 | 84.7 | 8.0 |
| MB8-5292-1m | 3.8 | 1.5 | 84.7 | 8.3 |
| 88b-5593-1 | 3.9 | 1.6 | 84.7 | 8.2 |
| MB8-5302-2m | 3.6 | 1.6 | 84.6 | 8.5 |
| MB8-4502-1m | 3.6 | 1.3 | 84.6 | 9.0 |
| MB8-5291-1m | 3.9 | 1.6 | 84.6 | 8.1 |
| MB8-4428-1m | 3.6 | 1.3 | 84.6 | 8.7 |
| MB8-4256-1m | 3.8 | 1.1 | 84.6 | 8.6 |
| MB8-5304-3m | 3.6 | 1.2 | 84.6 | 9.0 |
| MB8-5364-1m | 3.7 | 1.4 | 84.6 | 8.4 |
| MB8-5395-1m | 3.6 | 1.5 | 84.6 | 8.7 |
| MB8-4797-1m | 3.8 | 1.5 | 84.5 | 8.7 |
| MB8-5228-1m | 4.0 | 1.5 | 84.5 | 8.4 |
| MB8-5301-1m | 3.7 | 1.6 | 84.5 | 8.6 |
| MB8-5137-1m | 4.0 | 1.4 | 84.5 | 8.6 |
| MB8-4731-1m | 3.9 | 1.5 | 84.5 | 8.0 |
| MB8-5193-1m | 3.9 | 1.3 | 84.5 | 8.5 |
| MB8-5303-1m | 3.8 | 1.7 | 84.5 | 8.3 |
| 88b-5641-1 | 3.7 | 1.2 | 84.4 | 9.0 |
| MB8-5272-1m | 3.9 | 1.6 | 84.4 | 8.5 |
| MB8-4743-1m | 3.9 | 1.3 | 84.4 | 8.6 |
| MB8-5382-1m | 3.9 | 1.2 | 84.4 | 8.6 |
| MB8-4414-1m | 3.9 | 1.2 | 84.4 | 8.6 |
| MB8-5202-1m | 3.7 | 1.2 | 84.4 | 9.2 |
| MB8-4264-1m | 3.4 | 1.2 | 84.4 | 9.1 |
| 88b-6295-1 | 3.7 | 1.6 | 84.4 | 8.8 |
| MB8-5204-1m | 3.7 | 1.2 | 84.4 | 9.1 |
| MB8-5478-1m | 3.7 | 1.2 | 84.4 | 8.8 |
| MB8-4437-1m | 3.8 | 1.3 | 84.4 | 8.7 |
| MB8-5326-1m | 4.1 | 1.3 | 84.3 | 8.6 |
| MB8-5388-1m | 3.9 | 1.4 | 84.3 | 8.7 |
| MB8-4608-1m | 3.8 | 1.5 | 84.3 | 8.5 |
| MB8-5365-1m | 3.7 | 1.5 | 84.3 | 8.4 |
| MB8-5227-1m | 3.9 | 1.6 | 84.3 | 8.7 |
| MB8-5361-1m | 3.7 | 1.2 | 84.3 | 9.1 |
| MB8-5086-1m | 3.9 | 1.5 | 84.3 | 8.9 |
| MB8-4415-1m | 3.9 | 1.3 | 84.3 | 8.4 |
| MB8-5282-1m | 3.5 | 1.2 | 84.3 | 9.3 |
| MB8-5082-1m | 4.0 | 1.3 | 84.3 | 9.2 |
| MB8-5184-1m | 3.8 | 1.4 | 84.3 | 8.9 |
| MB8-4404-1m | 3.7 | 1.2 | 84.3 | 9.0 |
| MB8-4729-1m | 4.1 | 1.3 | 84.2 | 8.2 |
| MB8-5195-1m | 4.1 | 1.3 | 84.2 | 8.6 |
| MB8-4416-1m | 3.6 | 1.2 | 84.2 | 9.1 |
| MB8-5375-1m | 4.0 | 1.6 | 84.2 | 8.2 |
| MB8-5362-1m | 3.6 | 1.3 | 84.2 | 9.1 |
| MB8-5270-1m | 3.8 | 1.4 | 84.2 | 9.1 |
| 88b-6019-5 | 3.8 | 1.6 | 84.2 | 8.8 |
| MB8-5258-1m | 3.8 | 1.7 | 84.2 | 8.8 |
| MB8-4421-1m | 3.8 | 1.1 | 84.2 | 9.2 |
| MB8-5246-1m | 4.0 | 1.6 | 84.2 | 8.7 |
| MB8-5201-1m | 3.7 | 1.3 | 84.2 | 8.8 |
| MB8-5360-3m | 3.8 | 1.2 | 84.2 | 8.9 |
| MB8-5313-1m | 3.6 | 1.2 | 84.2 | 9.4 |
| 88b-5504-1 | 4.0 | 1.2 | 84.2 | 8.9 |
| MB8-5359-4m | 3.7 | 1.2 | 84.2 | 9.6 |
| MB8-5192-1m | 3.9 | 1.2 | 84.2 | 9.1 |
| MB8-4435-1m | 3.7 | 1.2 | 84.1 | 9.1 |
| MB8-5386-1m | 3.8 | 1.2 | 84.1 | 9.2 |
| MB8-4990-1m | 3.9 | 1.5 | 84.1 | 9.1 |
| MB8-5203-1m | 3.7 | 1.2 | 84.1 | 9.4 |
| 88b-6019-1 | 3.7 | 1.5 | 84.1 | 9.0 |
| MB8-5209-1m | 3.9 | 1.3 | 84.1 | 8.9 |
| MB8-4248-1m | 3.7 | 1.1 | 84.1 | 9.2 |
| MB8-4423-1m | 3.6 | 1.1 | 84.1 | 9.5 |
| MB8-5368-1m | 3.7 | 1.3 | 84.1 | 9.0 |
| MB8-4208-1m | 3.7 | 1.3 | 84.1 | 9.0 |
| 88b-6014-3 | 3.6 | 1.4 | 84.1 | 9.3 |
| 88b-5499-1 | 3.8 | 1.3 | 84.1 | 8.7 |
| MB8-5354-1m | 3.8 | 1.5 | 84.1 | 0.9 |
| MB8-4402-1m | 3.5 | 1.2 | 84.1 | 9.2 |
| MB8-5400-1m | 3.9 | 1.8 | 84.0 | 8.3 |
| MB8-5078-1m | 4.0 | 1.5 | 84.0 | 9.4 |
| MB8-5360-2m | 3.5 | 1.1 | 84.0 | 9.8 |
| MB8-4197-1m | 4.0 | 1.4 | 84.0 | 8.9 |
| MB8-5359-3m | 3.8 | 1.2 | 34.0 | 9.4 |
| MB8-4250-1m | 3.6 | 1.0 | 84.0 | 9.7 |
| MB8-5385-1m | 4.2 | 1.2 | 84.0 | 8.7 |
| MB8-5324-1m | 3.7 | 1.3 | 84.0 | 9.3 |
| MB8-5381-1m | 4.0 | 1.5 | 84.0 | 8.4 |
| MB8-5269-1m | 4.1 | 1.4 | 84.0 | 8.8 |
| MB8-5317-1m | 3.7 | 1.2 | 84.0 | 9.4 |
| 88b-5650-1 | 3.9 | 1.1 | 84.0 | 9.1 |
| MB8-5376-1m | 3.9 | 1.5 | 84.0 | 3.7 |
| MB8-4247-1m | 3.7 | 1.1 | 84.0 | 9.6 |
| MB8-5371-1m | 3.7 | 1.3 | 84.0 | 8.9 |
| MB8-5083-2m | 3.8 | 1.5 | 84.0 | 9.2 |
| MB8-5321-1m | 4.0 | 1.5 | 84.0 | 9.0 |
| MB8-4446-1m | 3.7 | 1.1 | 84.0 | 9.5 |
| 88b-5660-1 | 3.9 | 1.4 | 84.0 | 8.9 |
| MB8-5284-1m | 3.7 | 1.3 | 83.9 | 9.8 |
| MB8-5281-1m | 3.8 | 1.2 | 83.9 | 9.7 |
| 88b-5509-1 | 3.9 | 1.1 | 83.9 | 9.2 |
| MB8-5479-1m | 3.8 | 1.3 | 83.9 | 9.3 |
| MB8-5133-1m | 4.0 | 1.3 | 83.9 | 8.9 |
| 88b-6019-4 | 3.7 | 1.6 | 83.9 | 9.1 |
| MB8-5136-1m | 4.0 | 1.3 | 83.9 | 9.0 |
| MB8-4122-1m | 3.7 | 1.1 | 83.9 | 9.5 |
| MB8-5214-1m | 4.0 | 1.2 | 83.9 | 9.4 |
| 88b-5637-1 | 3.8 | 1.5 | 83.9 | 9.2 |
| MB8-4424-1m | 3.6 | 1.3 | 83.9 | 9.5 |
| MB8-5366-1m | 3.8 | 1.3 | 83.9 | 9.0 |
| MB8-4737-1m | 3.7 | 1.3 | 83.9 | 9.2 |
| MB8-5476-1m | 3.8 | 1.5 | 83.9 | 8.7 |
| MB8-5305-2m | 4.1 | 1.7 | 83.9 | 8.7 |
| MB8-4445-1m | 3.8 | 1.3 | 83.9 | 9.1 |
| MB8-5306-1m | 3.8 | 1.5 | 83.9 | 9.4 |
| 88b-5519-1 | 3.9 | 1.4 | 83.9 | 8.8 |
| MB8-4452-1m | 3.7 | 1.2 | 83.9 | 9.5 |
| MB8-5379-1m | 3.9 | 1.4 | 83.9 | 8.7 |
| MB8-4450-1m | 3.7 | 1.3 | 83.9 | 8.8 |
| 88b-6053-1 | 3.8 | 1.6 | 83.9 | 8.9 |
| MB8-5477-1m | 3.7 | 1.3 | 83.9 | 9.2 |
| 88b-5655-1 | 3.8 | 1.1 | 83.9 | 9.4 |
| MB8-4534-1m | 3.7 | 1.5 | 83.9 | 9.3 |
| MB8-4596-1m | 3.7 | 1.6 | 83.8 | 9.2 |
| MB8-5132-1m | 4.2 | 1.6 | 83.8 | 8.7 |
| MB8-4448-1m | 3.7 | 1.1 | 83.8 | 9.3 |
| 88b-6017-3 | 3.6 | 1.6 | 83.8 | 9.5 |
| MB8-4345-1m | 3.9 | 1.8 | 83.8 | 8.7 |
| 88b-6018-5 | 3.8 | 1.5 | 83.8 | 9.3 |
| MB8-5185-1m | 3.9 | 1.7 | 83.8 | 8.9 |
| MB8-4401-1m | 3.5 | 1.4 | 83.8 | 9.2 |
| 88b-6220-1 | 3.5 | 1.8 | 83.8 | 9.3 |
| 88b-6023-2 | 3.5 | 1.4 | 83.8 | 9.5 |
| MB8-5187-1m | 3.8 | 1.3 | 83.7 | 9.7 |
| 88b-6013-5 | 3.9 | 1.5 | 83.7 | 9.4 |
| MB8-5325-1m | 4.1 | 1.6 | 83.7 | 9.2 |
| 88b-6014-4 | 3.7 | 1.5 | 83.7 | 9.5 |
| MB8-5265-1m | 3.8 | 1.6 | 83.7 | 9.3 |
| 88b-6021-3 | 3.6 | 1.4 | 83.7 | 9.7 |
| MB8-5135-1m | 4.1 | 1.5 | 83.7 | 8.9 |
| 88b-6025-3 | 3.5 | 1.6 | 83.7 | 9.6 |
| MB8-5367-1m | 3.7 | 1.3 | 83.7 | 9.2 |
| 88b-6035-2 | 3.8 | 1.5 | 83.7 | 9.3 |
| 88b-6359-1 | 3.7 | 1.4 | 83.7 | 9.6 |
| MB8-4733-1m | 4.0 | 1.3 | 83.7 | 9.0 |
| MB8-5316-1m | 3.9 | 1.2 | 83.7 | 9.7 |
| MB8-4354-1m | 4.8 | 2.1 | 83.7 | 8.0 |
| MB8-5384-1m | 3.6 | 1.3 | 83.7 | 9.7 |
| MB8-4582-1m | 3.5 | 1.5 | 83.7 | 9.5 |
| 88b-6178-1 | 3.6 | 1.5 | 83.7 | 9.4 |
| 88b-6462-1 | 3.6 | 1.7 | 83.7 | 9.4 |
| 88b-6170-1 | 3.6 | 1.5 | 83.7 | 9.5 |
| MB8-4457-1m | 3.9 | 1.5 | 83.7 | 9.3 |
| MB8-5380-1m | 3.7 | 1.3 | 83.6 | 9.5 |
| 88b-6469-1 | 3.6 | 1.6 | 83.6 | 9.4 |
| MB8-4201-1m | 3.9 | 1.4 | 83.6 | 9.4 |
| 88b-6458-1 | 3.8 | 1.7 | 83.6 | 9.1 |
| MB8-5327-1m | 4.1 | 1.2 | 83.6 | 9.5 |
| 88b-6341-1 | 3.6 | 1.5 | 83.6 | 9.7 |
| MB8-5231-1m | 3.7 | 1.6 | 83.6 | 9.5 |
| 88b-6257-1 | 3.8 | 1.5 | 83.6 | 9.6 |
| MB8-5216-1m | 4.0 | 1.3 | 83.6 | 9.2 |
| 88b-6052-1 | 3.8 | 1.5 | 83.6 | 9.4 |
| MB8-5425-1m | 4.4 | 1.7 | 83.6 | 8.5 |
| 88b-6121-1 | 3.6 | 1.6 | 83.6 | 9.6 |
| MB8-5397-1m | 3.9 | 1.4 | 83.6 | 9.5 |
| 88b-6027-3 | 3.6 | 1.5 | 83.6 | 9.6 |
| MB8-5357-1m | 4.0 | 1.4 | 83.6 | 9.4 |
| 88b-6025-2 | 3.6 | 1.5 | 83.6 | 9.5 |
| 88b-6481-1 | 3.7 | 1.5 | 83.6 | 9.5 |
| 88b-6020-4 | 3.5 | 1.5 | 83.6 | 9.6 |
| MB8-5346-1m | 4.1 | 1.4 | 83.6 | 9.1 |
| 88b-6015-5 | 3.8 | 1.6 | 83.6 | 9.4 |
| MB8-5222-1m | 3.9 | 1.2 | 83.6 | 10.0 |
| MB8-4351-1m | 4.4 | 1.9 | 83.6 | 8.4 |
| MB8-5414-1m | 4.0 | 1.4 | 83.6 | 9.4 |
| MB8-4798-1m | 3.9 | 1.4 | 83.6 | 9.5 |
| MB8-5348-1m | 4.0 | 1.4 | 83.6 | 9.4 |
| MB8-4436-1m | 3.7 | 1.3 | 83.6 | 9.5 |
| MB8-5243-1m | 3.8 | 1.6 | 83.6 | 9.3 |
| MB8-4405-1m | 3.9 | 1.2 | 83.6 | 9.6 |
| MB8-5369-1m | 3.7 | 1.3 | 83.6 | 9.2 |
| MB8-5146-1m | 3.6 | 1.2 | 83.6 | 9.9 |
| 88b-6473-1 | 3.7 | 1.7 | 83.6 | 9.0 |
| MB8-4549-1m | 3.8 | 1.4 | 83.6 | 9.7 |
| MB8-4123-1m | 3.9 | 1.2 | 83.5 | 9.4 |
| 88b-6471-1 | 3.9 | 1.7 | 83.5 | 9.2 |
| 88b-6479-1 | 3.7 | 1.6 | 83.5 | 9.6 |
| MB8-5434-1m | 4.1 | 2.1 | 83.5 | 8.5 |
| MB8-5407-1m | 4.2 | 1.5 | 83.5 | 9.2 |
| 88b-6036-5 | 3.4 | 1.4 | 83.5 | 9.9 |
| MB8-5394-1m | 3.7 | 1.5 | 83.5 | 9.6 |
| 88b-6028-3 | 3.7 | 1.5 | 83.5 | 9.4 |
| 88b2555-2 | 3.9 | 1.6 | 83.5 | 9.4 |
| 88b-6022-5 | 3.7 | 1.6 | 83.5 | 9.6 |
| MB8-4544-1m | 3.6 | 1.4 | 83.5 | 9.9 |
| 88b-6017-1 | 3.9 | 1.5 | 83.5 | 9.4 |
| 88b-6106-1 | 3.8 | 1.5 | 83.5 | 9.5 |
| 88b-6011-3 | 3.7 | 1.6 | 83.5 | 9.5 |
| 88b-6115-1 | 3.7 | 1.5 | 83.5 | 9.7 |
| 88b1798-1m | 3.6 | 1.4 | 83.5 | 9.8 |
| MB8-4843-1m | 4.3 | 1.6 | 83.5 | 9.1 |
| MB8-5278-1m | 3.7 | 1.1 | 83.5 | 10.2 |
| MB8-4738-1m | 3.7 | 1.2 | 83.5 | 9.7 |
| MB8-5175-1m | 4.3 | 1.4 | 83.5 | 9.1 |
| MB8-4366-1m | 3.7 | 1.1 | 83.5 | 9.9 |
| MB8-5029-1m | 3.9 | 1.5 | 83.5 | 9.3 |
| MB8-5393-1m | 3.8 | 1.4 | 83.5 | 9.6 |
| 88b-6435-1 | 3.9 | 1.5 | 83.5 | 9.6 |
| MB8-5378-1m | 3.8 | 1.1 | 83.5 | 9.4 |
| 88b-6025-4 | 3.6 | 1.6 | 83.5 | 9.0 |
| MB8-5363-1m | 3.8 | 1.3 | 83.5 | 9.4 |
| 88b-6015-2 | 3.7 | 1.5 | 83.5 | 9.7 |
| MB8-5353-1m | 4.0 | 1.5 | 83.5 | 9.4 |
| MB8-5322-1m | 3.9 | 1.4 | 83.5 | 9.7 |
| 88b-6485-1&2 | 3.7 | 1.6 | 83.5 | 9.6 |
| MB8-5154-1m | 3.7 | 1.2 | 83.5 | 10.4 |
| 88b-6464-1 | 3.7 | 1.6 | 83.5 | 9.5 |
| 88b-6033-5 | 3.7 | 1.6 | 83.5 | 9.5 |
| 88b-6131-1 | 3.6 | 1.6 | 83.5 | 9.5 |
| 88b-6010-5 | 3.9 | 1.5 | 83.5 | 9.4 |
| 88b-6091-1 | 3.6 | 1.3 | 83.5 | 9.7 |
| MB8-5359-2m | 3.7 | 1.1 | 83.5 | 10.3 |
| MB8-5267-1m | 3.8 | 1.7 | 83.5 | 9.5 |
| 88b-6018-4 | 3.8 | 1.5 | 83.5 | 9.7 |
| 88b-6323-1 | 3.6 | 1.3 | 83.5 | 9.9 |
| 88b-6027-1 | 3.7 | 1.6 | 83.4 | 9.7 |
| MB8-5358-1m | 3.9 | 1.2 | 83.4 | 9.7 |
| 88b-6383-1 | 3.6 | 1.5 | 83.4 | 9.9 |
| MB8-5398-1m | 4.1 | 1.6 | 83.4 | 9.2 |
| 88b-6023-3 | 3.9 | 1.7 | 83.4 | 9.4 |
| MB8-5423-1m | 4.2 | 1.6 | 83.4 | 9.0 |
| 88b-6302-1 | 3.7 | 1.4 | 83.4 | 9.8 |
| MB8-5480-1m | 4.0 | 1.4 | 83.4 | 9.5 |
| 88b-6130-1 | 3.6 | 1.5 | 83.4 | 9.8 |
| MB8-5178-1m | 4.3 | 1.5 | 83.4 | 8.9 |
| MB8-4203-1m | 3.8 | 1.3 | 83.4 | 9.5 |
| MB8-5241-1m | 4.1 | 1.5 | 83.4 | 9.5 |
| MB8-4275-1m | 4.6 | 1.6 | 83.4 | 8.6 |
| MB8-5245-1m | 3.8 | 1.7 | 83.4 | 9.5 |
| MB8-4403-1m | 3.7 | 1.0 | 83.4 | 9.9 |
| MB8-5253-1m | 4.0 | 1.6 | 83.4 | 9.5 |
| MB8-4641-1m | 4.0 | 2.5 | 83.4 | 8.2 |
| MB8-5266-1m | 3.8 | 1.5 | 83.4 | 9.9 |
| MB8-4510-1m | 3.6 | 1.4 | 83.4 | 10.0 |
| MB8-5283-1m | 3.9 | 1.3 | 83.4 | 9.8 |
| 88b-6426-1 | 3.6 | 1.2 | 83.4 | 10.2 |
| MB8-5328-1m | 4.1 | 1.4 | 83.4 | 9.6 |
| 88b-6475-1 | 3.7 | 1.4 | 83.4 | 9.8 |
| 88b-6002-2 | 3.8 | 1.6 | 83.4 | 9.8 |
| 88b-6051-1 | 3.7 | 1.4 | 83.4 | 9.7 |
| 88b-6010-4 | 3.9 | 1.6 | 83.4 | 9.3 |
| 88b-6179-1 | 3.7 | 1.6 | 83.4 | 9.8 |
| 88b-6011-4 | 3.8 | 1.6 | 83.4 | 9.5 |
| MB8-4513-1m | 3.7 | 1.5 | 83.4 | 9.9 |
| 88b-6012-1 | 3.6 | 1.5 | 83.4 | 9.7 |
| MB8-4393-1m | 3.5 | 1.2 | 83.4 | 10.0 |
| 88b-6012-3 | 3.6 | 1.5 | 83.4 | 9.7 |
| MB8-4417-1m | 3.9 | 1.2 | 83.4 | 9.5 |
| 88b-6013-2 | 3.8 | 1.5 | 83.4 | 9.5 |
| 88b-5548-1 | 4.1 | 1.7 | 83.4 | 9.2 |
| 88b-6014-5 | 3.7 | 1.5 | 83.4 | 9.9 |
| 88b-6127-1 | 3.7 | 1.5 | 83.4 | 9.9 |
| 88b-6015-4 | 3.8 | 1.6 | 83.4 | 9.5 |
| MB8-4589-1m | 3.5 | 1.5 | 83.4 | 9.6 |
| 88b-6016-4 | 3.8 | 1.5 | 83.4 | 9.5 |
| 88b-6364-1 | 3.7 | 1.3 | 83.4 | 9.9 |
| 88b-6449-1 | 3.6 | 1.8 | 83.4 | 9.4 |
| MB8-4453-1m | 3.6 | 1.2 | 83.4 | 10.0 |
| 88b-6018-1 | 3.5 | 1.7 | 83.4 | 9.8 |

These 352 lines were selfed in 1989 and the seed analyzed for oleic acid production. Slight changes seen in the oleic acid content of the progeny with some lines approaching 87%. The mean temperature for July and August was 75.6°F and about 70.8°F, respectively. In order to increase the oleic acid content to greater than 87% and up to 90%, the previously obtained lines are intercrossed to derive new lines. These new lines produce seed having an oleic acid content of up to 90%.

### Example 7

### Uniformity of Oil Production

Several lines were followed between 1985 and 1988 to demonstrate the stability and inheritability of the high oleic acid trait. These lines were grown and the seed harvested and analyzed each year. The results are shown in Table 7.

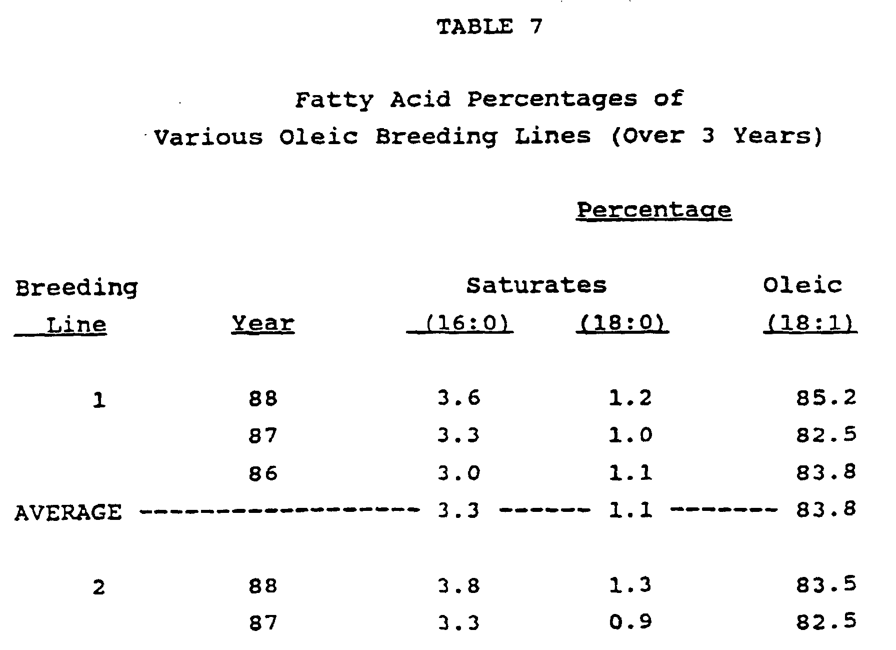

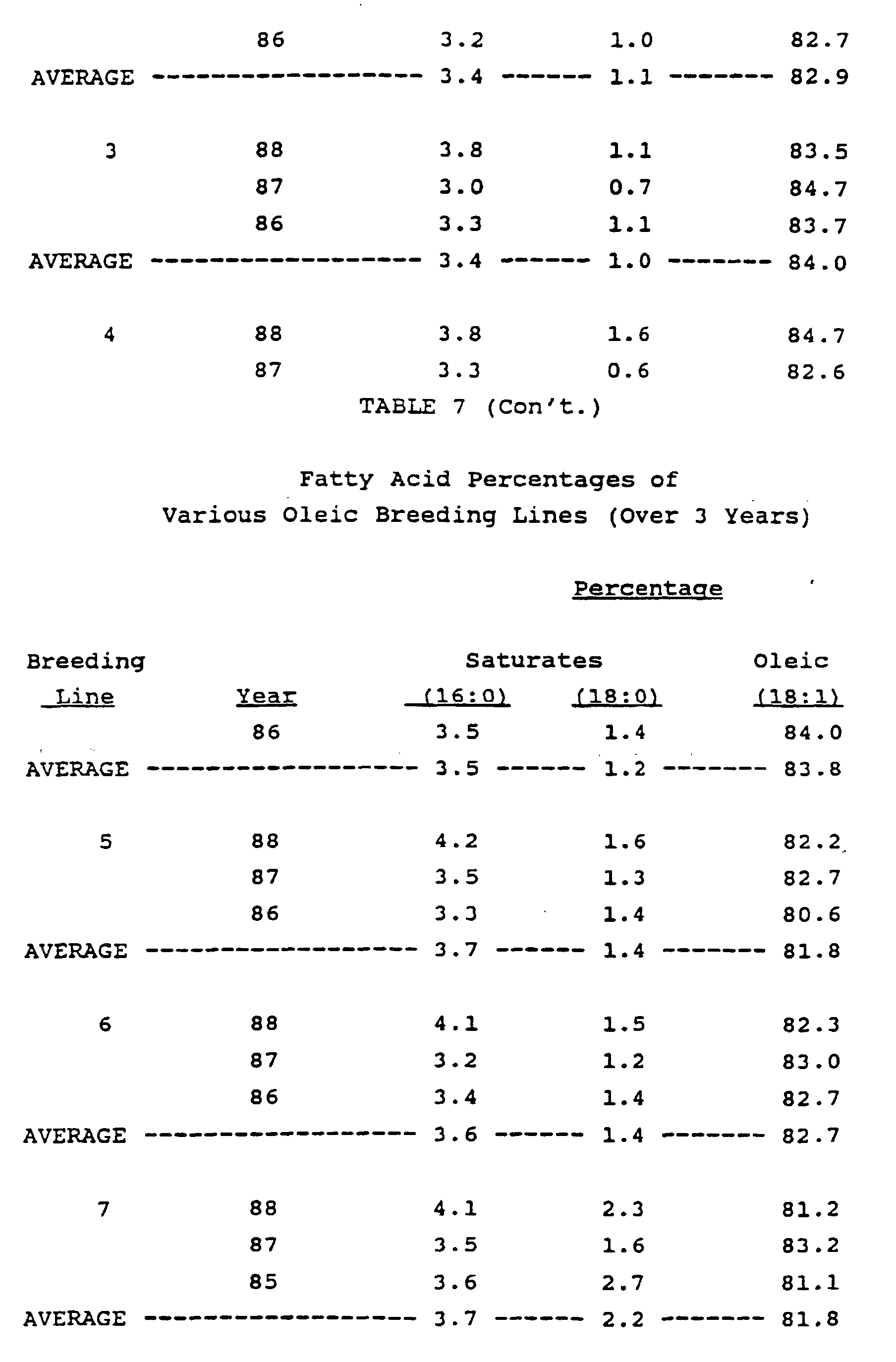

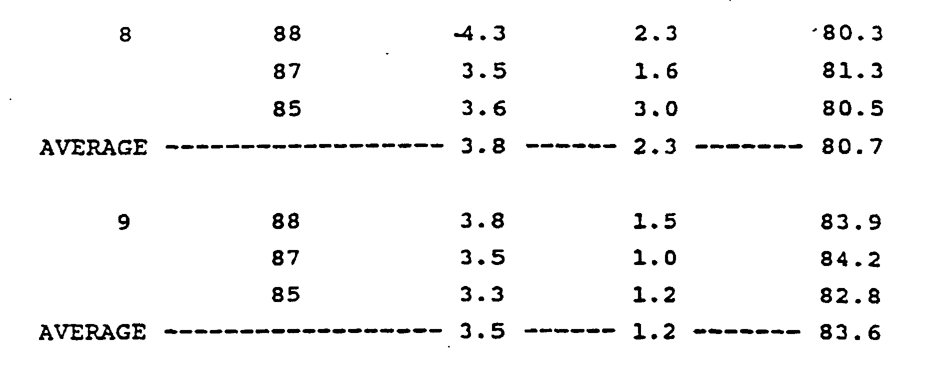

### Example 8

### Breeding Pedigree of Safflower Variety Montola-2000

Montola-2000 was derived from an individual F₆ plant selection from the 1982 cross of a female parent resulting from the multiple cross Frio/Sidney Selection 87-14-6///79AZ9543-1/[S-304/(White US-10/UC-1//Oleic Leed, White S-208//[UC-1/(White US-10/UC-1//Oleic Leed), White S-208]/[Th-5/C. nitidus//ol41-1]] with a male parent resulting from the multiple cross White US-10/UC-1//[S-304/(US-10/UC-1//Oleic leed), White S-208]. From the initial crosses of White flowered US-10/UC-1 and Th-5/ C. nitidus, a total of 19 crosses were made in the breeding of this cultivar. Individual plant selections were made during the F₂, F₃, F₄, F₅ and F₆ generations. Fatty acid determinations were made on seed in the F₆, F₇ and F₈ generations to verify the cultivar produced oil low in saturates and very high in oleic acid (greater than 80%).

The multiple crosses were made in the following scenario.

### Female Parent

cross 1 - Frio x Sidney Selection 87-14-6 (A)
   - White US-10 x UC-1 (B)
   - Th-5 x C. nitidus (C)
cross 2 - (B) progeny x Oleic Leed (D)
   - (C) progeny x ol41-1 (E)
cross 3 - (D) progeny x S-304 (F)
   - (D) progeny x UC-1 (G)
cross 6 - (G) progeny x (E) progeny (H)
cross 10 - (F) progeny x (H) progeny (I)
cross 11 - 79AZ9543-1 x (I) progeny (J)
cross 13 - (A) progeny x (J) progeny (Female Parent)

### Male Parent

cross 1 - US-10 x UC-1 (K)
cross 2 - (K) progeny x Oleic Leed (L)
cross 3 - (L) progeny x S-304 (M)
cross 4 - White US-10 x UC-1 (N)
cross 5 - (N) progeny x (M) progeny (Male Parent)

The objective description of variety Montola-2000 is as follows, in accordance with characteristics for a plant variety application.
Maturity - 120 days (Midwest location)
Plant height at maturity - 43 cm
Flower color - Yellow (fresh);
   Light orange base (wilted)
Spines on involucral bracts - present at tip and along margins; 2.2 mm in length numbering 15 with a spine index of 33 (class 2 = 21-40)

Heads - 25 mm diameter of primary head;
   1-10% seed shattering
Seed - White/white with blotch (4/100); normal hull; 4 mm wide, 7 mm long, 34 g/1000 seeds
Seedling vigor - 8 nodes; 21 cm soil surface to tip
Cold resistance - Rosette: -5°C:
   Bolting: 0°C;
   Flowering: 5°C

Disease - moderately susceptible to Alternaria leaf spot and Alternaria bud rot

Hull - 44.6%
Protein - 23.0%
Oil - 44.0%
Iodine - 84%
Saturated Acids - 5.5%
Oleic acid - 81.7%
Linoleic acid - 11.4%

The fatty acid profile of variety Montola-2000 was examined in 1987, 1988 and 1989. The results are shown in Table 8.

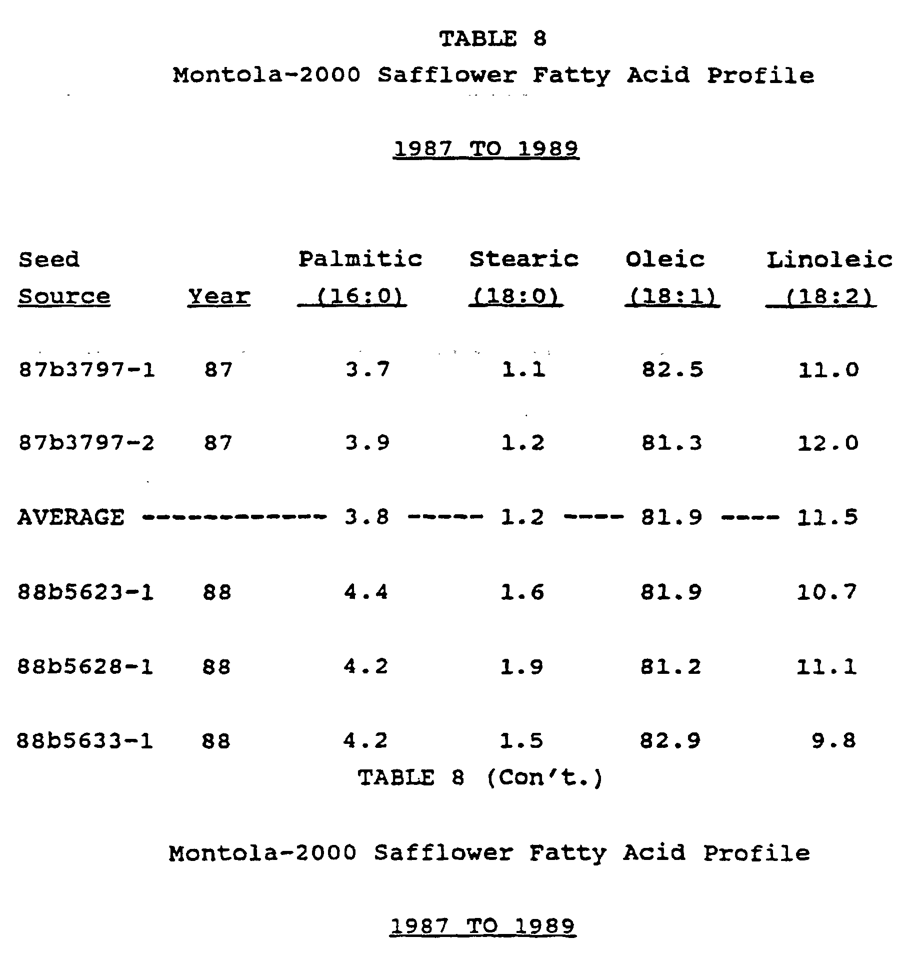

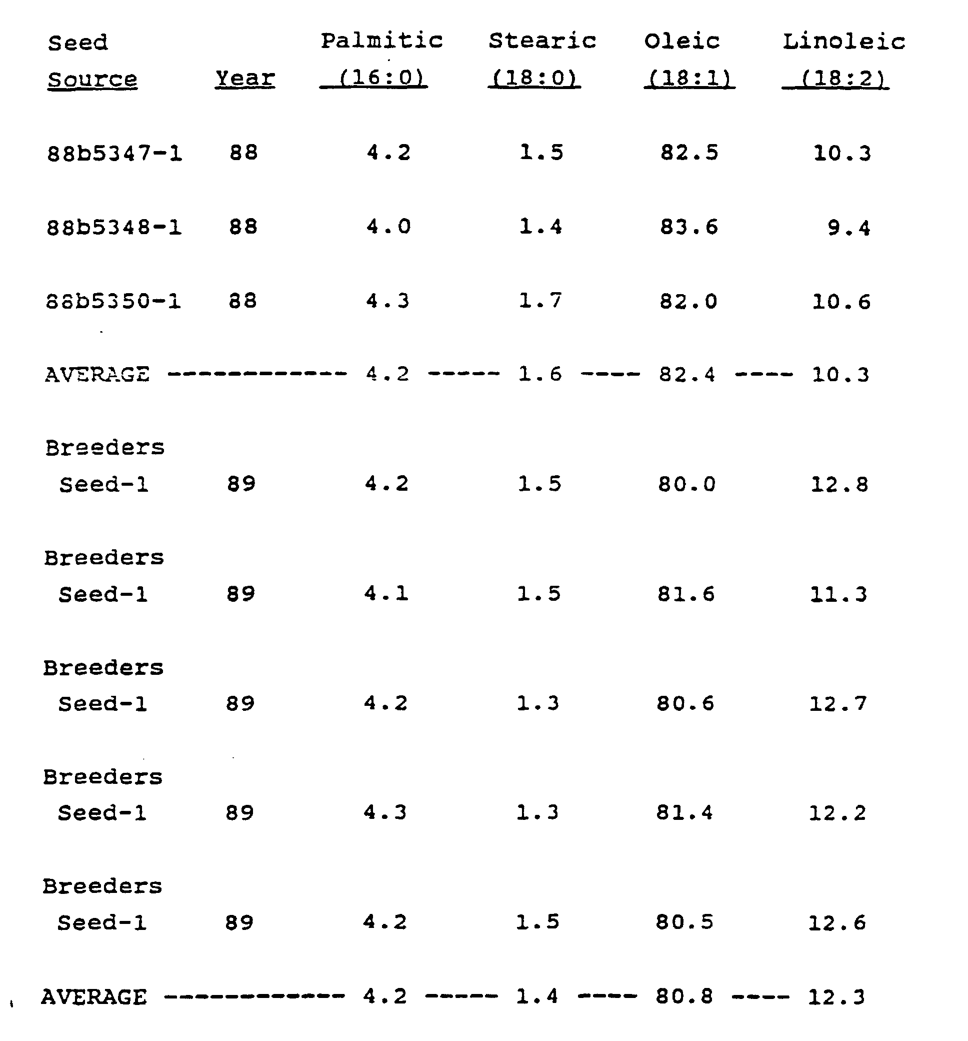

Variety Montola-2000 was compared with several other varieties commonly grown in Montana and North Dakota. The results are shown in Tables 9-19.

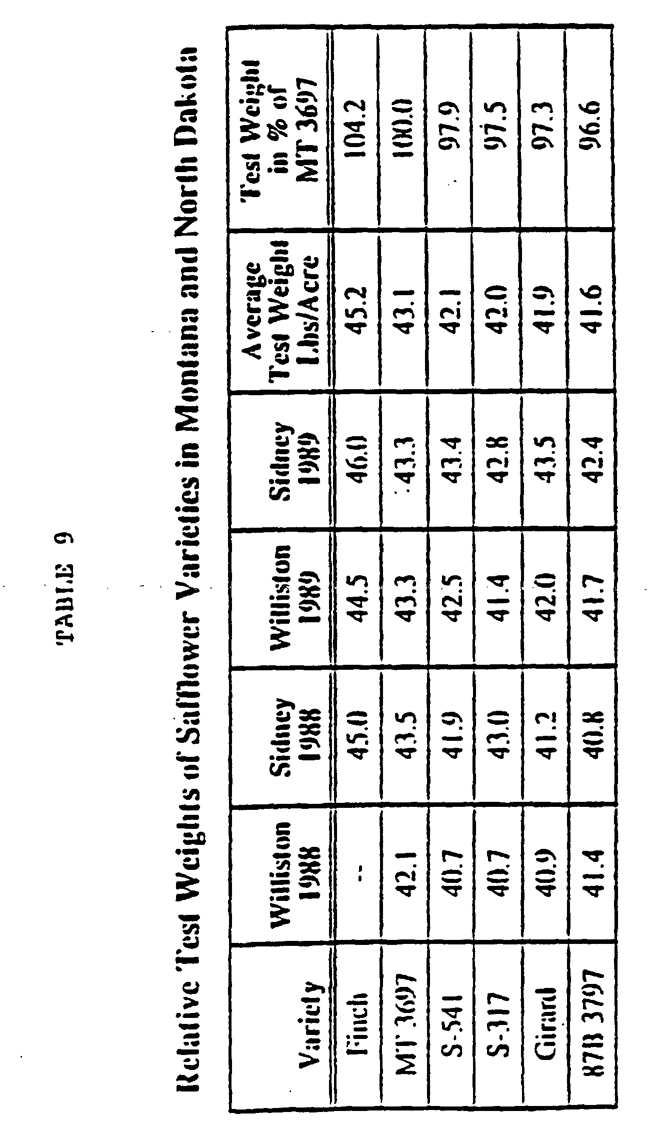

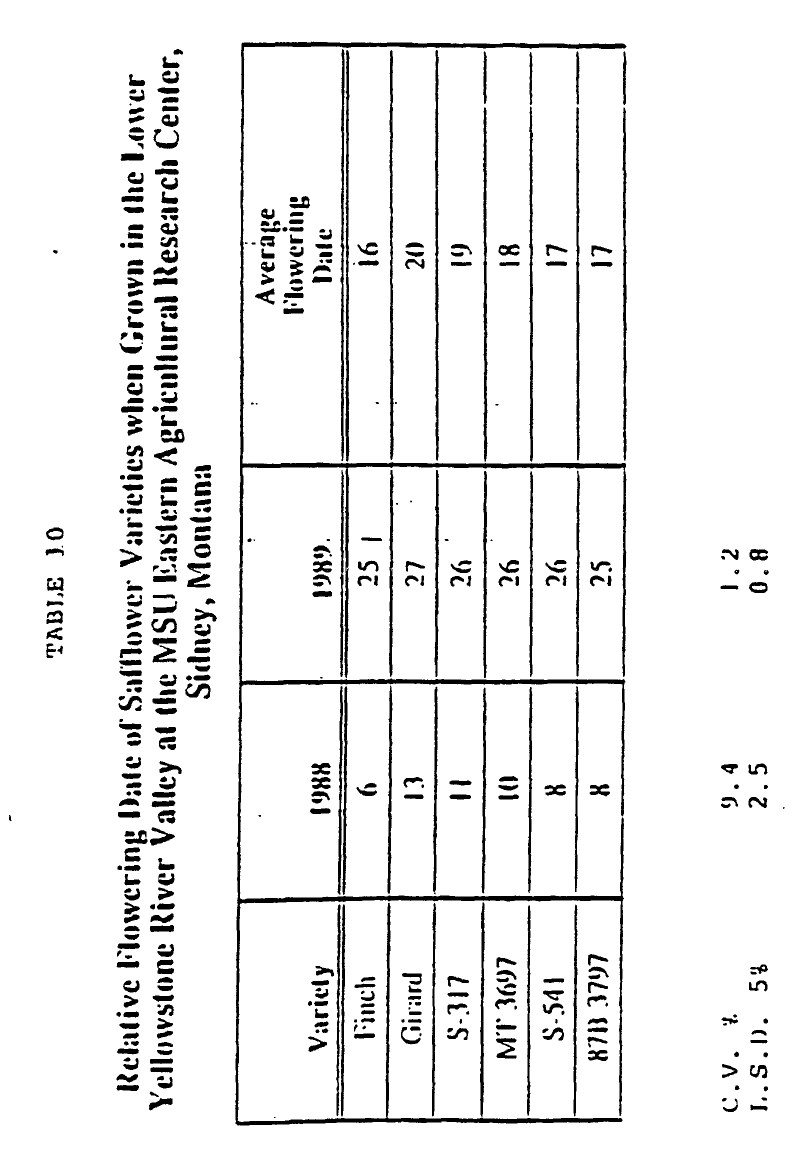

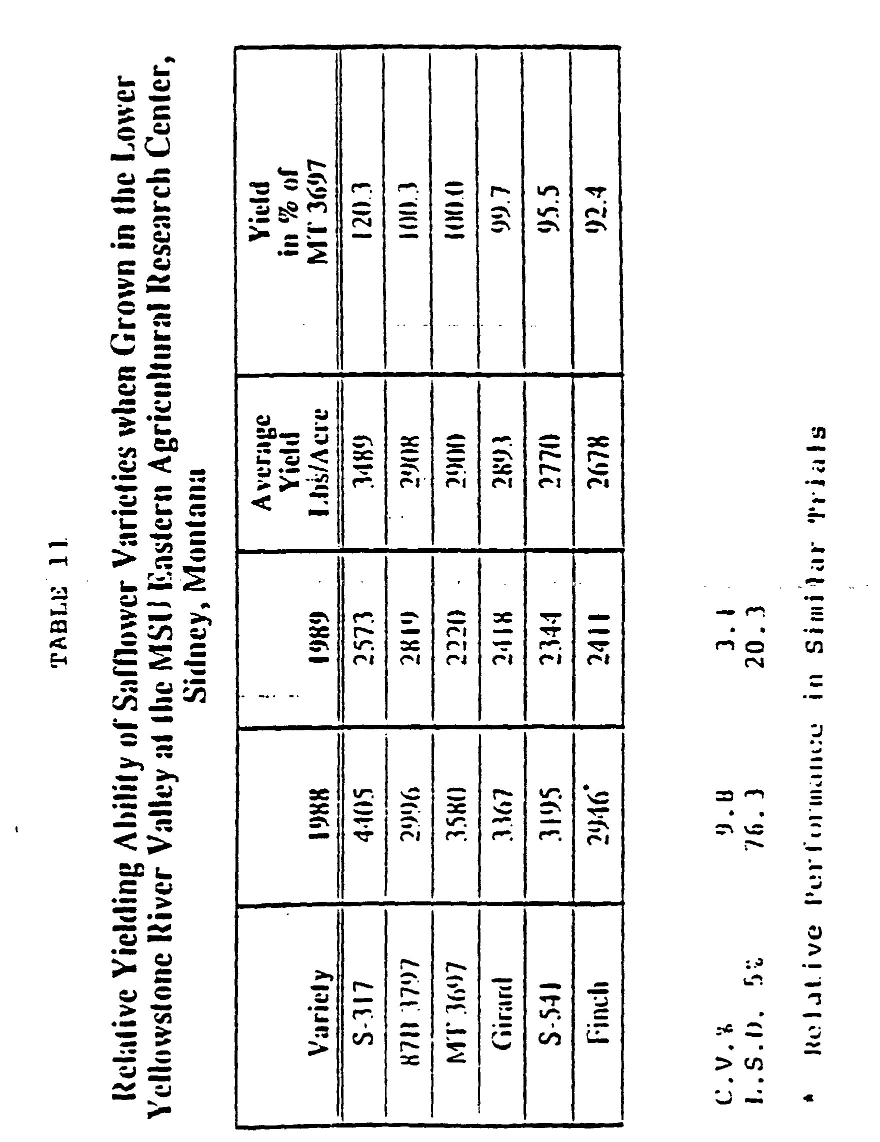

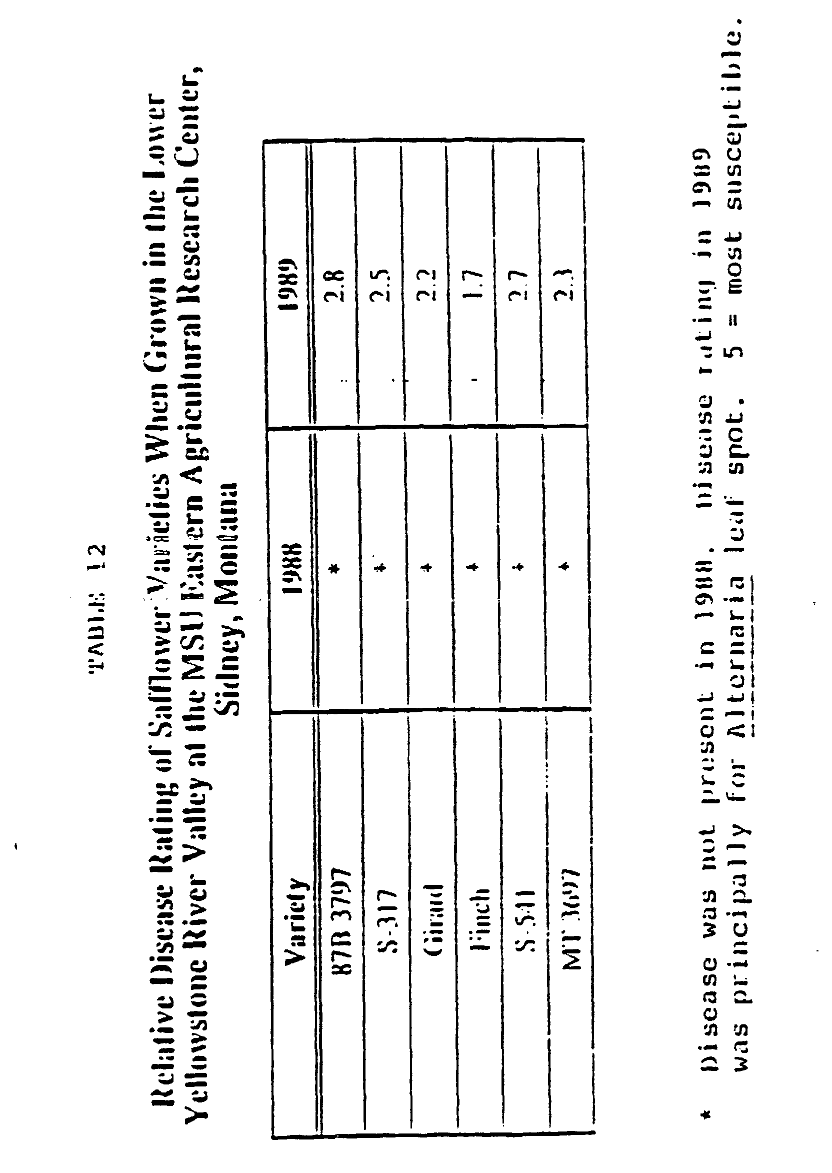

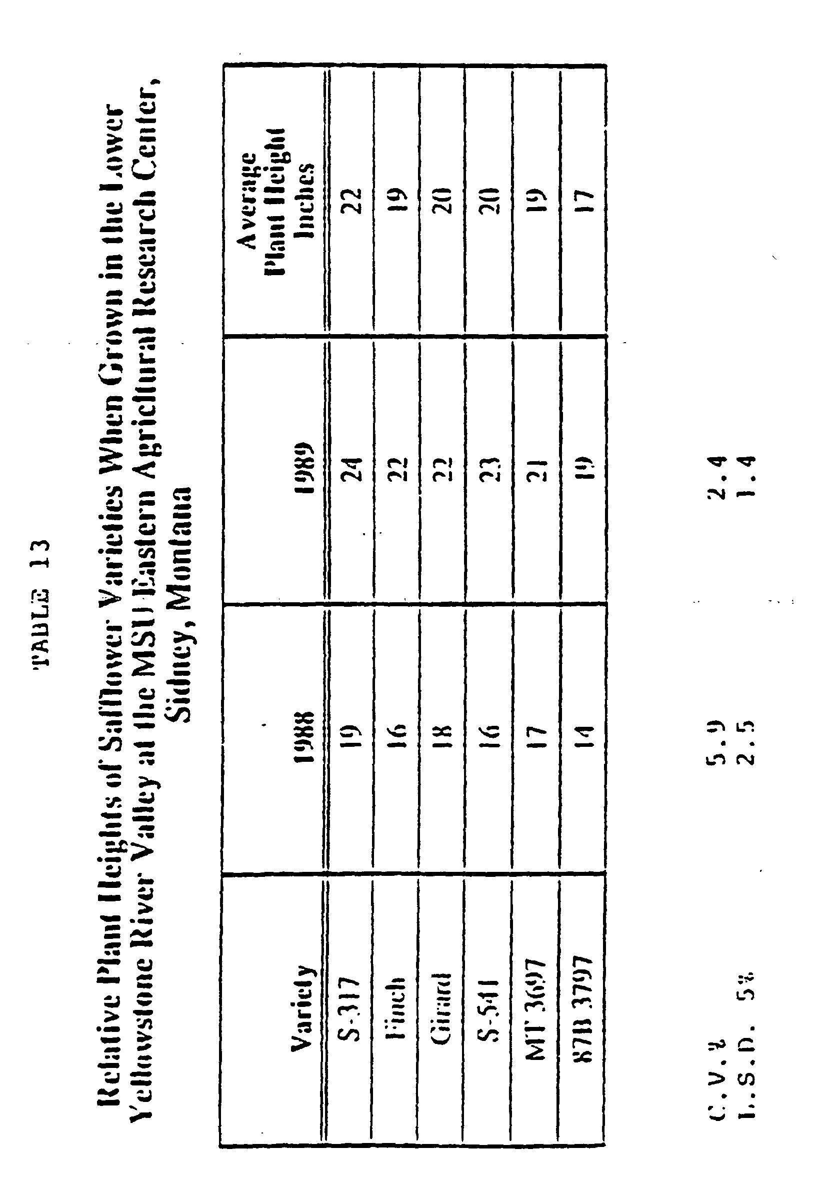

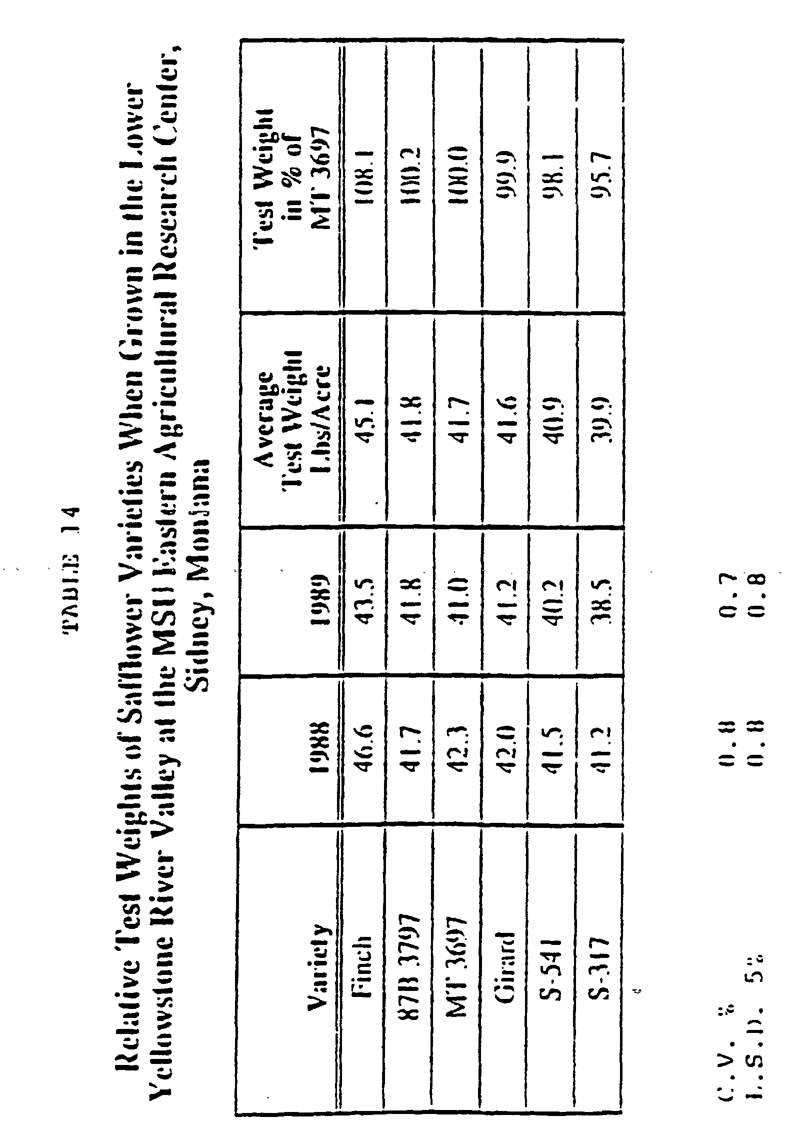

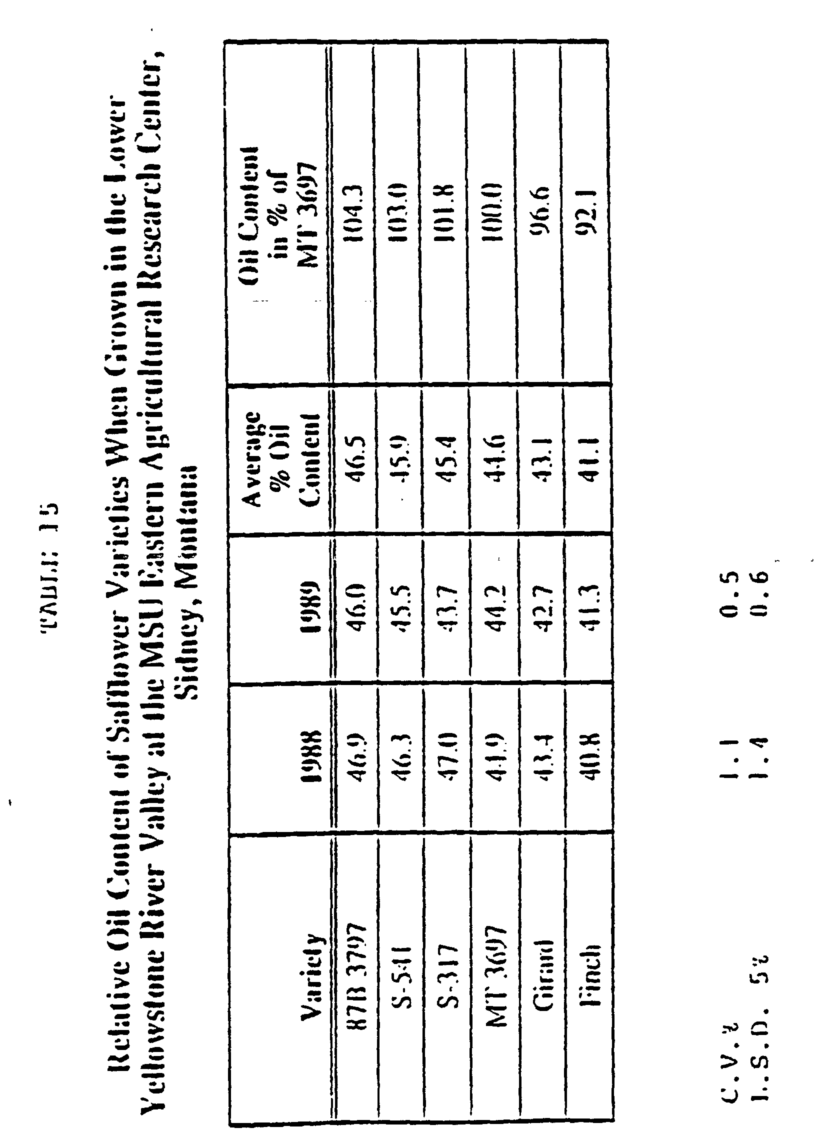

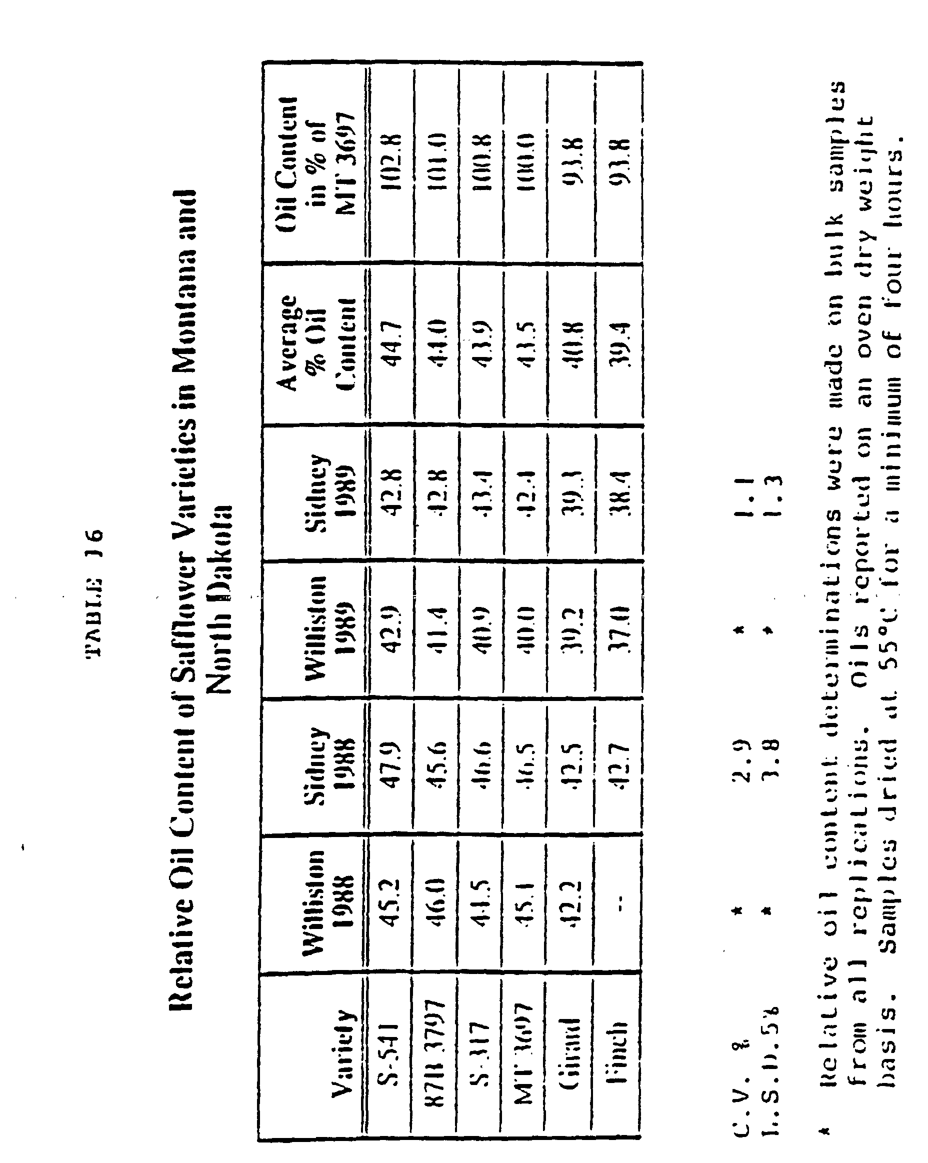

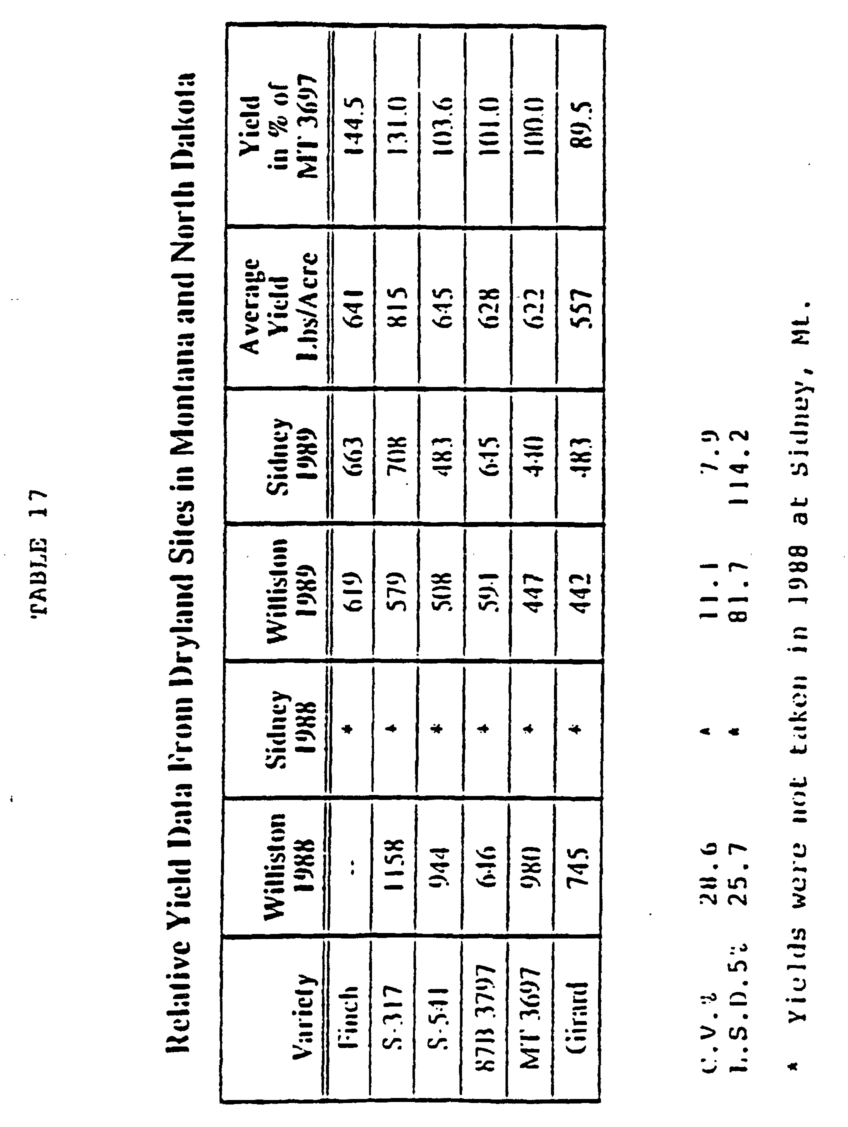

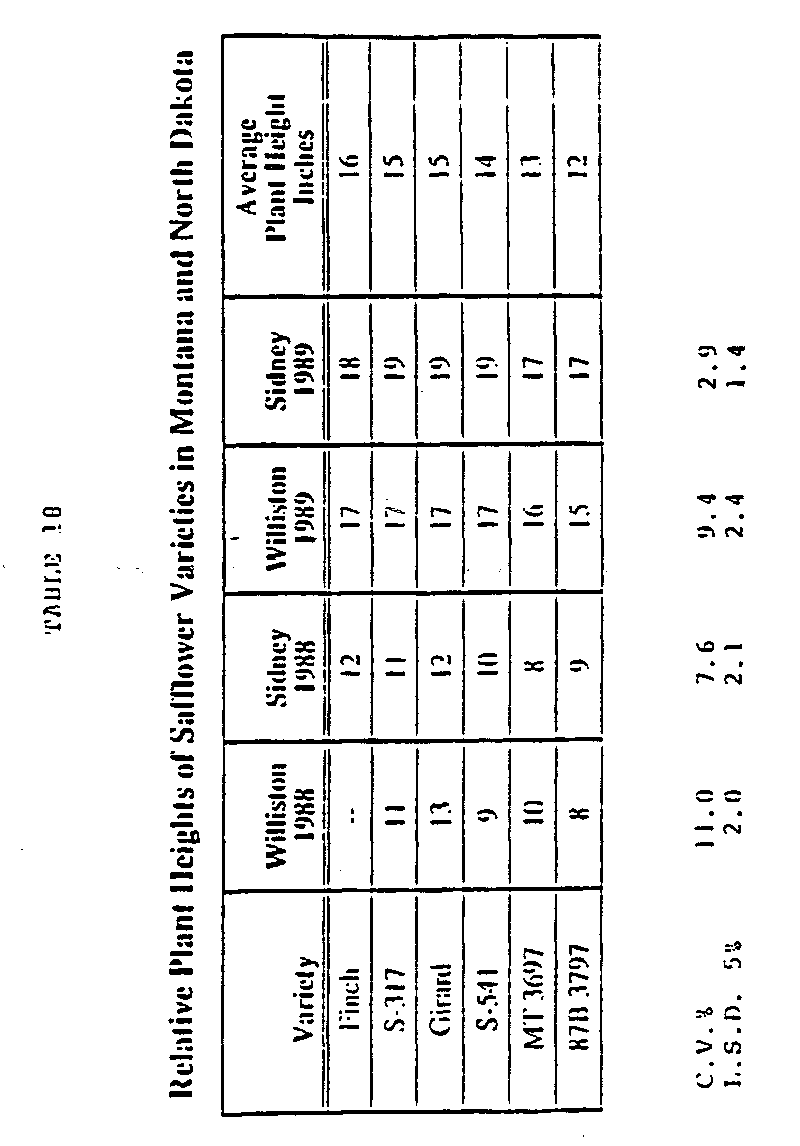

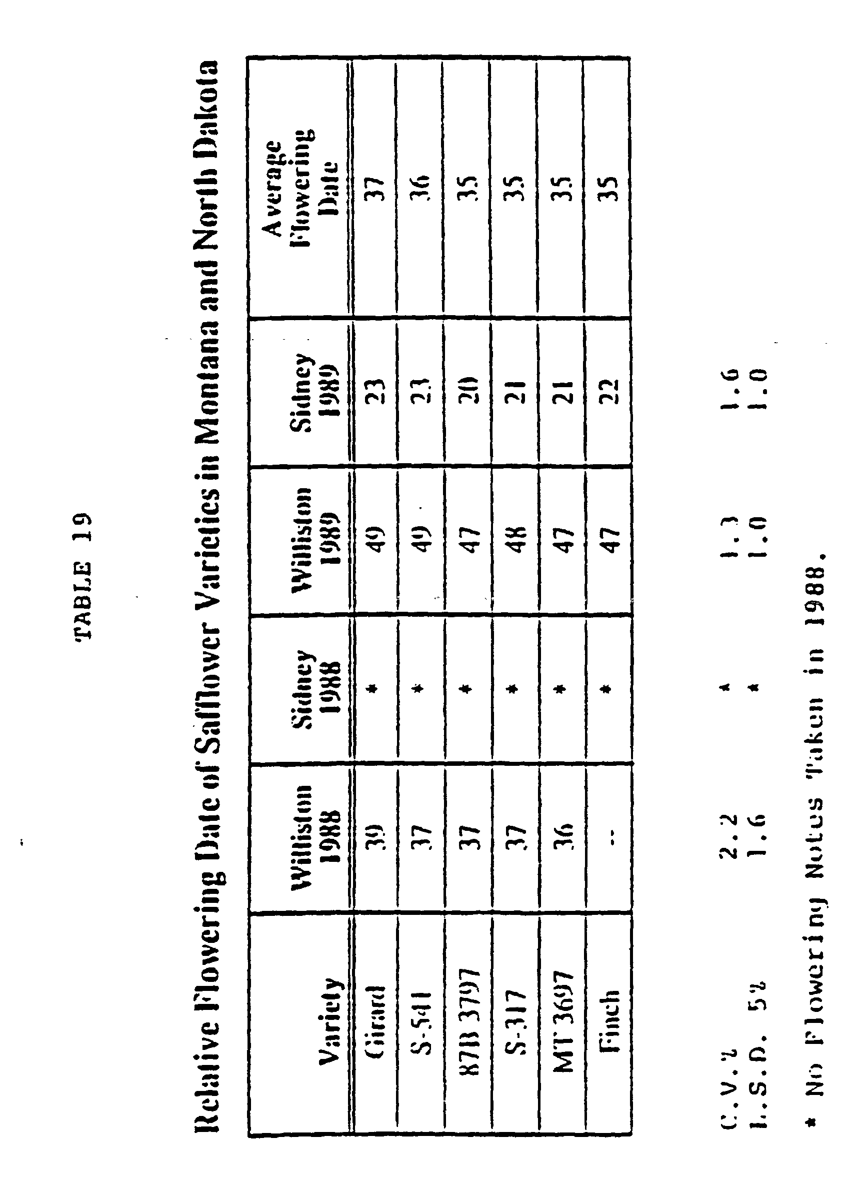

A total of 2500 seeds of variety Montola-2000 high oleic are concurrently being deposited with the American Type Culture Collection, Rockville, Maryland. The deposit is assigned number 40751. A plant variety protection application is also being concurrently filed.

A total of 2500 seeds of a high linoleic acid oil content are deposited with the American Type Culture Collection and designated as Morlin.

### Example 9

### Breeding Pedigrees of Representative Safflower Varieties

The following high oleic acid safflower varieties were obtained by the described breeding pedigree.

### (1) 88B5293-1

female parent: [(Biggs/PII95895//C.L.1653/87-42-3)//AC-1]/79AZ2603 crossed with
male parent: [(Frio/87-42-3//87-42-3/AC-1)/79AZ29379-7]

### (2) 88B5360-1

female parent: (AZ9322//87-14-6/S-208)//AZ2698/012-251-3-6 crossed with
male parent: (87-42-3/AC-1//AZ2698/012-251-3-6)///[(87-14-B/N-10//87-42-3/AC-1)//(87-42-3/AC-1//AZ2698/012-251-3-6)]//white 80 nurs. pigmentless

### (3) 89B6051-1

female parent: [(AZ2698/012-251-3-6//87-42-3/AC-1)// white 80 nurs. pigmentless]//UCD1982-620/Hartman///[(87-42-3/AC-1//AZ749-29)//79AZ2603] crossed with
male parent: S-317

### (4) 89B6594-1

female parent: 87-42-3/AC-1//[(87-14-B//87-42-3/AC-1)//OL35-2/PCM-1] crossed with
male parent: 79AZ9386-1

### (5)

female parent: [87-42-3/AC-1//[(87-42-3/AC-1//87-42-3/AC-1)//OL35-2/PCM-1]]///[(87-42-3/AC-1//88/74-2/N-10)//Mex.Dwarf-2//OC35-2/PCM-1)]//white 80 nurs. pigmentless crossed with
male parent: (87-42-3/AC-1//AZ746-29)//79AZ2603

Examples 10-13 are directed to the development of high linoleic acid varieties.

### Example 10

### 1985 Breeding Program and Results

The top 316 lines for linoleic acid production were selected from a group of 6000 crosses which were made in 1985. The 1985 lines produced from 83.2% to 89.2% linoleic acid in the seed. The oleic acid content ranged from 3.9% to 8.8% and the saturated fatty acid content ranged from 487% to 8.0%. The ratio of saturated fatty acids to linoleic acid ranged from 0.063 to 0.096. The mean temperatures for July and August were 71.4°F and 66.3°F, respectively. The results of 192 representative lines are shown in Table 20.

**TABLE 20**

| SAMPLE # | % PALMITIC | % STEARIC | % OLEIC | % LINOLEIC |
|---|---|---|---|---|
| 3595 | 3.4 | 1.4 | 3.9 | 89.2 |
| 3576 | 3.8 | 1.7 | 5.3 | 87.2 |
| 3605 | 4.3 | 1.3 | 5.4 | 86.8 |
| 885 | 3.8 | 1.6 | 6.5 | 86.1 |
| 3598 | 4.8 | 1.3 | 6.1 | 85.7 |
| 718 | 4.0 | 1.7 | 7.2 | 85.6 |
| 886 | 4.2 | 1.7 | 6.9 | 85.5 |
| 565 | 4.6 | 1.5 | 6.5 | 85.5 |
| 882 | 4.1 | 1.8 | 7.3 | 85.5 |
| 2537 | 4.8 | 1.5 | 6.0 | 85.3 |
| 2723 | 5.0 | 1.7 | 6.3 | 85.2 |
| 2536 | 4.8 | 1.5 | 6.0 | 85.2 |
| 3449 | 5.2 | 1.8 | 5.9 | 85.1 |
| 2538 | 4.9 | 1.6 | 6.2 | 85.1 |
| 3450 | 5.2 | 1.8 | 6.0 | 85.0 |
| 2812 | 5.4 | 1.6 | 5.8 | 85.0 |
| 2656 | 5.4 | 1.6 | 6.0 | 85.0 |
| 2811 | 5.5 | 1.7 | 5.8 | 84.9 |
| 3447 | 5.2 | 1.8 | 6.1 | 84.9 |
| 2657 | 5.6 | 1.5 | 6.2 | 84.9 |
| 2655 | 5.5 | 1.6 | 6.2 | 84.9 |
| 2783 | 5.1 | 1.6 | 6.5 | 84.9 |
| 2541 | 5.0 | 1.5 | 6.1 | 84.9 |
| 3132 | 5.4 | 1.6 | 6.1 | 84.8 |
| 3590 | 4.7 | 1.8 | 6.9 | 84.8 |
| 1246 | 4.8 | 1.8 | 7.1 | 84.8 |
| 3956 | 5.4 | 1.8 | 6.0 | 84.8 |
| 3596 | 4.9 | 1.7 | 6.7 | 84.8 |
| 3448 | 5.2 | 1.8 | 6.4 | 84.7 |
| 3446 | 5.2 | 1.8 | 6.3 | 84.7 |
| 3606 | 4.1 | 1.5 | 7.8 | 84.7 |
| 2784 | 5.3 | 1.6 | 6.5 | 84.7 |
| 2810 | 5.2 | 1.6 | 6.4 | 84.7 |
| 2782 | 5.1 | 1.6 | 6.8 | 84.7 |
| 3452 | 5.3 | 1.9 | 6.3 | 84.6 |
| 1254 | 4.7 | 1.7 | 7.3 | 84.6 |
| 2833 | 4.7 | 1.6 | 7.2 | 84.6 |
| 458 | 3.9 | 1.9 | 8.2 | 84.6 |
| 3451 | 5.2 | 1.9 | 6.4 | 84.6 |
| 3905 | 5.0 | 1.9 | 6.6 | 84.6 |
| 3131 | 5.1 | 1.7 | 6.8 | 84.6 |
| 1245 | 4.9 | 1.9 | 7.1 | 84.5 |
| 1252 | 4.7 | 1.6 | 7.6 | 84.5 |
| 3453 | 5.4 | 1.8 | 6.2 | 84.5 |
| 2316 | 4.9 | 1.5 | 7.7 | 84.5 |
| 2688 | 5.1 | 1.7 | 7.0 | 84.5 |
| 2625 | 4.6 | 2.2 | 7.0 | 84.4 |
| 1256 | 4.7 | 2.1 | 7.3 | 84.4 |
| 3368 | 5.4 | 2.1 | 7.2 | 83.4 |
| 2017 | 5.5 | 2.1 | 7.5 | 83.4 |
| 3517 | 5.6 | 1.6 | 7.5 | 83.4 |
| 2153 | 5.1 | 1.5 | 8.8 | 83.4 |
| 62 | 5.1 | 1.9 | 8.0 | 83.4 |
| 2512 | 5.1 | 1.6 | 8.0 | 83.4 |
| 2218 | 5.1 | 1.9 | 8.1 | 83.4 |
| 2866 | 5.4 | 1.8 | 7.7 | 83.4 |
| 3511 | 5.5 | 1.7 | 7.6 | 83.4 |
| 2015 | 5.5 | 2.2 | 7.6 | 83.4 |
| 2105 | 5.2 | 1.8 | 8.2 | 83.4 |
| 1853 | 6.0 | 1.8 | 7.4 | 83.4 |
| 2162 | 5.4 | 1.8 | 8.1 | 83.4 |
| 2382 | 5.5 | 1.8 | 7.9 | 83.4 |
| 3256 | 5.1 | 1.5 | 8.1 | 83.4 |
| 3383 | 5.4 | 1.7 | 7.6 | 83.4 |
| 3875 | 5.3 | 1.8 | 7.6 | 83.4 |
| 1849 | 6.1 | 1.7 | 7.4 | 83.4 |
| 1847 | 6.0 | 1.7 | 7.5 | 83.4 |
| 4353 | 5.0 | 1.7 | 8.3 | 83.4 |
| 921 | 4.7 | 1.6 | 8.6 | 83.4 |
| 2677 | 5.0 | 1.8 | 7.7 | 83.4 |
| 714 | 5.2 | 1.7 | 8.0 | 83.4 |
| 1674 | 5.1 | 1.9 | 8.0 | 83.4 |
| 2513 | 5.1 | 1.6 | 8.2 | 83.4 |
| 3496 | 5.3 | 2.1 | 7.3 | 83.4 |
| 3654 | 5.5 | 1.5 | 7.7 | 83.4 |
| 1019 | 5.0 | 2.0 | 7.7 | 83.4 |
| 4682 | 5.1 | 1.6 | 8.2 | 83.4 |
| 3508 | 5.3 | 2.0 | 7.5 | 83.3 |
| 3515 | 5.4 | 1.6 | 7.7 | 83.3 |
| 111 | 6.1 | 1.6 | 6.8 | 83.3 |
| 1525 | 5.3 | 1.6 | 8.5 | 83.3 |
| 2497 | 5.0 | 1.7 | 8.2 | 83.3 |
| 2771 | 5.3 | 2.2 | 7.2 | 83.3 |
| OKER | 5.1 | 1.8 | 7.6 | 83.3 |
| 2776 | 5.3 | 2.1 | 7.3 | 83.3 |
| 3538 | 5.2 | 1.9 | 7.6 | 83.3 |
| 4130 | 4.8 | 2.1 | 8.0 | 83.3 |
| 53 | 5.6 | 1.5 | 7.5 | 83.3 |
| 650 | 5.1 | 2.2 | 8.0 | 83.3 |
| 2163 | 5.2 | 1.8 | 8.4 | 83.3 |
| 1854 | 6.2 | 1.8 | 7.3 | 83.3 |
| 2384 | 5.5 | 1.7 | 8.0 | 83.3 |
| 2166 | 5.2 | 1.8 | 8.3 | 83.3 |
| 2498 | 5.0 | 1.8 | 8.1 | 83.3 |
| 3002 | 5.5 | 1.9 | 7.3 | 83.3 |
| 2778 | 5.2 | 2.0 | 7.5 | 83.3 |
| 2190 | 5.1 | 1.7 | 8.5 | 83.3 |
| 1779 | 5.1 | 1.9 | 8.0 | 83.3 |
| 2643 | 5.1 | 1.7 | 7.9 | 83.3 |
| 537 | 4.9 | 1.6 | 8.6 | 83.3 |
| 583 | 5.0 | 1.8 | 8.6 | 83.3 |
| 2870 | 5.4 | 1.7 | 7.5 | 83.3 |
| 3650 | 5.2 | 1.8 | 7.7 | 83.3 |
| 2074 | 5.1 | 1.8 | 8.5 | 83.3 |
| 2125 | 5.4 | 1.9 | 7.9 | 83.3 |
| 129 | 6.2 | 1.7 | 7.5 | 83.3 |
| 2213 | 5.2 | 2.1 | 8.0 | 83.3 |
| 3512 | 5.5 | 1.8 | 7.5 | 83.3 |
| 3498 | 5.3 | 2.0 | 7.4 | 83.3 |
| 2711 | 5.6 | 1.4 | 7.6 | 83.3 |
| 2225 | 5.0 | 1.8 | 8.4 | 83.3 |
| 695 | 5.6 | 1.7 | 8.1 | 83.3 |
| 3445 | 5.2 | 1.5 | 8.4 | 83.3 |
| 113 | 5.8 | 1.7 | 7.0 | 83.3 |
| 908 | 5.2 | 1.5 | 8.8 | 83.3 |
| 693 | 5.2 | 1.7 | 8.5 | 83.3 |
| 654 | 5.2 | 1.7 | 7.7 | 83.3 |
| 104 | 6.1 | 1.7 | 7.0 | 83.3 |
| 3426 | 5.2 | 1.7 | 7.9 | 83.2 |
| 1618 | 5.2 | 1.9 | 8.0 | 83.2 |
| 3643 | 5.2 | 2.5 | 7.3 | 83.2 |
| 124 | 5.7 | 1.8 | 7.8 | 83.2 |
| 10 | 4.3 | 2.1 | 7.8 | 83.2 |
| 3364 | 5.2 | 2.3 | 7.4 | 83.2 |
| 1863 | 6.1 | 1.8 | 7.5 | 83.2 |
| 535 | 4.7 | 1.7 | 9.0 | 83.2 |
| 443 | 5.1 | 2.3 | 7.7 | 83.2 |
| 1523 | 5.6 | 1.9 | 7.9 | 83.2 |
| 1872 | 6.1 | 1.7 | 7.6 | 83.2 |
| 3543 | 5.1 | 1.8 | 7.9 | 83.2 |
| 2409 | 5.0 | 1.8 | 8.5 | 83.2 |
| 2600 | 5.0 | 1.9 | 8.0 | 83.2 |
| 2155 | 5.0 | 1.7 | 8.7 | 83.2 |
| 2193 | 4.9 | 1.5 | 8.8 | 83.2 |
| 3029 | 4.7 | 1.4 | 8.8 | 83.2 |
| 4804 | 5.4 | 1.5 | 8.2 | 83.2 |
| 2673 | 5.0 | 1.7 | 8.2 | 83.2 |
| 3572 | 5.3 | 1.7 | 8.0 | 83.2 |
| 436 | 5.1 | 2.3 | 7.9 | 83.2 |
| 3858 | 5.0 | 2.0 | 7.9 | 83.2 |
| 3586 | 4.9 | 1.8 | 8.2 | 83.2 |
| 3894 | 5.0 | 1.8 | 8.2 | 83.2 |
| 123 | 5.6 | 1.8 | 7.9 | 83.2 |
| 3214 | 4.7 | 2.4 | 8.0 | 83.2 |
| 3454 | 5.2 | 1.9 | 6.6 | 84.4 |
| 3828 | 5.0 | 1.8 | 7.0 | 84.3 |
| 3573 | 5.1 | 1.5 | 7.2 | 84.3 |
| 2830 | 4.6 | 1.6 | 7.5 | 84.3 |
| 2829 | 4.7 | 1.7 | 7.3 | 84.3 |
| 1579 | 5.2 | 2.0 | 7.3 | 84.3 |
| 2626 | 4.6 | 2.2 | 7.1 | 84.3 |
| 2391 | 4.4 | 1.5 | 8.3 | 84.3 |
| 4472 | 5.3 | 1.7 | 6.9 | 84.3 |
| 3592 | 5.0 | 1.6 | 7.4 | 84.2 |
| 3098 | 5.3 | 1.9 | 6.5 | 84.2 |
| 3601 | 4.7 | 1.7 | 7.6 | 84.2 |
| 1251 | 5.0 | 1.7 | 7.5 | 84.2 |
| 2687 | 5.0 | 1.7 | 7.1 | 84.2 |
| 1859 | 5.1 | 2.1 | 7.4 | 84.2 |
| 2809 | 5.3 | 1.8 | 6.8 | 84.2 |
| 2147 | 5.0 | 1.7 | 7.7 | 84.2 |
| 2775 | 5.3 | 1.9 | 6.7 | 84.2 |
| 1 | 4.2 | 2.3 | 7.0 | 84.1 |
| 522 | 4.6 | 1.9 | 7.9 | 84.1 |
| 2510 | 5.1 | 1.7 | 7.1 | 84.1 |
| 3417 | 5.3 | 1.6 | 7.2 | 84.1 |
| 2831 | 4.8 | 1.8 | 7.5 | 84.1 |
| 3130 | 5.3 | 1.9 | 6.8 | 84.1 |
| 3869 | 5.2 | 1.8 | 7.2 | 84.1 |
| 4212 | 5.3 | 2.1 | 6.6 | 84.1 |
| 2871 | 5.3 | 1.6 | 7.2 | 84.1 |
| 521 | 5.1 | 1.8 | 7.7 | 84.1 |
| 1247 | 4.9 | 1.9 | 7.5 | 84.0 |
| 2151 | 4.9 | 1.8 | 8.0 | 84.0 |
| 3455 | 5.4 | 1.9 | 6.7 | 84.0 |
| 1253 | 4.8 | 2.0 | 7.6 | 84.0 |
| 484 | 4.5 | 1.5 | 8.3 | 84.0 |
| 3906 | 5.3 | 1.6 | 7.2 | 84.0 |
| 883 | 4.5 | 2.1 | 8.1 | 84.0 |
| 2317 | 5.0 | 1.6 | 8.1 | 84.0 |
| 3502 | 5.1 | 2.0 | 7.1 | 84.0 |
| 247 | 5.0 | 1.2 | 7.6 | 84.0 |
| 4765 | 5.8 | 1.9 | 6.6 | 84.0 |
| 3649 | 5.1 | 1.7 | 7.4 | 84.0 |
| 2707 | 5.0 | 1.5 | 7.6 | 84.0 |
| 4599 | 5.1 | 1.9 | 7.2 | 83.9 |
| 3102 | 5.7 | 1.8 | 6.4 | 83.9 |
| 3101 | 5.4 | 2.1 | 6.4 | 83.9 |
| 1249 | 5.0 | 2.1 | 7.4 | 83.9 |
| 3506 | 5.1 | 1.7 | 7.5 | 83.9 |
| 534 | 5.0 | 1.5 | 8.4 | 83.9 |
| 2148 | 5.0 | 1.8 | 7.9 | 83.9 |

### Example 11

### 1987 Breeding Program and Results

The previously obtained lines were selfed in 1987 and the seed analyzed for linoleic acid production. A total of 294 lines were selected after the 1987 season which produced seeds having a linoleic acid content greater than 80%. The range in linoleic acid content of these lines was 86.0% to 89.1%. The oleic acid content ranged from 3.9% to 8.0% and the saturated fatty acid content ranged from 4.7% to 7.2%, with one line at 7.6%.
The ratio of saturated fatty acids to linoleic acid ranged from 0.054 to 0.084. The mean temperatures for July and August were 71.3°F and 66.0°F, respectively. The results are shown in Table 21.

**TABLE 21**

| FIELD ENTRY | % PALMITIC | % STEARIC | % OLEIC | % LINOLEIC |
|---|---|---|---|---|
| # | 16:0 | 18:0 | 18:1 | 18:2 |
| 7b4229-5 | 5.2 | 1.7 | 5.6 | 87.4 |
| 7b4239-3 | 5.8 | 1.4 | 5.6 | 87.3 |
| 7b4226-1 | 4.8 | 1.5 | 5.5 | 87.3 |
| 7b4239-4 | 4.4 | 1.8 | 5.3 | 87.3 |
| 7b3694-2 | 3.4 | 1.5 | 6.5 | 87.3 |
| 7b50-2 | 4.4 | 1.5 | 6.8 | 87.3 |
| 7b4223-1 | 4.8 | 1.5 | 5.1 | 87.3 |
| 7b4239-5 | 4.5 | 1.4 | 5.9 | 87.2 |
| 7b4238-1 | 5.2 | 1.6 | 6.1 | 87.2 |
| 7b4349-1 | 4.3 | 1.3 | 6.1 | 87.2 |
| 7b4233-4 | 4.4 | 1.6 | 5.5 | 87.2 |
| 7b1175-2 | 3.7 | 2.0 | 5.3 | 87.2 |
| 7b4330-5 | 5.1 | 1.5 | 5.9 | 87.2 |
| 7b3695-1 | 4.3 | 1.8 | 4.9 | 87.2 |
| 7b4233-4 | 4.7 | 1.6 | 5.4 | 87.2 |
| 7b4133-5 | 4.6 | 1.8 | 6.5 | 87.1 |
| 7b4231-2 | 5.0 | 1.8 | 6.1 | 87.1 |
| 7b4226-2 | 5.2 | 1.3 | 5.8 | 87.1 |
| 7b1274-1 | 5.0 | 1.7 | 5.2 | 87.1 |
| 7b3648-2 | 4.7 | 1.4 | 4.9 | 87.1 |
| 7b4328-2 | 4.5 | 1.5 | 5.7 | 87.1 |
| 7b4108-2 | 3.7 | 1.5 | 6.8 | 87.1 |
| 7b4239-1 | 5.1 | 1.3 | 5.7 | 87.0 |
| 7b3666-5 | 4.3 | 1.4 | 7.0 | 87.0 |
| 7b3643a-4 | 5.0 | 2.0 | 6.0 | 87.0 |
| 7b4108-2 | 4.5 | 1.4 | 7.2 | 87.0 |
| 7b4239-4 | 4.9 | 1.4 | 5.4 | 87.0 |
| 7b4236-3 | 5.3 | 1.5 | 5.4 | 87.0 |
| 7b4224-1 | 5.2 | 1.5 | 6.4 | 86.9 |
| 7b4108-2 | 4.1 | 1.5 | 7.0 | 86.5 |
| 7b4236-3 | 5.6 | 1.3 | 5.7 | 86.5 |
| 7b4231-3 | 4.8 | 1.8 | 6.0 | 86.5 |
| 7b1274-3 | 5.0 | 1.8 | 5.6 | 86.5 |
| 7b4108-2 | 4.6 | 1.6 | 7.3 | 86.5 |
| 7b3684-1 | 4.5 | 1.8 | 6.0 | 86.5 |
| 7b4225-4 | 5.0 | 1.6 | 6.1 | 86.5 |
| 7b3684-1 | 4.3 | 1.7 | 5.9 | 86.5 |
| 7b4238-1 | 5.0 | 1.8 | 6.7 | 86.5 |
| 7b4249-4 | 5.0 | 1.7 | 5.9 | 86.5 |
| 7b4226-1 | 4.9 | 1.6 | 5.9 | 86.5 |
| 7b1274-1 | 5.3 | 1.6 | 5.7 | 86.5 |
| 7b4349-2 | 4.3 | 1.8 | 6.1 | 86.5 |
| 7b4236-2 | 5.2 | 1.5 | 5.7 | 86.5 |
| 7b2145-2 | 4.8 | 1.6 | 5.4 | 86.5 |
| 7b4237-2 | 4.9 | 1.5 | 5.7 | 86.5 |
| 7b2145-1 | 5.4 | 1.4 | 5.2 | 86.5 |
| 7b4359-2 | 5.1 | 1.8 | 6.3 | 86.5 |
| 7b1207-1 | 3.9 | 1.6 | 6.6 | 86.4 |
| 7b4108-2 | 4.3 | 1.5 | 6.8 | 86.4 |
| 7b4299-1 | 4.8 | 1.3 | 7.3 | 86.4 |
| 7b4349-1 | 4.5 | 1.6 | 5.5 | 86.4 |
| 7b4239-4 | 4.9 | 1.4 | 6.0 | 86.4 |
| 7b4359-5 | 4.8 | 1.7 | 6.1 | 86.4 |
| 7b4236-2 | 5.4 | 1.7 | 6.5 | 86.4 |
| 7b1274-5 | 5.1 | 2.0 | 5.7 | 86.4 |
| 7b4132-5 | 4.5 | 1.5 | 6.7 | 86.4 |
| 7b3668-2 | 4.8 | 1.6 | 5.5 | 86.4 |
| 7b4238-1 | 5.2 | 1.7 | 5.8 | 86.4 |
| 7b3648-2 | 5.0 | 2.0 | 6.0 | 86.0 |
| 7b2115-1 | 4.2 | 1.6 | 6.6 | 86.0 |
| 7b4228-4 | 5.1 | 1.9 | 6.0 | 86.0 |
| 7b3658-1 | 5.0 | 2.0 | 7.0 | 86.0 |
| 7b4229-5 | 4.6 | 1.6 | 6.4 | 86.0 |
| 7b3665-5 | 5.0 | 2.0 | 7.0 | 86.0 |
| 7b4236-3 | 5.6 | 1.4 | 7.1 | 86.0 |
| 7b3667-1 | 4.4 | 2.0 | 7.0 | 86.0 |
| 7b4359-4 | 5.2 | 1.5 | 6.1 | 86.0 |
| 7b4238-5 | 4.9 | 1.7 | 6.4 | 86.0 |
| 7b4349-4 | 4.3 | 1.8 | 6.3 | 86.0 |
| 7b1274-1 | 5.1 | 1.8 | 6.2 | 86.0 |
| 7b4330-1 | 5.2 | 1.5 | 6.7 | 86.0 |
| 7b4168-5 | 4.6 | 1.4 | 7.1 | 86.0 |
| 7b4307-2 | 4.8 | 1.7 | 7.1 | 86.0 |
| 7b4189-1 | 4.6 | 1.6 | 6.8 | 86.0 |
| 7b4244-2 | 4.7 | 1.6 | 6.5 | 86.0 |
| 7b4224-2 | 5.6 | 1.1 | 6.4 | 86.0 |
| 7b3626-5 | 3.1 | 1.6 | 8.0 | 86.0 |
| 7b2171-1 | 4.6 | 1.4 | 6.4 | 86.0 |
| 7b3666-4 | 4.4 | 2.0 | 7.0 | 86.0 |
| 7b3631-4 | 5.2 | 2.0 | 5.2 | 86.0 |
| 7b3685-4 | 4.5 | 1.6 | 6.6 | 86.0 |
| 7b1272-4 | 5.1 | 1.8 | 4.7 | 86.0 |
| 7b3635-1 | 5.0 | 2.0 | 6.3 | 86.0 |
| 7b4168-5 | 4.6 | 1.7 | 6.6 | 86.0 |
| 7b3644-2 | 5.1 | 2.0 | 6.0 | 86.0 |
| 7b4224-4 | 4.8 | 1.7 | 6.7 | 86.0 |
| 7b3666-3 | 5.0 | 1.4 | 7.0 | 86.0 |

### Example 12

### 1988 Breeding Program and Results

These 294 lines were selfed in 1988 and the seed analyzed for linoleic acid production. A total of 300 lines were selected after the 1988 season which produced seeds having a linoleic acid content greater than 80%. The range in linoleic acid content of these lines was 83.0% to 90.4%. The oleic acid content ranged from 3.3% to 12.4% and the saturated fatty acid content ranged from 5.1% to 8.3%. The ratio of saturated fatty acids to oleic acid ranged from 0.056 to 0.099. The mean temperatures for July and August were 73.7°F and about 70.6°F, respectively. The results are shown in Table 22.

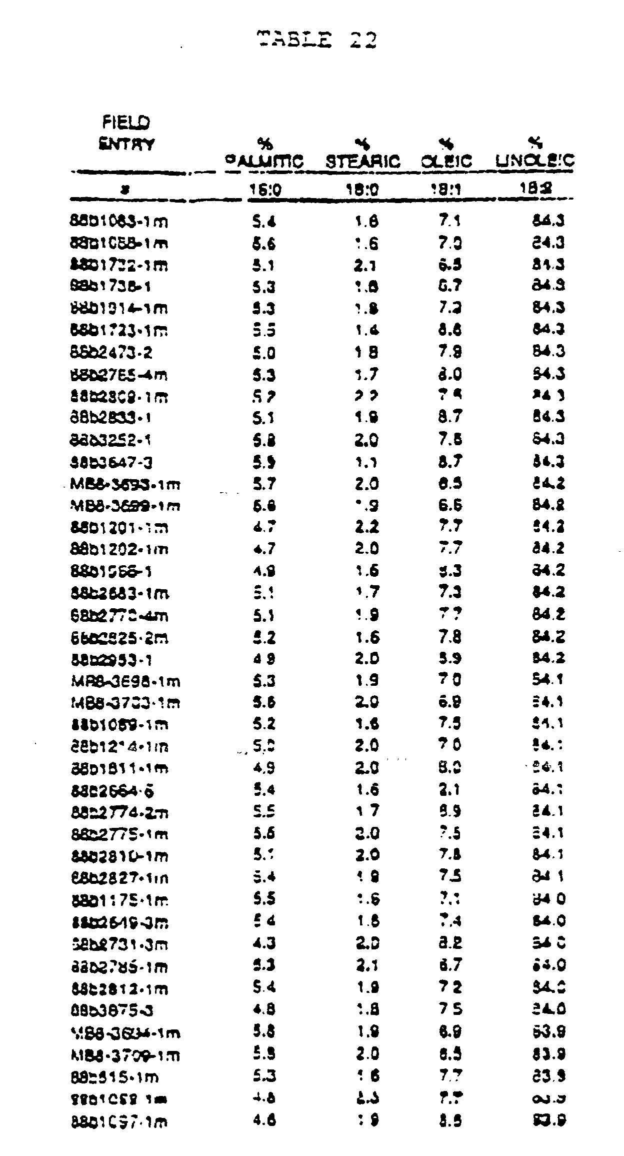

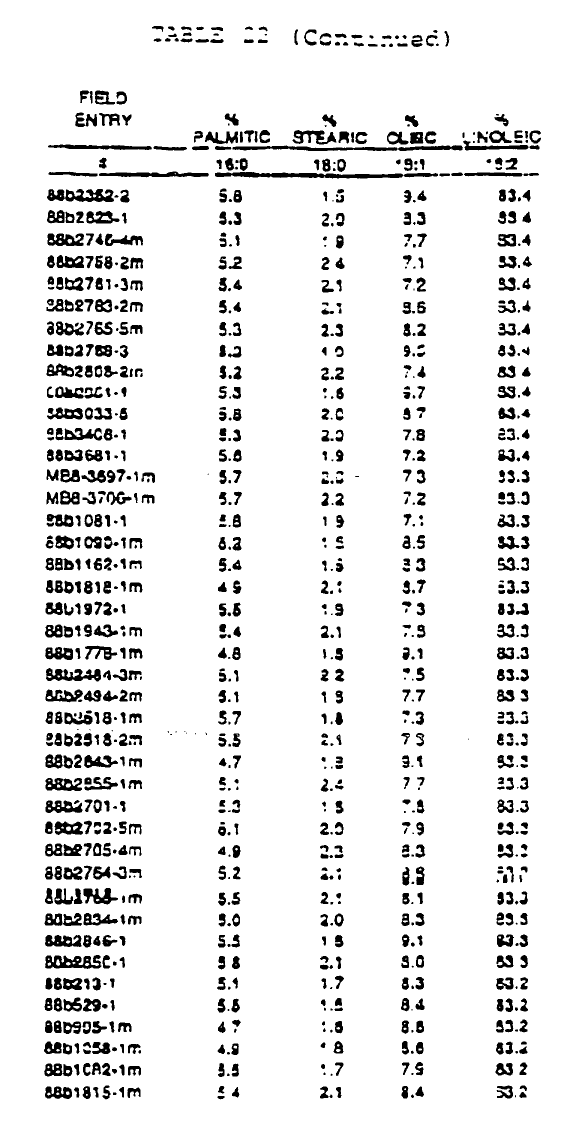

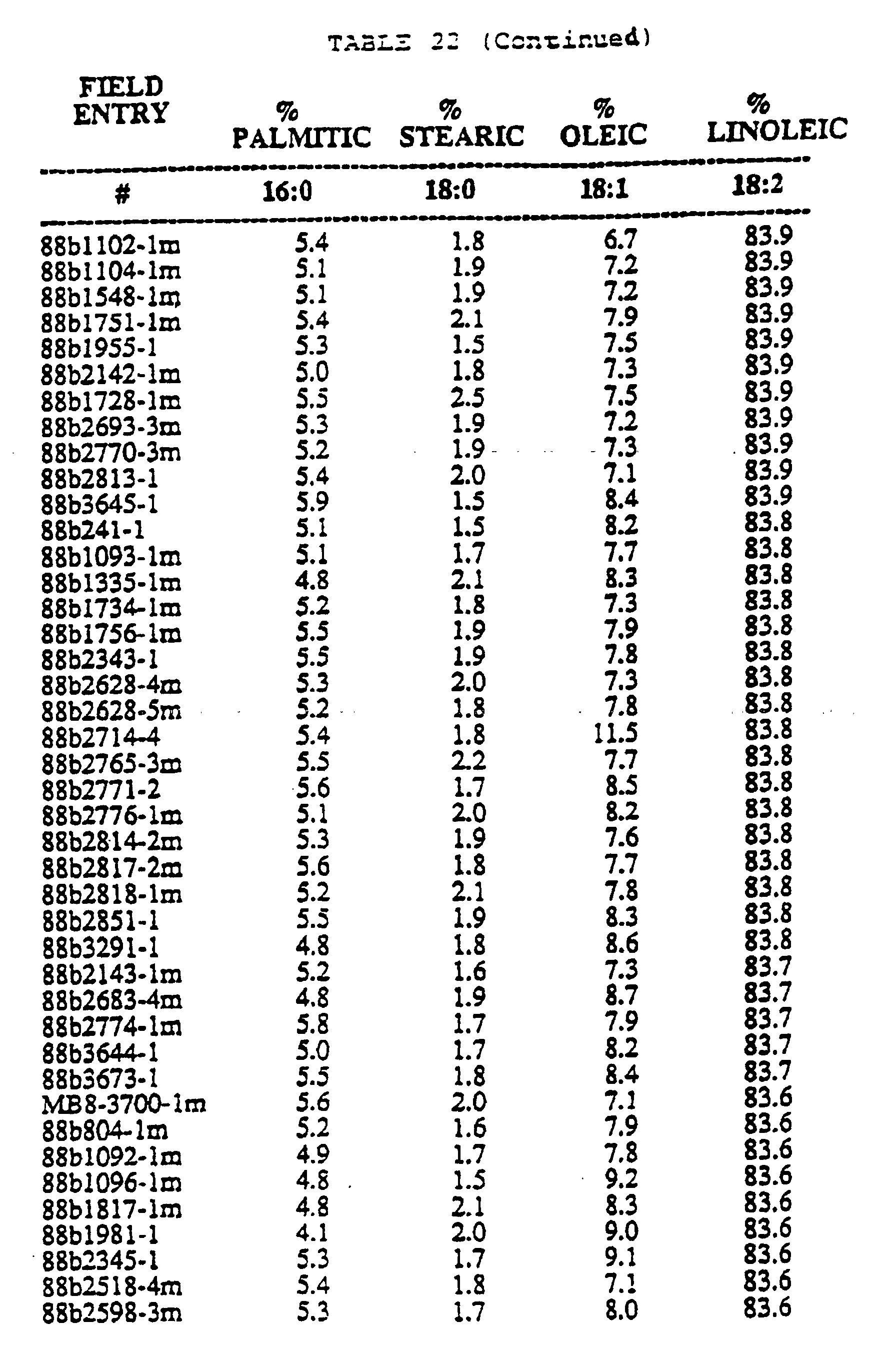

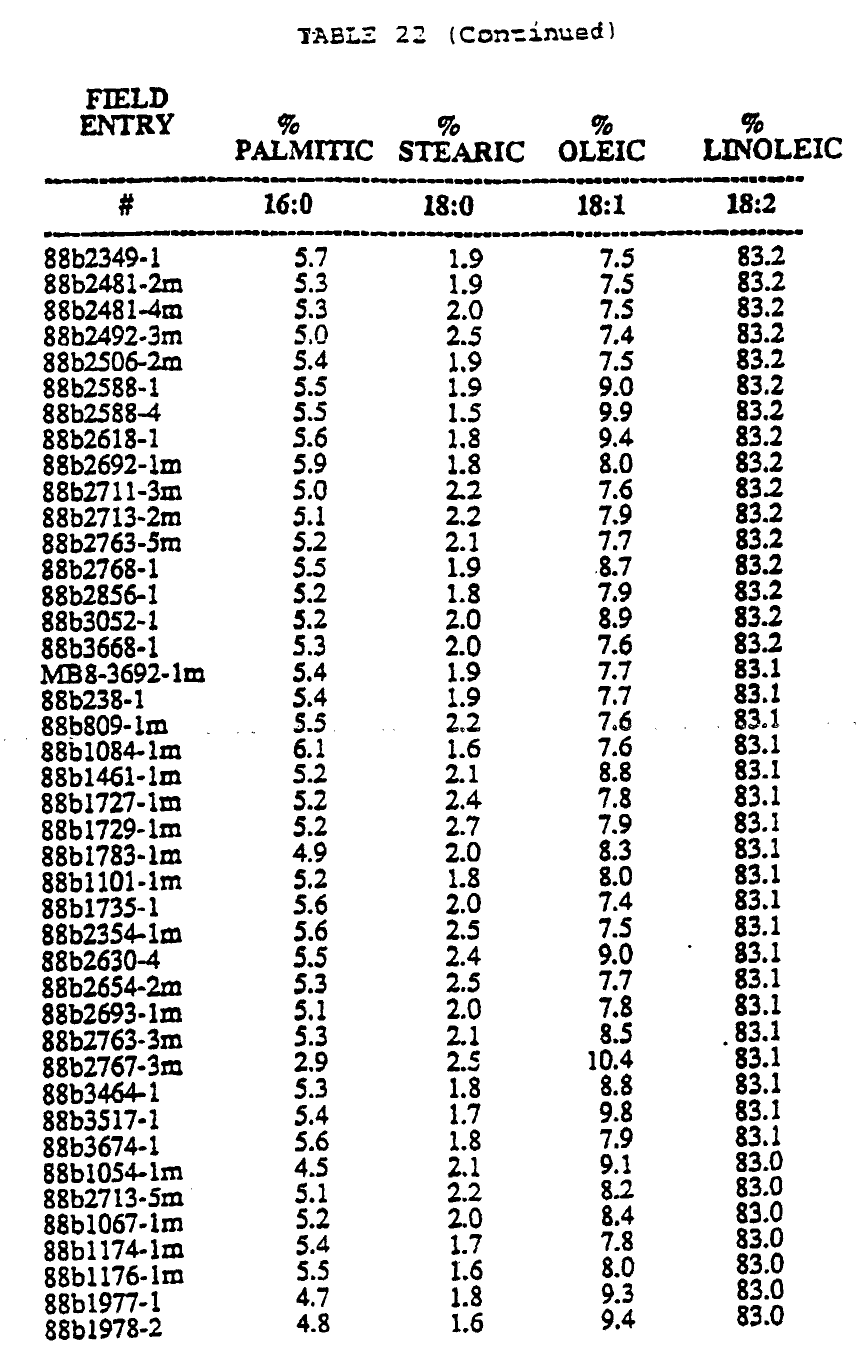

### Example 13

### Breeding Pedigrees of Representative Safflower Varieties

The following high linoleic acid safflower varieties were obtained by the described breeding pedigree.

### (1) 89B7503-5

female parent: Roundup Tolerant Selection 1961 world bulk composite crossed with
male parent: [87-42-3/AC-1//(87-42-3/AC-1//AZ2698/012-251-3-6]///(87-42-3/AC-1//88-74-2/N-10)//(Mex.dwarf-2//OL35-2/PCM-1)//white 80 nurs. pigmentless

### (2) 89B7613

female parent: 87-42-3/AC-1//Cargill dwarf crossed with
male parent: 87-42-3/AC-1//(87-42-3/AC-1//AZ2698/012-251-3-6)

### (3) 89B7311-1

female parent: 87-42-3/AC-1///[[(87-42-3/AC-1//AZ2698/012-251-3-6)]//87-42-3/AC-1] crossed with
male parent: [87-42-3/AC-1//(87-42-3/AC-1//AZ2698/012-251-3-6)]///[(87-42-3/AC-1//88-74-2/N-10)//(Mex.dwarf-2//OL35-2/PCM-1)//white 80 nurs. pigmentless

### (4) 89B7557-3

female parent: [(Biggs/PI195895//C.L.1653/87-42-3)//UCD1982-620/Oker] crossed with
male parent: [87-42-3/AC-1//(87-42-3/AC-1//AZ2698/012-251-3-6)]///[(87-42-3/AC-1//88-74-2/N-10)//(Mex.dwarf-2//OL35-2/PCM-1)//white 80 nurs. pigmentless

### (5) 89B7475

female parent: 87-42-3/AC-1//Cargill dwarf crossed with
male parent: [87-42-3/AC-1//(87-42-3/AC-1//AZ2698/012-251-3-6)]///[(87-42-3/AC-1//88-74-2/N-10)//(Mex.dwarf-2//OL35-2/PCM-1)//white 80 nurs. pigmentless

### (6) 89B934-3

female parent: 88-45-4/S-208//N-4051/Gila crossed with
male parent: 87-42-3/AC-1

### (7) 89B7448-1

female parent: (87-42-3/AC-1///[[(87-42-3/AC-1//AZ2698/012-251-3-6)]//[(87-14-B/N-10)//87-42-3/AC-1)//(87-42-3/AC-1)//AZ2698/012-251-3-6]]//white 80 nurs. pigmentless crossed with
male parent: 87-42-3/AC-1//[(87-42-3?AC-1)//AZ2698/012-251-3-6)]

The oil content of 89B7448-1 is 4.4% palmitic acid, 2.0% stearic acid, 5.9% oleic acid and 86.0% linoleic acid. The characteristics of this line are similar to other commercial high linoleic acid lines.

Additional test results obtained on the subject matter of the invention.

| OLEIC 1990 INCREASE OIL QUALITY ANALYSES | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 90-OL-8111 | 1142 | | | 3.3 | 1.3 | 83.7 | 10.0 |
| 90-OL-8091 | 1140 | | | 3.4 | 1.1 | 83.6 | 10.7 |
| 90-OL-8041 | 1135 | | | 3.4 | 1.2 | 83.2 | 10.5 |
| | | | | | AVG | 83.5 | |

| 1989 89b- Oil Quality Analyses TOP OLEIC LINES | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-6394-1 | 1600 | 51.1 | 106 | 3.3 | 1.3 | 85.5 | 8.4 |
| 89B-6739-1 | 1927 | 36.8 | 19 | 3.4 | 1.6 | 85.5 | 8.0 |
| 89B-6127-1 | 1366 | 43.4 | 76 | 3.6 | 1.5 | 85.4 | 7.9 |
| 89B-6397-1 | 1603 | 50.4 | 99 | 3.4 | 1.2 | 85.4 | 8.6 |
| 89B-6709-1 | 1898 | 49.1 | 42 | 3.2 | 1.1 | 85.4 | 8.8 |
| 89B-6732-1 | 1920 | 40.3 | 35 | 3.6 | 1.4 | 85.4 | 8.2 |
| 89B-6704-1 | 1893 | 51.2 | 37 | 3.3 | 1.3 | 85.3 | 8.4 |
| 89B-6709-4 | 2421 | 49.1 | 42 | 3.1 | 1.4 | 85.2 | 8.6 |
| 89B-6726-5 | 2426 | 45.1 | 95 | 3.6 | 1.1 | 85.0 | 8.8 |
| 89B-6731-1 | 1919 | 51.1 | 62 | 3.6 | 1.1 | 85.0 | 8.6 |
| 89B-6731-5 | 2429 | 51.1 | 62 | 3.4 | 1.1 | 85.0 | 8.9 |
| 89B-6563-1 | 1768 | 51.2 | 290 | 3.3 | 0.9 | 84.9 | 9.3 |
| 89B-6024-1 | 1288 | 53.8 | 181 | 3.3 | 0.9 | 84.8 | 9.4 |
| 89B-6294-1 | 1508 | 49.9 | 75 | 3.6 | 1.1 | 84.8 | 9.0 |
| 89B-6726-1 | 1914 | 45.1 | 95 | 3.7 | 1.2 | 84.8 | 8.7 |
| 89B-6739-3 | 2435 | 36.8 | 19 | 3.4 | 1.7 | 84.8 | 8.4 |
| 89B-6294-5 | 1668 | 49.9 | 75 | 3.5 | 1.2 | 84.7 | 8.9 |
| 89B-6731-3 | 2428 | 51.1 | 62 | 3.6 | 1.2 | 84.7 | 9.0 |
| 89B-6752-4 | 2439 | 51.4 | 37 | 3.6 | 1.4 | 84.6 | 8.7 |
| 89B-6563-5 | 2150 | 51.2 | 290 | 3.6 | 1.4 | 84.5 | 8.3 |
| 89B-6701-1 | 1891 | N/A | 14 | 3.6 | 1.4 | 84.5 | 8.9 |
| 89B-6752-1 | 1939 | 51.4 | 37 | 3.6 | 1.2 | 84.5 | 8.7 |
| 89B-6485-1 | 1693 | 54.0 | 89 | 3.6 | 1.2 | 84.4 | 9.3 |
| 89B-6744-1 | 1930 | 50.6 | 43 | 3.5 | 1.1 | 84.4 | 9.2 |
| 89B-6746-1 | 1933 | 49.0 | 18 | 3.5 | 1.4 | 84.4 | 8.6 |
| 89B-6752-2 | 2437 | 51.4 | 37 | 3.7 | 1.1 | 84.4 | 9.3 |
| 89B-6018-1 | 1273 | 52.2 | 106 | 3.5 | 1.0 | 84.3 | 9.6 |
| 89B-6709-3 | 2420 | 49.1 | 42 | 3.6 | 1.3 | 84.3 | 9.3 |
| 89B-6709-5 | 2422 | 49.1 | 42 | 3.6 | 1.4 | 84.3 | 9.0 |
| 89B-6732-5 | 2433 | 40.3 | 35 | 3.8 | 1.7 | 84.3 | 8.7 |
| 89B-6736-1 | 1924 | N/A | 12 | 3.7 | 1.6 | 84.3 | 8.8 |
| 89B-6765-1 | 1950 | 49.4 | 25 | 3.5 | 1.2 | 84.3 | 9.1 |
| 89B-6159-1 | 1395 | 41.6 | 118 | 3.7 | 2.4 | 84.2 | 8.8 |
| 89B-6483-1 | 1691 | 44.6 | 86 | 3.6 | 1.2 | 84.2 | 9.3 |
| 89B-6704-4 | 2417 | 51.2 | 37 | 3.5 | 1.3 | 84.2 | 9.4 |
| 89B-6732-2 | 2430 | 40.3 | 35 | 3.3 | 1.3 | 84.2 | 9.7 |
| 89B-6022-1 | 1283 | 52.3 | 86 | 3.3 | 1.3 | 84.1 | 10.0 |
| 89B-6706-1 | 1895 | 51.0 | 60 | 3.4 | 1.2 | 84.1 | 9.3 |
| 89B-6726-2 | 2423 | 45.1 | 95 | 3.6 | 1.1 | 84.1 | 9.6 |
| 89B-6766-1 | 1951 | 50.6 | 26 | 3.7 | 1.3 | 84.1 | 9.0 |
| 89B-6145-1 | 1382 | 50.2 | 367 | 3.8 | 1.5 | 84.0 | 9.3 |
| 89B-6594-1 | 1795 | 49.3 | 63 | 3.6 | 1.5 | 84.0 | 9.4 |
| 89B-6669-1 | 1860 | N/A | 17 | 3.5 | 1.4 | 84.0 | 9.7 |
| 89B-6710-1 | 1899 | 47.4 | 42 | 3.6 | 1.5 | 84.0 | 9.3 |
| 89B-6713-1 | 1902 | 49.4 | 30 | 3.4 | 1.2 | 84.0 | 9.6 |
| 89B-6732-3 | 2431 | 40.3 | 35 | 3.6 | 1.7 | 84.0 | 9.2 |
| 89B-6745-1 | 1932 | 51.1 | 49 | 3.6 | 1.2 | 84.0 | 9.4 |

| 1989 89b- Oil Quality Analyses TOP OLEIC LINES | | | | | | | |
|---|---|---|---|---|---|---|---|
| FIELD ENTRY | LAB SAMPLE | OPEN SEED OIL % | TOTAL OPEN SEED | PALMITIC % % | STEARIC | OLEIC % | % LINOLEIC |
| # | # | NMR | grams | 16:0 | 18:0 | 18:1 | 18:2 |
| 89B-6027-1 | 1302 | 55.0 | 79 | 3.4 | 1.0 | 83.9 | 10.2 |
| 89B-6127-5 | 1664 | 43.4 | 76 | 3.9 | 1.2 | 83.9 | 9.2 |
| 89B-6731-2 | 2427 | 51.1 | 62 | 3.7 | 1.3 | 83.9 | 9.4 |
| 89B-6747-1 | 1934 | N/A | 01 | 3.9 | 1.6 | 83.9 | 9.1 |
| 89B-6359-1 | 1572 | 53.3 | 39 | 3.5 | 1.2 | 83.7 | 9.9 |
| 89B-6705-1 | 1894 | 50.9 | 76 | 3.5 | 1.2 | 83.7 | 10.1 |
| 89B-6735-1 | 1923 | N/A | 05 | 3.7 | 1.5 | 83.7 | 9.1 |
| 89B-6478-1 | 1687 | 44.4 | 84 | 3.7 | 1.1 | 83.6 | 10.1 |
| 89B-6711-1 | 1900 | 46.1 | 19 | 3.5 | 1.3 | 83.6 | 10.1 |
| 89B-6728-1 | 1916 | 44.7 | 96 | 3.8 | 1.3 | 83.6 | 9.8 |
| 89B-6762-1 | 1947 | 51.1 | 80 | 3.3 | 1.2 | 83.6 | 9.7 |
| 89B-7429-1 | 2355 | 52.2 | 51 | 3.9 | 1.5 | 83.6 | 9.4 |
| 89B-6146-1 | 1383 | 49.7 | 349 | 3.9 | 1.4 | 83.5 | 10.0 |
| 89B-6294-4 | 1667 | 49.9 | 75 | 3.6 | 1.3 | 83.5 | 9.8 |
| 89B-6393-1 | 1599 | 52.0 | 133 | 3.5 | 1.1 | 83.5 | 10.5 |
| 89B-6616-1 | 1888 | 53.5 | 25 | 3.8 | 1.4 | 83.5 | 9.8 |
| 89B-6701-5 | 2414 | N/A | 14 | 3.5 | 1.3 | 83.5 | 10.2 |
| 89B-6730-1 | 1918 | 50.3 | 27 | 3.5 | 1.1 | 83.5 | 9.9 |
| 89B-6739-2 | 2434 | 36.8 | 19 | 3.5 | 1.6 | 83.5 | 10.0 |
| 89B-6068-1 | 1337 | 47.4 | 119 | 3.6 | 1.1 | 83.4 | 10.5 |
| 89B-6098-1 | 1518 | 52.4 | 55 | 3.6 | 1.2 | 83.4 | 9.9 |
| 89B-6391-1 | 1598 | 50.3 | 78 | 3.5 | 1.2 | 83.4 | 10.4 |
| 89B-6438-1 | 1641 | 55.9 | 51 | 3.9 | 1.4 | 83.4 | 9.8 |
| 89B-6704-2 | 2415 | 51.2 | 37 | 3.5 | 1.3 | 83.4 | 10.1 |
| 89B-7427-1 | 2353 | 56.1 | 64 | 3.7 | 1.3 | 83.4 | 10.3 |
| 89B-6026-1 | 1297 | 51.6 | 108 | 3.4 | 1.2 | 83.3 | 10.5 |
| 89B-6127-4 | 1663 | 43.4 | 76 | 3.8 | 1.2 | 83.3 | 10.0 |
| 89B-6142-1 | 1380 | 50.1 | 401 | 3.9 | 1.4 | 83.3 | 9.9 |
| 89B-6621-1 | 1819 | 52.3 | 107 | 3.6 | 1.5 | 83.3 | 10.0 |
| 89B-6725-1 | 1913 | 50.0 | 132 | 3.4 | 1.6 | 83.3 | 10.1 |
| 89B-7037-1 | 1999 | 34.1 | 44 | 3.5 | | 83.3 | 11.3 |
| 89B-6136-1 | 1375 | 51.0 | 387 | 3.9 | 1.5 | 83.2 | 10.2 |
| 89B-6785-1 | 1966 | N/A | 5 | 4.3 | 1.6 | 83.2 | 9.0 |
| 89B-7439-1 | 2365 | 42.4 | 104 | 3.6 | 1.6 | 83.2 | 9.8 |
| 89B-6127-2 | 1661 | 43.4 | 76 | 3.6 | 1.3 | 83.1 | 10.4 |
| 89B-6129-1 | 1368 | 53.3 | 88 | 3.8 | 1.2 | 83.1 | 10.1 |
| 89B-6358-1 | 1571 | 51.8 | 85 | 3.4 | 1.1 | 83.1 | 10.6 |
| 89B-6482-1 | 1690 | 46.9 | 135 | 3.5 | 1.0 | 83.1 | 10.8 |
| 89B-6671-1 | 1861 | 49.2 | 25 | 3.8 | 2.0 | 83.1 | 9.7 |
| 89B-6691-1 | 1878 | 50.7 | 35 | 3.5 | 1.3 | 83.1 | 10.5 |
| 89B-6708-1 | 1897 | 49.9 | 87 | 3.6 | 1.2 | 83.1 | 10.1 |
| 89B-6763-1 | 1948 | 48.6 | 20 | 3.6 | 1.0 | 83.1 | 10.2 |
| 89B-6768-1 | 1953 | 50.1 | 69 | 3.7 | 1.0 | 83.1 | 10.3 |
| 89B-7432-1 | 2358 | 45.6 | 73 | 3.9 | 1.4 | 83.1 | 9.9 |
| 89B-6028-1 | 1304 | 53.9 | 135 | 3.4 | 1.2 | 83.0 | 11.3 |
| 89B-6029-1 | 1309 | 52.3 | 97 | 3.4 | 1.3 | 83.0 | 10.8 |
| 89B-6401-1 | 1606 | 48.1 | 26 | 4.0 | 1.5 | 83.0 | 9.8 |

| 1990 Montola-2000 Safflower Single Row Results | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 6720-4 | 3264 | | | 3.8 | 1.4 | 82.8 | 10.0 |
| 6700-5 | 3225 | | | 4.0 | 1.4 | 82.3 | 9.5 |
| 6340-4 | 2954 | | | 3.9 | 1.4 | 82.1 | 9.9 |
| 6380-1 | 1647 | | | 3.8 | 1.6 | 82.1 | 11.1 |
| 6380-1 | 3593 | | | 4.0 | 1.6 | 81.5 | 11.1 |
| 6720-5 | 3265 | | | 4.0 | 1.4 | 81.4 | 11.3 |
| 6340-3 | 2953 | | | 4.4 | 1.7 | 81.4 | 10.9 |
| 6720-3 | 3263 | | | 4.4 | 1.4 | 81.0 | 11.5 |
| 6720-1 | 2033 | | | 3.8 | 1.6 | 80.9 | 12.1 |
| 6340-1 | 1583 | | | 3.9 | 1.2 | 80.6 | 12.9 |
| 6700-1 | 2014 | | | 4.0 | 1.7 | 80.5 | 12.0 |
| 6380-4 | 2966 | | | 4.1 | 1.4 | 80.2 | 12.4 |
| 6380-3 | 2965 | | | 3.9 | 1.2 | 80.1 | 13.1 |
| 6720-2 | 3262 | | | 4.0 | 1.1 | 80.0 | 13.6 |
| 6700-4 | 3224 | | | 4.1 | 1.5 | 80.0 | 12.0 |
| 6900-1 | 2215 | | | 4.2 | 1.5 | 79.9 | 13.0 |
| 6740-1 | 2054 | | | 4.1 | 1.3 | 79.8 | 12.9 |
| 6020-1 | 1259 | | | 4.0 | 1.4 | 79.8 | 13.2 |
| 6300-1 | 1542 | | | 4.0 | 1.3 | 79.6 | 13.2 |
| 6500-1 | 1811 | | | 4.1 | 1.6 | 79.5 | 13.1 |
| 6140-1 | 1381 | | | 4.0 | 1.4 | 79.3 | 13.6 |
| 6340-2 | 2952 | | | 6.2 | 1.7 | 79.3 | 11.3 |
| 6580-1 | 1892 | | | 3.7 | 1.4 | 79.0 | 14.3 |
| | | | | | avg | 80.6 | |

| 1990 Oleic Safflower Single Row Results | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 6751-3 | 3339 | | | 3.5 | 1.2 | 85.7 | 7.3 |
| 6296-2 | 2927 | | | 3.0 | 1.2 | 85.5 | 8.2 |
| 6298-3 | 2936 | | | 3.2 | 1.2 | 85.3 | 7.9 |
| 6132-3 | 2815 | | | 3.3 | 1.0 | 85.2 | 8.5 |
| 6775-5 | 3393 | | | 3.3 | 1.2 | 85.1 | 8.4 |
| 6749.3 | 3331 | | | 3.5 | 1.5 | 85.1 | 7.8 |
| 6748-4 | 3328 | | | 3.2 | 1.3 | 85.1 | 7.4 |
| 6804-2 | 3459 | | | 3.4 | 1.3 | 84.9 | 8.5 |
| 6751-2 | 3338 | | | 3.6 | 1.1 | 84.9 | 10.4 |
| 6789-3 | 3427 | | | 3.4 | 1.2 | 84.8 | 8.9 |
| 6752-4 | 3344 | | | 3.3 | 1.5 | 84.8 | 8.0 |
| 6697-4 | 3220 | | | 3.2 | 1.1 | 84.8 | 8.8 |
| 6095-4 | 2759 | | | 3.3 | 1.2 | 84.7 | 8.5 |
| 6747-4 | 3324 | | | 3.5 | 1.2 | 84.5 | 8.6 |
| 6379-2 | 2960 | | | 3.5 | 1.0 | 84.5 | 8.7 |
| 6585-3 | 3124 | | | 3.5 | 1.1 | 84.5 | 8.8 |
| 6218-3 | 2871 | | | 3.7 | 1.2 | 84.5 | 9.0 |
| 6497-5 | 3049 | | | 3.4 | 1.7 | 84.5 | 8.5 |
| 6681-2 | 3189 | | | 3.5 | 1.3 | 84.4 | 8.4 |
| 6298-5 | 2938 | | | 3.5 | 1.3 | 84.4 | 8.4 |
| 6298-2 | 2935 | | | 3.6 | 1.1 | 84.4 | 8.8 |
| 6208-3 | 2847 | | | 3.6 | 1.4 | 84.4 | 8.4 |
| 6749-1 | 2063 | | | 3.4 | 1.2 | 84.4 | 9.2 |
| 6330-5 | 3150 | | | 3.4 | 1.6 | 84.4 | 8.5 |
| 6068-2 | 2709 | | | 3.5 | 1.0 | 84.4 | 8.6 |
| 6585-5 | 3126 | | | 3.4 | 1.3 | 84.4 | 9.4 |
| 6073-2 | 2717 | | | 3.5 | 0.9 | 84.3 | 9.3 |
| 6658-5 | 3171 | | | 3.5 | 1.3 | 84.3 | 9.1 |
| 6761-5 | 3369 | | | 3.3 | 1.0 | 84.3 | 9.3 |
| 6632-3 | 3157 | | | 3.8 | 1.4 | 84.3 | 9.2 |
| 6095-2 | 2757 | | | 3.5 | 1.1 | 84.3 | 9.1 |
| 6069.5 | 2716 | | | 3.4 | 1.1 | 84.3 | 9.0 |
| 6693-2 | 3127 | | | 3.6 | 1.0 | 84.3 | 8.9 |
| 6218-2 | 2870 | | | 3.4 | 1.4 | 84.3 | 9.1 |
| 6631-2 | 3152 | | | 3.5 | 1.5 | 84.2 | 9.2 |
| 6525-4 | 3080 | | | 3.5 | 1.2 | 84.2 | 9.3 |
| 6328-1 | 3589 | | | 3.9 | 1.4 | 84.2 | 9.1 |
| 6498-2 | 3050 | | | 3.6 | 1.3 | 84.2 | 9.4 |
| 6759-2 | 3362 | | | 3.2 | 1.3 | 84.2 | 9.3 |
| 6752-3 | 3343 | | | 3.3 | 1.5 | 84.2 | 9.1 |
| 6448-2 | 2981 | | | 3.4 | 1.2 | 84.1 | 9.0 |
| 6123-3 | 2795 | | | 3.6 | 1.3 | 84.1 | 9.1 |
| 6913-4 | 3523 | | | 3.8 | 1.4 | 84.0 | 8.9 |

| 1990 Oleic Safflower Single Row Results | | | | | | | |
|---|---|---|---|---|---|---|---|
| FIELD ENTRY | LAB SAMPLEOIL | OPEN SEED | % TOTAL OPEN SEED | % PALMITIC | % STEARIC | % OLEIC | % LINOLEIC |
| # | # | NMR | grams | 16:0 | 18:0 | 18:1 | 18:2 |
| 6757-5 | 3361 | | | 3.5 | 1.3 | 83.9 | 10.8 |
| 6078-5 | 2736 | | | 3.4 | 1.0 | 83.9 | 10.0 |
| 6747-1 | 2061 | | | 3.6 | 1.1 | 83.9 | 9.4 |
| 6656-3 | 3161 | | | 3.3 | 1.3 | 83.9 | 9.5 |
| 6209-4 | 2852 | | | 3.3 | 1.1 | 83.9 | 9.5 |
| 6299-3 | 2940 | | | 3.5 | 1.2 | 83.9 | 9.2 |
| 6632-4 | 3158 | | | 3.4 | 1.3 | 83.9 | 8.5 |
| 6096-4 | 2764 | | | 3.6 | 1.2 | 83.9 | 9.2 |
| 6252-1 | 3579 | | | 3.7 | 1.5 | 83.8 | 9.4 |
| 6301-1 | 3587 | | | 3.5 | 1.7 | 83.8 | 8.2 |
| 6658-3 | 3169 | | | 3.7 | 1.5 | 83.8 | 9.0 |
| 6236.1 | 3574 | | | 3.4 | 1.0 | 83.8 | 9.8 |
| 6069-4 | 2715 | | | 3.6 | 1.2 | 83.8 | 9.3 |
| 6235-4 | 2893 | | | 3.3 | 1.0 | 83.8 | 9.8 |
| 6130-4 | 2812 | | | 3.5 | 1.1 | 83.8 | 9.6 |
| 8111-1 | 1142 | | | 3.3 | 1.3 | 83.7 | 10.0 |
| 6267-3 | 2920 | | | 3.2 | 1.4 | 83.7 | 9.6 |
| 6097-4 | 2768 | | | 3.3 | 1.1 | 83.7 | 9.5 |
| 6766-3 | 3375 | | | 3.5 | 1.1 | 83.7 | 9.5 |
| 6249-2 | 2907 | | | 4.0 | 1.4 | 83.7 | 9.3 |
| 6301-3 | 2944 | | | 3.1 | 1.2 | 83.7 | 9.8 |
| 6379-5 | 2963 | | | 3.6 | 1.1 | 83.7 | 9.4 |
| 6749-2 | 3330 | | | 3.4 | 1.0 | 83.7 | 9.5 |
| 6703-3 | 3235 | | | 3.5 | 1.0 | 83.7 | 9.3 |
| 6879-5 | 3494 | | | 3.9 | 1.5 | 83.7 | 8.7 |
| 6585-2 | 3123 | | | 3.3 | 1.0 | 83.7 | 9.5 |
| 6883-3 | 3506 | | | 4.0 | 1.9 | 83.7 | 8.2 |
| 6065-2 | 2701 | | | 3.6 | 1.0 | 83.7 | 9.6 |
| 6790-3 | 3431 | | | 3.7 | 1.2 | 83.7 | 9.4 |
| 6497-2 | 3046 | | | 3.7 | 1.4 | 83.7 | 9.4 |
| 8091-1 | 1140 | | | 3.4 | 1.1 | 83.6 | 10.7 |
| 6065-4 | 2703 | | | 3.4 | 0.9 | 83.6 | 10.3 |
| 6753-4 | 3348 | | | 3.6 | 1.6 | 83.6 | 8.9 |
| 6753-2 | 3346 | | | 3.3 | 1.0 | 83.6 | 8.6 |
| 6068-3 | 2710 | | | 3.7 | 1.1 | 83.6 | 9.3 |
| 6750-4 | 3336 | | | 3.4 | 1.1 | 83.6 | 9.0 |
| 6221-4 | 2876 | | | 3.6 | 1.4 | 83.6 | 9.7 |
| 6499-1 | 3615 | | | 3.8 | 1.5 | 83.6 | 9.3 |
| 6244-5 | 2906 | | | 3.8 | 1.4 | 83.6 | 9.2 |
| 6915-2 | 3242 | | | 3.2 | 1.3 | 83.6 | 8.6 |
| 6545-1 | 1857 | | | 3.4 | 1.1 | 83.6 | 10.1 |
| 6789-2 | 3426 | | | 3.6 | 1.1 | 83.6 | 9.5 |
| 6073-3 | 2718 | | | 3.5 | 1.2 | 83.6 | 9.4 |
| 6076-4 | 2727 | | | 3.6 | 1.2 | 83.6 | 9.6 |
| 6585-4 | 3125 | | | 3.6 | 1.2 | 83.6 | 10.1 |
| 6299-1 | 3586 | | | 3.7 | 1.5 | 83.5 | 9.1 |
| 6748-5 | 3329 | | | 3.5 | 1.0 | 83.5 | 11.4 |

| 1990 Oleic Safflower Single Row Results | | | | | | | |
|---|---|---|---|---|---|---|---|
| FIELD ENTRY | LAB SAMPLE OIL | OPEN SEED % | TOTAL OPEN SEED | % PALMITIC | % STEARIC | % OLEIC | % LINOLEIC |
| # | # | NMR | grams | 16:0 | 18:0 | 18:1 | 18:2 |
| 6778-3 | 3403 | | | 3.3 | 1.0 | 83.5 | 9.9 |
| 6490-3 | 3043 | | | 3.9 | 1.6 | 83.5 | 9.5 |
| 6610-5 | 3134 | | | 3.3 | 1.1 | 83.5 | 10.1 |
| 6077-1 | 3533 | | | 3.8 | 1.2 | 83.5 | 9.8 |
| 6754-2 | 3350 | | | 3.5 | 2.1 | 83.5 | 8.2 |
| 6099-1 | 1339 | | | 3.4 | 1.1 | 83.5 | 10.2 |
| 6761-4 | 3368 | | | 3.5 | 1.3 | 83.5 | 9.6 |
| 6777-2 | 3398 | | | 3.4 | 1.1 | 83.5 | 10.3 |
| 6803-4 | 3457 | | | 3.7 | 1.3 | 83.5 | 9.9 |
| 6696-4 | 3216 | | | 3.4 | 1.0 | 83.5 | 9.9 |
| 6737-4 | 3296 | | | 3.8 | 1.4 | 83.4 | 9.6 |
| 6074-5 | 2724 | | | 3.5 | 1.0 | 83.4 | 10.0 |
| 6484-4 | 3028 | | | 3.6 | 1.3 | 83.4 | 10.1 |
| 6511-2 | 3062 | | | 3.3 | 1.1 | 83.4 | 10.2 |
| 6552-4 | 3100 | | | 4.1 | 1.1 | 83.4 | 9.9 |
| 6129-3 | 2807 | | | 3.6 | 1.1 | 83.4 | 9.9 |
| 6519.3 | 3075 | | | 3.7 | 1.4 | 83.4 | 10.2 |
| 6777-1 | 2090 | | | 3.4 | 1.1 | 83.4 | 10.6 |
| 6079-1 | 3535 | | | 3.5 | 1.0 | 83.4 | 10.0 |
| 6473-5 | 3012 | | | 3.8 | 1.8 | 83.4 | 9.2 |
| 6087-1 | 3538 | | | 3.6 | 1.4 | 83.4 | 9.6 |
| 6102-1 | 3544 | | | 3.4 | 1.1 | 83.4 | 9.6 |
| 6297-2 | 2931 | | | 3.6 | 1.5 | 83.4 | 9.4 |
| 6701-2 | 3226 | | | 3.5 | 1.3 | 83.4 | 9.1 |
| 6657-2 | 3164 | | | 3.8 | 1.3 | 83.3 | 9.7 |
| 6218-5 | 2873 | | | 3.5 | 1.2 | 83.3 | 10.5 |
| 6077-3 | 2730 | | | 3.7 | 1.2 | 83.3 | 9.8 |
| 6009-2 | 2697 | | | 3.6 | 1.1 | 83.3 | 10.5 |
| 6915-3 | 3243 | | | 3.3 | 1.2 | 83.3 | 9.3 |
| 6656-2 | 3160 | | | 3.9 | 1.5 | 83.3 | 8.7 |
| 8041-1 | 1135 | | | 3.4 | 1.2 | 83.2 | 10.5 |
| 6790-2 | 3430 | | | 3.8 | 1.2 | 83.2 | 10.1 |
| 6749-5 | 3333 | | | 3.3 | 1.3 | 83.2 | 7.2 |
| 6497-3 | 3047 | | | 3.7 | 1.4 | 83.2 | 9.6 |
| 6789-5 | 3429 | | | 3.7 | 1.2 | 83.2 | 9.0 |
| 6743-3 | 3311 | | | 3.4 | 1.5 | 83.2 | 9.0 |
| 6250-1 | 3578 | | | 4.0 | 1.4 | 83.2 | 9.6 |
| 6906-3 | 3514 | | | 3.9 | 1.4 | 83.2 | 9.8 |
| 6695-2 | 3210 | | | 3.6 | 1.2 | 83.2 | 9.9 |
| 6114-4 | 2784 | | | 3.7 | 1.5 | 83.2 | 9.2 |
| 6552-3 | 3099 | | | 4.1 | 1.3 | 83.2 | 9.7 |
| 6128-5 | 2805 | | | 3.4 | 1.0 | 83.2 | 10.5 |
| 6218-4 | 2872 | | | 3.7 | 1.5 | 83.2 | 9.5 |
| 6657-5 | 3167 | | | 3.6 | 1.2 | 83.2 | 9.9 |
| 6690-3 | 3199 | | | 3.7 | 1.4 | 83.2 | 9.3 |
| 6210-5 | 2857 | | | 3.7 | 1.3 | 83.2 | 10.1 |
| 6065-1 | 3526 | | | 3.7 | 1.2 | 83.2 | 10.3 |

| 1990 Oleic Safflower Single Row Results | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 6883-2 | 3505 | | | 4.0 | 1.5 | 83.2 | 8.6 |
| 6913-3 | 3522 | | | 3.8 | 1.1 | 83.2 | 10.6 |
| 6205-4 | 2844 | | | 3.7 | 1.2 | 83.1 | 9.6 |
| 6249-3 | 2908 | | | 4.2 | 1.3 | 83.1 | 9.5 |
| 6759-4 | 3364 | | | 3.4 | 1.3 | 83.1 | 10.8 |
| 6069-1 | 3529 | | | 3.5 | 1.2 | 83.1 | 10.0 |
| 6499-5 | 3057 | | | 3.7 | 1.4 | 83.1 | 9.8 |
| 6627-3 | 3140 | | | 3.6 | 1.2 | 83.1 | 9.7 |
| 6296-3 | 2928 | | | 3.2 | 1.5 | 83.1 | 7.8 |
| 6296-5 | 2930 | | | 3.1 | 1.1 | 83.1 | 10.4 |
| 6132-2 | 2814 | | | 3.5 | 1.0 | 83.1 | 10.4 |
| 6107-1 | 1347 | | | 3.4 | 1.1 | 83.1 | 10.3 |
| 6659-1 | 1973 | | | 3.3 | 1.8 | 83.1 | 10.1 |
| 6218-1 | 3569 | | | 3.5 | 1.3 | 83.1 | 10.1 |
| 6697-3 | 3219 | | | 3.4 | 1.2 | 83.1 | 9.6 |
| 6656-5 | 3163 | | | 3.7 | 1.6 | 83.1 | 9.4 |
| 6381-2 | 2968 | | | 3.8 | 1.2 | 83.1 | 9.8 |
| 6803-5 | 3458 | | | 3.7 | 1.2 | 83.1 | 10.2 |
| 6208-2 | 2846 | | | 3.6 | 1.3 | 83.1 | 9.7 |
| 6878-2 | 3487 | | | 4.0 | 1.4 | 83.1 | 10.8 |
| 6269-2 | 2923 | | | 3.3 | 1.4 | 83.0 | 10.1 |
| 6130-5 | 2813 | | | 3.4 | 1.0 | 83.0 | 10.5 |
| 6298-1 | 3585 | | | 3.6 | 1.1 | 83.0 | 9.8 |
| 6133-3 | 2819 | | | 3.5 | 1.0 | 83.0 | 10.4 |
| 6098-3 | 2771 | | | 3.6 | 1.1 | 83.0 | 10.1 |
| 6610-4 | 3133 | | | 3.3 | 1.2 | 83.0 | 10.5 |
| 6221-3 | 2875 | | | 3.7 | 1.5 | 83.0 | 10.0 |
| 6915-5 | 3245 | | | 3.5 | 1.5 | 83.0 | 9.3 |
| 6744.3 | 3315 | | | 3.9 | 1.0 | 83.0 | 10.1 |
| 6126-5 | 2801 | | | 3.6 | 1.0 | 83.0 | 10.5 |
| 6205-3 | 2843 | | | 3.7 | 1.2 | 83.0 | 10.0 |
| 6210-1 | 3565 | | | 3.3 | 1.0 | 83.0 | 9.2 |
| 6448-3 | 2982 | | | 3.4 | 1.0 | 83.0 | 10.3 |
| 6775-1 | 2088 | | | 3.5 | 1.1 | 83.0 | 10.7 |

| LINOLEIC 1990 INCREASE OIL QUALITY ANALYSES | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 90-LI-1154-1 | 1178 | | | 5.4 | 1.9 | 6.5 | 86.2 |
| 90-LI-1094-1 | 1166 | | | 5.5 | 1.8 | 6.2 | 85.9 |
| 90-LI-1079-1 | 1161 | | | 5.1 | 1.6 | 7.0 | 85.7 |
| 90-LI-1205-1 | 1188 | | | 5.3 | 1.7 | 6.6 | 85.7 |
| 90-LI-1152-1 | 1176 | | | 5.4 | 1.8 | 7.2 | 85.6 |
| 90-LI-1180-1 | 1183 | | | 5.4 | 2.0 | 7.1 | 85.6 |
| 90-LI-1060-1 | 1157 | | | 5.6 | 1.7 | 6.5 | 85.3 |
| 90-LI-1080-1 | 1162 | | | 5.4 | 1.9 | 7.4 | 85.3 |
| 90-LI-1156-1 | 1193 | | | 5.1 | 1.9 | 6.6 | 85.3 |
| 90-LI-1061-1 | 1158 | | | 5.2 | 1.7 | 6.9 | 85.2 |
| 90-LI-1117-1 | 1170 | | | 5.6 | 1.7 | 6.6 | 85.1 |
| 90-LI-1219-1 | 1191 | | | 5.6 | 1.8 | 7.4 | 85.1 |
| 90-LI-1162-1 | 1179 | | | 5.6 | 1.9 | 6.9 | 85.0 |
| 90-LI-1144-1 | 1175 | | | 5.6 | 1.9 | 7.6 | 85.0 |
| | | | | | | AVG | 85.4 |

| 1989 89b- Oil Quality Analyses TOP LINOLEIC | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-1607-3 | 4549 | 30.5 | 53 | 3.9 | 1.3 | 4.1 | 90.7 |
| 89B-1607-2 | 4548 | 30.5 | 53 | 3.7 | 1.3 | 4.4 | 90.6 |
| 89B-7557-1 | 2521 | 30.8 | 101 | 3.6 | 1.6 | 3.7 | 90.5 |
| 89B-1397-3 | 4109 | N/A | N/A | 4.0 | | 6.0 | 90.4 |
| 89B-1397-1 | 3679 | N/A | N/A | 3.9 | 1.4 | 4.5 | 90.2 |
| 89B-1604-4 | 4547 | N/A | 15 | 4.0 | 1.6 | 4.2 | 90.2 |
| 89B-7524-1 | 2490 | N/A | 8 | 3.6 | 1.4 | 4.2 | 90.2 |
| 89B-7557-5 | 2916 | 30.8 | 101 | 3.8 | 1.3 | 3.9 | 90.2 |
| 89B-1604-2 | 3934 | N/A | 15 | 3.8 | 1.5 | 4.5 | 90.1 |
| 89B-1607-5 | 4551 | 30.5 | 53 | 3.7 | 1.3 | 4.9 | 90.1 |
| 89B-1602-4 | 4545 | N/A | 06 | 4.1 | 1.5 | 4.4 | 90.0 |
| 89B-7527-1 | 2493 | N/A | 7 | 4.1 | 1.1 | 3.8 | 90.0 |
| 89B-7563-3 | 2921 | 41.2 | 62 | 4.0 | 1.1 | 4.1 | 90.0 |
| 89B-1608-1 | 3937 | 29.9 | 60 | 3.9 | 1.5 | 4.8 | 89.9 |
| 89B-1394-3 | 4065 | N/A | 12 | 3.6 | 1.6 | 4.9 | 89.8 |
| 89B-1578-4 | 4513 | N/A | 08 | 4.3 | 1.2 | 4.3 | 89.8 |
| 89B-1602-2 | 4543 | N/A | 06 | 4.0 | 1.5 | 4.8 | 89.7 |
| 89B-1607-4 | 4550 | 30.5 | 53 | 4.0 | 1.5 | 4.9 | 89.6 |
| 89B-1603-3 | 4553 | 29.9 | 60 | 3.8 | 1.5 | 5.1 | 89.6 |
| 89B-1610-2 | 3938 | N/A | 04 | 3.9 | 1.4 | 5.1 | 89.6 |
| 89B-7557-3 | 2914 | 30.8 | 101 | 3.7 | 1.5 | 3.4 | 89.5 |
| 89B-1394-5 | 4067 | N/A | 12 | 3.9 | 1.6 | 5.1 | 89.4 |
| 89B-1584-3 | 4517 | 38.5 | 411 | 4.5 | 1.4 | 5.0 | 89.4 |
| 89B-2097-1 | 4645 | 33.8 | 31 | 3.5 | 1.6 | 5.5 | 89.4 |
| 89B-2098-1 | 4646 | N/A | 8 | 4.4 | 1.2 | 5.0 | 89.4 |
| 89B-1394-2 | 4064 | N/A | 12 | 3.6 | 1.5 | 5.6 | 89.3 |
| 89B-1578-5 | 4514 | N/A | 08 | 4.4 | 1.2 | 4.6 | 89.3 |
| 89B-1610-4 | 4557 | N/A | 04 | 3.8 | 1.4 | 5.6 | 89.3 |
| 89B-7557-2 | 2913 | 30.8 | 101 | 3.7 | 1.6 | 4.3 | 89.3 |
| 89B-1397-4 | 4110 | N/A | N/A | 3.9 | 1.8 | 5.1 | 89.2 |
| 89B-1578-1 | 3913 | N/A | 08 | 4.5 | 1.4 | 4.9 | 89.2 |
| 89B-1608-2 | 4552 | 29.9 | 60 | 4.0 | 1.7 | 5.2 | 89.2 |
| 89B-7526-3 | 2898 | 29.8 | 72 | 4.1 | 1.5 | 4.1 | 89.1 |
| 89B-1608-4 | 4554 | 29.9 | 60 | 4.0 | 1.5 | 5.6 | 88.9 |
| 89B-1608-5 | 4555 | 29.9 | 60 | 4.1 | 1.2 | 5.8 | 88.9 |
| 89B-2091-1 | 4639 | 23.5 | 34 | 3.5 | 1.6 | 6.0 | 88.9 |
| 89B-7526-1 | 2492 | 29.8 | 72 | 4.1 | 1.5 | 4.8 | 88.9 |
| 89B-1602-3 | 4544 | N/A | 06 | 4.1 | 1.7 | 5.5 | 88.8 |
| 89B-1610-3 | 4556 | N/A | 04 | 4.1 | 1.6 | 5.6 | 88.8 |
| 89B-1607-1 | 3936 | 30.5 | 53 | 4.1 | 1.8 | 5.4 | 88.7 |
| 89B-2094-2 | 4642 | 34.5 | 22 | 3.8 | 1.8 | 5.8 | 88.7 |
| 89B-7557-4 | 2915 | 30.8 | 101 | 3.9 | 1.6 | 4.2 | 88.7 |
| 89B-1394-4 | 4066 | N/A | 12 | 3.8 | 1.7 | 6.0 | 88.6 |
| 89B-1397-2 | 4108 | N/A | N/A | 3.8 | 1.8 | 5.7 | 88.6 |
| 89B-7524-5 | 2896 | N/A | 8 | 4.3 | 1.4 | 5.2 | 88.4 |
| 89B-7526-2 | 2897 | 29.8 | 72 | 4.4 | 1.3 | 4.5 | 88.4 |
| 89B-1578-3 | 4512 | N/A | 08 | 4.5 | 1.3 | 4.6 | 88.3 |

| 1989 89b- Oil Quality Analyses TOP LINOLEIC | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-7527-2 | 2899 | N/A | 7 | 4.4 | 1.1 | 4.3 | 88.3 |
| 89B-7527-4 | 2900 | N/A | 7 | 4.8 | 1.1 | 3.7 | 88.3 |
| 89B-1209-2 | 4018 | 39.8 | 185 | 5.1 | 1.6 | 5.1 | 88.2 |
| 89B-1192-1 | 3498 | 45.8 | 121 | 5.2 | 1.7 | 5.1 | 88.1 |
| 89B-1394-1 | 3677 | N/A | 12 | 4.3 | 1.7 | 6.0 | 88.0 |
| 89B-7524-2 | 2893 | N/A | 8 | 4.4 | 1.3 | 5.3 | 87.8 |
| 89B-7524-3 | 2894 | N/A | 8 | 4.2 | 1.3 | 5.4 | 87.7 |
| 89B-894-4 | 3805 | N/A | N/A | 5.2 | 1.8 | 5.4 | 87.7 |
| 89B-1194-3 | 4000 | 46.4 | 54 | 4.9 | 1.6 | 5.8 | 87.6 |
| 89B-1612-2 | 4558 | 36.1 | 243 | 4.4 | 1.5 | 6.4 | 87.6 |
| 89B-1602-1 | 3933 | N/A | 06 | 4.6 | 1.5 | 6.4 | 87.5 |
| 89B-1857-1 | 4237 | 51.4 | 313 | 4.8 | 1.6 | 6.1 | 87.4 |
| 89B-7346-5 | 2768 | N/A | N/A | 5.2 | 1.5 | 4.3 | 87.4 |
| 89B-1209-5 | 4021 | 39.8 | 185 | 5.1 | 1.8 | 5.9 | 87.3 |
| 89B-1612-5 | 4561 | 36.1 | 243 | 4.3 | 1.6 | 5.5 | 87.3 |
| CENTENNIAL | 4022 | | | 5.2 | 1.5 | 6.0 | 87.3 |
| 89B-1210-1 | 3514 | 41.8 | 136 | 5.1 | 1.6 | 5.3 | 87.2 |
| 89B-1211-4 | 4029 | 42.9 | 159 | 4.9 | 1.8 | 6.1 | 87.1 |
| 89B-7346-4 | 2767 | N/A | N/A | 4.7 | 1.5 | 5.5 | 87.1 |
| 89B-1212-1 | 3516 | 39.1 | 164 | 5.4 | 1.4 | 5.4 | 87.0 |
| 89B-1592-1 | 3925 | 26.0 | 314 | 4.6 | 1.4 | 6.7 | 87.0 |
| 89B-894-3 | 3804 | N/A | N/A | 5.4 | 1.8 | 5.7 | 87.0 |
| 89B-1193-2 | 3995 | 45.0 | 162 | 5.0 | 1.7 | 5.8 | 86.9 |
| 89B-1204-3 | 4008 | 40.6 | 105 | 4.8 | 1.4 | 6.9 | 86.9 |
| 89B-1210-2 | 4023 | 41.8 | 136 | 5.2 | 1.7 | 6.2 | 86.9 |
| 89B-7563-1 | 2526 | 41.2 | 62 | 4.2 | 1.4 | 6.3 | 86.9 |
| 89B-1188-1 | 3495 | 45.9 | 178 | 4.7 | 1.3 | 7.2 | 86.8 |
| 89B-1193-5 | 3998 | 45.0 | 162 | 5.2 | 1.8 | 6.2 | 86.8 |
| 89B-1592-5 | 4530 | 26.0 | 314 | 4.7 | 1.3 | 6.7 | 86.8 |
| 89B-1193-1 | 3499 | 45.0 | 162 | 5.2 | 1.8 | 6.1 | 86.6 |
| 89B-1195-3 | 4004 | 43.5 | 42 | 4.9 | 1.8 | 6.7 | 86.6 |
| 89B-1592-2 | 4527 | 26.0 | 314 | 4.8 | 1.6 | 7.0 | 86.6 |
| 89B-1601-4 | 4541 | 32.5 | 231 | 4.3 | 1.5 | 7.6 | 86.6 |
| 89B-2102-1 | 4374 | 35.9 | 102 | 4.9 | 1.7 | 6.5 | 86.6 |
| 89B-2126-1 | 4396 | 36.3 | 260 | 4.6 | 1.7 | 6.5 | 86.6 |
| 89B-7477-1 | 2400 | 39.6 | 205 | 5.5 | 1.5 | 4.6 | 86.6 |
| 89B-1193-4 | 3997 | 45.0 | 162 | 5.2 | 1.7 | 6.6 | 86.5 |
| 89B-1195-1 | 3501 | 43.5 | 42 | 5.3 | 1.8 | 6.5 | 86.5 |
| 89B-1209-4 | 4020 | 39.8 | 185 | 5.2 | 1.6 | 6.4 | 86.5 |
| 89B-1211-2 | 4027 | 42.9 | 159 | 5.6 | 1.6 | 6.3 | 86.5 |
| 89B-1584-1 | 3917 | 38.5 | 411 | 4.9 | 1.6 | 6.9 | 86.5 |
| 89B-1612-3 | 4559 | 36.1 | 243 | 5.1 | 1.8 | 6.4 | 86.5 |
| 89B-789-3 | 1145 | 50.0 | 176 | 5.1 | 1.9 | 5.9 | 86.5 |
| 89B-1184-3 | 3978 | 46.8 | 203 | 5.3 | 1.7 | 6.6 | 86.4 |
| 89B-1195-4 | 4005 | 43.5 | 42 | 5.0 | 1.6 | 6.6 | 86.4 |
| 89B-1204-2 | 4007 | 40.6 | 105 | 5.2 | 1.6 | 6.3 | 86.4 |
| 89B-1211-3 | 4028 | 42.9 | 159 | 5.2 | | 8.4 | 86.4 |

| 1989 89b- Oil Quality Analyses TOP LINOLEIC | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-1214-1 | 3518 | 47.0 | 205 | 5.2 | 1.7 | 6.0 | 86.4 |
| 89B-1633-3 | 4578 | 38.4 | 260 | 4.7 | 1.7 | 6.1 | 86.4 |
| 89B-2029-1 | 4491 | 36.9 | 231 | 4.2 | 1.4 | 7.9 | 86.4 |
| 89B-895-1 | 3241 | N/A | N/A | 4.8 | 1.9 | 5.9 | 86.4 |
| 89B-1194-4 | 4001 | 46.4 | 54 | 5.3 | 2.0 | 6.5 | 86.3 |
| 89B-1194-5 | 4002 | 46.4 | 54 | 5.0 | 1.5 | 7.2 | 86.3 |
| 89B-1210-3 | 4024 | 41.8 | 136 | 5.2 | 1.7 | 6.8 | 86.3 |
| 89B-1212-4 | 4033 | 39.1 | 164 | 5.4 | 1.5 | 6.4 | 86.3 |
| 89B-1591-5 | 4526 | 29.0 | 206 | 4.5 | 1.3 | 7.4 | 86.3 |
| 89B-1026-2 | 3818 | 45.9 | 21 | 4.9 | 1.6 | 7.3 | 86.2 |
| 89B-1190-3 | 3988 | 46.6 | 115 | 4.9 | 1.6 | 7.2 | 86.2 |
| 89B-1190.4 | 3989 | 46.6 | 115 | 4.8 | 1.5 | 7.4 | 86.2 |
| 89B-1190-5 | 3990 | 46.6 | 115 | 4.9 | 1.4 | 7.5 | 86.2 |
| 89B-1192-4 | 3993 | 45.8 | 121 | 5.0 | 1.7 | 6.7 | 86.2 |
| 89B-1208-3 | 4015 | 40.8 | 175 | 5.2 | 1.5 | 6.3 | 86.2 |
| 89B-1601-5 | 4542 | 32.5 | 231 | 4.5 | 1.4 | 7.3 | 86.2 |
| 89B-2110-1 | 4382 | 29.6 | 149 | 4.7 | 1.7 | 6.2 | 86.2 |
| 89B-789-5 | 1147 | 50.0 | 176 | 5.3 | 1.9 | 5.8 | 86.2 |
| 89B-825-1 | 3179 | 52.5 | 224 | 5.1 | 1.4 | 7.3 | 86.2 |
| 89B-894-2 | 3803 | N/A | N/A | 5.0 | 1.6 | 7.1 | 86.2 |
| 89B-1192-3 | 3992 | 45.8 | 121 | 5.3 | 1.7 | 6.4 | 86.1 |
| 89B-1194-2 | 3999 | 46.4 | 54 | 5.5 | 1.6 | 6.4 | 86.1 |
| 89B-1195-5 | 4006 | 43.5 | 42 | 5.0 | 1.9 | 7.0 | 86.1 |
| 89B-1204-1 | 3509 | 40.6 | 105 | 5.4 | 1.7 | 6.8 | 86.1 |
| 89B-1205-1 | 3510 | 38.6 | 23 | 5.3 | 1.8 | 6.1 | 86.1 |
| 89B-1208-4 | 4016 | 40.8 | 175 | 5.2 | 2.0 | 6.8 | 86.1 |
| 89B-1211-5 | 4030 | 42.9 | 159 | 5.2 | 1.8 | 6.9 | 86.1 |
| 89B-1223-1 | 3525 | 48.5 | 139 | 5.1 | 1.6 | 7.3 | 86.1 |
| 89B-1316-1 | 3613 | 38.2 | 274 | 4.4 | 1.3 | 8.1 | 86.1 |
| 89B-1593-1 | 3926 | 45.0 | 320 | 5.0 | 1.4 | 7.1 | 86.1 |
| 89B-1612-4 | 4560 | 36.1 | 243 | 4.4 | 1.7 | 7.1 | 86.1 |
| 89B-2117-1 | 4389 | 43.6 | 94 | 4.8 | 1.6 | 6.5 | 86.1 |
| 89B-7141-1 | 2092 | 38.4 | 168 | 5.0 | 1.6 | 5.5 | 86.1 |
| 89B-894-1 | 3240 | N/A | N/A | 5.0 | 1.8 | 7.1 | 86.1 |
| 89B-895-2 | 3807 | N/A | N/A | 5.4 | 2.0 | 6.1 | 86.1 |
| 89B-1181-3 | 3970 | 42.2 | 264 | 4.8 | 1.6 | 7.6 | 86.0 |
| 89B-1195-2 | 4003 | 43.5 | 42 | 5.2 | 1.8 | 6.3 | 86.0 |
| 89B-1208-2 | 4014 | 40.8 | 175 | 5.3 | 1.7 | 7.0 | 86.0 |
| 89B-1212-3 | 4032 | 39.1 | 164 | 5.7 | 1.5 | 6.3 | 86.0 |
| 89B-1856-1 | 4236 | 48.4 | 325 | 4.8 | 1.5 | 6.6 | 86.0 |
| 89B-2108-1 | 4380 | 37.2 | 17 | 4.6 | 1.7 | 7.0 | 86.0 |
| 89B-2122-1 | 4392 | 43.3 | 105 | 5.3 | 1.7 | 7.0 | 86.0 |
| 89B-2127-1 | 4397 | 44.2 | 232 | 4.8 | 1.6 | 6.8 | 86.0 |
| 89B-2213-1 | 4659 | 42.8 | 88 | 4.4 | 1.5 | 8.1 | 86.0 |
| 89B-7311-4 | 2763 | 44.4 | 155 | 4.6 | 1.7 | 6.6 | 86.0 |
| 89B-7448-1 | 2373 | 46.3 | 146 | 4.4 | 2.0 | 5.9 | 86.0 |
| 89B-7475-5 | 2882 | 44.4 | 252 | 5.0 | 1.7 | 5.7 | 86.0 |

| 1989 89b- Oil Quality Analyses TOP LINOLEIC | | | | | | | |
|---|---|---|---|---|---|---|---|
| FIELD ENTRY | LAB SAMPLE | OPEN SEED OIL% | TOTAL OPEN SEED | PALMITIC % | % STEARIC | OLEIC % | % LINOLEIC |
| # | # | NMR | grams | 16:0 | 18:0 | 18:1 | 18:2 |
| 89B-1181-2 | 3969 | 42.2 | 264 | 4.9 | 1.9 | 7.3 | 85.9 |
| 89B-1184-2 | 3977 | 46.8 | 203 | 5.0 | 1.7 | 7.5 | 85.9 |
| 89B-1189-2 | 3985 | 38.1 | 40 | 4.8 | 1.4 | 7.2 | 85.9 |
| 89B-1190-1 | 3497 | 46.6 | 115 | 5.0 | 1.7 | 7.4 | 85.9 |
| 89B-1208-1 | 3512 | 40.8 | 175 | 5.6 | 1.7 | 6.0 | 85.9 |
| 89B-1214-2 | 4035 | 47.0 | 205 | 4.8 | 1.6 | 7.6 | 85.9 |
| 89B-1363-1 | 3654 | 40.1 | 70 | 4.8 | 1.2 | 8.1 | 85.9 |
| 89B-1590-3 | 4521 | 32.3 | 236 | 4.9 | 1.5 | 7.1 | 85.9 |
| 89B-1724-1 | 4152 | 39.3 | 359 | 4.4 | 1.5 | 8.3 | 85.9 |
| 89B-1903-1 | 4282 | 43.0 | 291 | 5.0 | 1.7 | 7.5 | 85.9 |
| 89B-2030-1 | 4492 | 38.2 | 366 | 5.0 | 1.6 | 7.1 | 85.9 |
| 89B-7346-2 | 2765 | N/A | N/A | 5.4 | 1.7 | 5.7 | 85.9 |
| 89B-7346-3 | 2766 | N/A | N/A | 5.0 | 1.8 | 6.2 | 85.9 |
| 89B-7475-2 | 2879 | 44.4 | 252 | 4.6 | 1.6 | 6.3 | 85.9 |
| 89B-934-3 | 1180 | 49.7 | 123 | 4.8 | 1.8 | 7.4 | 85.9 |
| 89B-1183-1 | 3973 | 42.0 | 267 | 5.2 | 1.9 | 6.5 | 85.8 |
| 89B-1184-4 | 3979 | 46.8 | 203 | 5.1 | 1.7 | 7.4 | 85.8 |
| 89B-1186-1 | 3493 | 47.1 | 230 | 4.9 | 1.8 | 7.5 | 85.8 |
| 89B-1209-3 | 4019 | 39.8 | 185 | 5.1 | 1.9 | 7.2 | 85.8 |
| 89B-1316-2 | 4043 | 38.2 | 274 | 4.6 | 1.4 | 8.2 | 85.8 |
| 89B-1593-3 | 4532 | 45.0 | 320 | 5.0 | 1.6 | 7.5 | 85.8 |
| 89B-1596-1 | 3929 | 39.3 | 228 | 5.0 | 1.7 | 7.6 | 85.8 |
| 89B-1601-1 | 3932 | 32.5 | 231 | 4.7 | 1.6 | 8.0 | 85.8 |
| 89B-1632-1 | 3957 | 38.7 | 238 | 4.9 | 1.9 | 7.4 | 85.8 |
| 89B-1636-1 | 4073 | 39.5 | 215 | 4.5 | 1.6 | 7.3 | 85.8 |
| 89B-2027-1 | 4489 | 30.0 | 250 | 4.5 | 1.5 | 6.7 | 85.8 |
| 89B-2103-1 | 4375 | 41.9 | 114 | 4.9 | 1.9 | 7.4 | 85.8 |
| 89B-2153-1 | 4422 | 43.8 | 153 | 4.8 | 1.9 | 7.5 | 85.8 |
| 89B-656-1 | 3043 | 44.1 | 237 | 5.3 | 1.6 | 7.2 | 85.8 |
| 89B-656-2 | 3836 | 44.1 | 237 | 4.9 | 1.4 | 8.0 | 85.8 |
| 89B-7311-1 | 2247 | 44.4 | 155 | 4.6 | 1.4 | 6.5 | 85.8 |
| 89B-7473-3 | 2876 | 45.4 | 193 | 5.0 | 1.6 | 6.0 | 85.8 |
| 89B-7477-5 | 2887 | 39.6 | 205 | 5.3 | 1.7 | 5.3 | 85.8 |
| 89B-7503-5 | 2892 | 31.3 | 215 | 4.7 | 1.6 | 6.8 | 85.8 |
| 89B-1026-3 | 3819 | 45.9 | 21 | 5.1 | 1.6 | 7.6 | 85.7 |
| 89B-1132-1 | 3447 | 46.3 | 155 | 4.9 | 1.9 | 7.0 | 85.7 |
| 89B-1181-1 | 3488 | 42.2 | 264 | 4.4 | 1.6 | 7.7 | 85.7 |
| 89B-1192-2 | 3991 | 45.8 | 121 | 5.2 | 1.7 | 6.6 | 85.7 |
| 89B-1212-2 | 4031 | 39.1 | 164 | 5.6 | 1.6 | 7.1 | 85.7 |
| 89B-1341-1 | 3633 | 37.5 | 89 | 6.1 | 1.5 | 6.7 | 85.7 |
| 89B-1517-1 | 3857 | 39.6 | 277 | 5.0 | 1.9 | 7.3 | 85.7 |
| 89B-1593-4 | 4533 | 45.0 | 320 | 4.6 | 1.9 | 7.4 | 85.7 |
| 89B-1602-5 | 4546 | N/A | 06 | 4.8 | 1.5 | 6.6 | 85.7 |
| 89B-1632-3 | 4574 | 38.7 | 238 | 4.2 | 2.0 | 6.6 | 85.7 |
| 89B-1635-3 | 4612 | 40.5 | 186 | 4.6 | 1.9 | 6.3 | 85.7 |
| 89B-1636-4 | 4617 | 39.5 | 215 | 4.6 | 1.6 | 6.8 | 85.7 |
| 89B-2107-1 | 4379 | 35.9 | 122 | 5.8 | 1.6 | 6.9 | 85.7 |

| 1989 89b- Oil Quality Analyses TOP LINOLEIC | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-2114-1 | 4386 | 42.1 | 171 | 4.9 | 1.4 | 8.0 | 85.7 |
| 89B-7475-1 | 2398 | 44.4 | 252 | 6.0 | 1.5 | 4.9 | 85.7 |
| 89B-7477-4 | 2885 | 39.6 | 205 | 5.4 | 1.5 | 5.7 | 85.7 |
| 89B-1072-4 | 3822 | 48.3 | 198 | 3.1 | 1.7 | 6.9 | 85.6 |
| 89B-1184-1 | 3491 | 46.8 | 203 | 5.0 | 1.6 | 7.8 | 85.6 |
| 89B-1186-4 | 3983 | 47.1 | 230 | 4.4 | 1.8 | 8.2 | 85.6 |
| 89B-1210-5 | 4026 | 41.8 | 135 | 5.2 | 1.7 | 6.8 | 85.6 |
| 89B-1488-1 | 3756 | 37.5 | 125 | 4.7 | 1.3 | 7.9 | 85.6 |
| 89B-1533-1 | 3872 | 38.3 | 112 | 4.8 | 1.4 | 8.1 | 85.6 |
| 89B-1592-4 | 4529 | 26.0 | 314 | 5.0 | 1.5 | 6.8 | 85.6 |
| 89B-1634-1 | 3959 | 37.8 | 195 | 4.6 | 1.9 | 8.0 | 85.6 |
| 89B-2002-1 | 4466 | 42.1 | 59 | 4.8 | 2.0 | 7.6 | 85.6 |
| 89B-609-2 | 1084 | 46.6 | 181 | 3.3 | 1.4 | 7.5 | 85.6 |
| 89B-609-3 | 1085 | 46.6 | 181 | 5.3 | 1.5 | 6.9 | 85.6 |
| 89B-7139-2 | 2572 | 35.3 | 117 | 4.8 | 1.8 | 6.3 | 85.6 |
| 89B-7477-3 | 2884 | 39.6 | 205 | 5.5 | 1.5 | 5.6 | 85.6 |
| 89B-7587-1 | 2548 | 39.5 | 51 | 4.7 | 1.8 | 6.3 | 85.6 |
| 89B-1183-4 | 3975 | 42.0 | 267 | 5.1 | 1.6 | 7.9 | 85.5 |
| 89B-1205-3 | 4012 | 38.6 | 23 | 5.8 | 1.7 | 6.9 | 85.5 |
| 89B-1209-1 | 3513 | 39.8 | 185 | 5.0 | 1.6 | 6.7 | 85.5 |
| 89B-1316-3 | 4044 | 38.2 | 274 | 4.7 | 1.5 | 8.3 | 85.5 |
| 89B-1341-3 | 4049 | 37.5 | 89 | 6.0 | 1.4 | 7.1 | 85.5 |
| 89B-1522-1 | 3861 | 39.4 | 94 | 5.4 | 1.4 | 7.3 | 85.5 |
| 89B-1591-1 | 3924 | 29.0 | 206 | 5.1 | 1.8 | 7.6 | 85.5 |
| 89B-1637-1 | 4074 | 38.6 | 169 | 4.8 | 1.9 | 7.8 | 85.5 |
| 89B-1637-2 | 4619 | 38.6 | 169 | 4.3 | 1.8 | 6.9 | 85.5 |
| 89B-607-5 | 1077 | 46.2 | 217 | 5.1 | 1.7 | 7.6 | 85.5 |
| 89B-7167-2 | 2580 | 43.8 | 300 | 4.7 | 1.9 | 6.5 | 85.5 |
| 89B-7346-1 | 2280 | N/A | N/A | 5.2 | 1.8 | 5.8 | 85.5 |
| 89B-7534-1 | 2499 | 38.9 | 148 | 4.6 | 2.0 | 6.7 | 85.5 |
| 89B-7536-1 | 2501 | 46.3 | 223 | 5.0 | 1.5 | 6.8 | 85.5 |
| 89B-789-4 | 1146 | 50.0 | 176 | 5.6 | 1.9 | 6.4 | 85.5 |
| 89B-885-1 | 3231 | 49.0 | 101 | 5.2 | 1.5 | 7.8 | 85.5 |
| 89B-1072-3 | 3821 | 48.3 | 198 | 5.1 | 1.6 | 7.4 | 85.4 |
| 89B-1132-3 | 3962 | 46.3 | 155 | 4.9 | 1.8 | 6.7 | 85.4 |
| 89B-1186-3 | 3982 | 47.1 | 230 | 4.9 | 1.7 | 8.1 | 85.4 |
| 89B-1189-1 | 3496 | 38.1 | 40 | 4.9 | 1.8 | 7.9 | 85.4 |
| 89B-1190-2 | 3987 | 46.6 | 115 | 4.9 | 1.7 | 8.1 | 85.4 |
| 89B-1204-5 | 4010 | 40.6 | 105 | 5.9 | 1.4 | 6.9 | 85.4 |
| 89B-1205-4 | 4013 | 38.6 | 23 | 5.3 | 1.9 | 6.9 | 85.4 |
| 89B-1214-3 | 4036 | 47.0 | 205 | 4.9 | 1.6 | 8.1 | 85.4 |
| 89B-1363-2 | 4056 | 40.1 | 70 | 5.2 | 1.4 | 8.0 | 85.4 |
| 89B-1637-3 | 4620 | 38.6 | 169 | 4.5 | 1.7 | 6.7 | 85.4 |
| 89B-1922-1 | 4298 | 43.2 | 238 | 4.2 | 1.4 | 8.4 | 85.4 |
| 89B-1948-1 | 4324 | 42.0 | 270 | 4.7 | 1.5 | 8.1 | 85.4 |
| 89B-2141-1 | 4409 | 41.9 | 184 | 4.9 | 1.9 | 7.8 | 85.4 |
| 89B-7139-1 | 2091 | 35.3 | 117 | 4.8 | 1.9 | 6.2 | 85.4 |

| 1989 89b- Oil Quality Analyses TOP LINOLEIC | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-7192-2 | 2588 | 43.4 | 167 | 5.0 | 1.4 | 6.6 | 85.4 |
| 89B-7394-2 | 2868 | 49.4 | 54 | 5.0 | 1.6 | 6.7 | 85.4 |
| 89B-7462-1 | 2386 | 40.8 | 295 | 4.8 | 1.6 | 6.5 | 85.4 |
| 89B-7563-2 | 2920 | 41.2 | 62 | 4.2 | 1.6 | 7.2 | 85.4 |
| 89B-7613-1 | 2570 | 44.3 | 44 | 4.3 | 1.7 | 7.1 | 85.4 |
| 89B-774-4 | 1112 | 49.3 | 190 | 5.3 | 1.6 | 7.7 | 85.4 |
| 89B-851-1 | 3200 | 44.9 | 91 | 5.8 | 1.3 | 7.5 | 85.4 |
| 89B-909-4 | 1176 | 43.4 | 287 | 5.5 | 1.5 | 7.4 | 85.4 |
| 89B-917-1 | 3258 | 48.3 | 302 | 5.3 | 1.5 | 7.8 | 85.4 |
| 89B-917-4 | 3814 | 48.3 | 302 | 5.4 | 1.8 | 7.4 | 85.4 |
| 89B-1026-1 | 3351 | 45.9 | 21 | 4.7 | 1.4 | 7.1 | 85.3 |
| 89B-1072-2 | 3820 | 48.3 | 198 | 5.5 | 1.8 | 7.4 | 85.3 |
| 89B-1183-5 | 3976 | 42.0 | 267 | 5.3 | 1.4 | 7.5 | 85.3 |
| 89B-1192-5 | 3994 | 45.8 | 121 | 5.5 | 1.5 | 7.6 | 85.3 |
| 89B-1590-1 | 3923 | 32.3 | 236 | 5.3 | 1.3 | 8.1 | 85.3 |
| 89B-1601-3 | 4540 | 32.5 | 231 | 4.7 | 1.4 | 8.6 | 85.3 |
| 89B-1612.1 | 3939 | 36.1 | 243 | 5.1 | 1.8 | 7.7 | 85.3 |
| 89B-1623-2 | 3948 | 43.7 | 122 | 5.1 | 1.7 | 7.9 | 85.3 |
| 89B-1633-4 | 4579 | 38.4 | 260 | 4.6 | 1.9 | 7.0 | 85.3 |
| 89B-2106-1 | 4378 | 40.3 | 64 | 5.3 | 1.6 | 6.9 | 85.3 |
| 89B-2112-1 | 4384 | 30.3 | 266 | 4.8 | 1.8 | 8.1 | 85.3 |
| 89B-2116-1 | 4388 | 43.8 | 119 | 4.6 | 1.8 | 7.8 | 85.3 |
| 89B-2125-1 | 4395 | 31.9 | 200 | 5.0 | 1.5 | 7.7 | 85.3 |
| 89B-625-1 | 3013 | 45.1 | 291 | 5.4 | 1.4 | 7.6 | 85.3 |
| 89B-633-1 | 3021 | 45.9 | 255 | 5.4 | 1.2 | 7.6 | 85.3 |
| 89B-633-4 | 3830 | 45.9 | 255 | 5.6 | 1.4 | 7.7 | 85.3 |
| 89B-644-1 | 3030 | 44.1 | 288 | 5.4 | 1.6 | 7.7 | 85.3 |
| 89B-656-4 | 3838 | 44.1 | 237 | 4.8 | 1.3 | 8.1 | 85.3 |
| 89B-7139-3 | 2573 | 35.3 | 117 | 5.1 | 1.6 | 6.7 | 85.3 |
| 89B-7394-1 | 2324 | 49.4 | 54 | 4.9 | 1.6 | 6.4 | 85.3 |
| 89B-7447-1 | 2372 | 37.6 | 262 | 4.7 | 1.9 | 6.6 | 85.3 |
| 89B-7448-5 | 2863 | 46.3 | 146 | 4.6 | 1.9 | 6.8 | 85.3 |
| 89B-1193-3 | 3996 | 45.0 | 162 | 5.4 | 1.8 | 7.2 | 85.2 |
| 89B-1211-1 | 3515 | 42.9 | 159 | 5.2 | 1.4 | 5.2 | 85.2 |
| 89B-1212-5 | 4034 | 39.1 | 164 | 5.6 | 1.8 | 6.9 | 85.2 |
| 89B-1351-1 | 3643 | 49.5 | 271 | 5.4 | 2.0 | 7.3 | 85.2 |
| 89B-1364-1 | 3655 | 45.8 | 50 | 4.3 | 1.6 | 8.9 | 85.2 |
| 89B-1503-1 | 3843 | 41.4 | 311 | 4.6 | 1.7 | 8.4 | 85.2 |
| 89B-1632-2 | 4573 | 38.7 | 238 | 4.4 | 1.8 | 7.0 | 85.2 |
| 89B-1635-1 | 4072 | 40.5 | 186 | 4.9 | 1.9 | 7.0 | 85.2 |
| 89B-1877-1 | 4256 | 47.3 | 60 | 5.2 | 1.6 | 7.5 | 85.2 |
| 89B-1884-1 | 4265 | 43.0 | 18 | 5.4 | 1.4 | 7.9 | 85.2 |
| 89B-1952-1 | 4328 | 40.0 | 261 | 4.6 | 1.5 | 8.3 | 85.2 |
| 89B-2096-1 | 4644 | 37.1 | 113 | 4.5 | 2.0 | 7.8 | 85.2 |
| 89B-2128-1 | 4398 | 44.9 | 118 | 5.6 | 1.6 | 7.0 | 85.2 |
| 89B-590-2 | 1069 | 46.4 | 158 | 5.3 | 1.6 | 7.4 | 85.2 |
| 89B-7081-2 | 2461 | 41.1 | 274 | 4.6 | 1.7 | 7.5 | 85.2 |

| 1989 89b- Oil Quality Analyses TOP LINOLEIC | | | | | | | |
|---|---|---|---|---|---|---|---|
| FIELD ENTRY | LAB SAMPLE | OPEN SEED OIL % | TOTAL OPEN SEED | PALMITIC % | % STEARIC | OLEIC % | % LINOLEIC |
| # | # | NMR | grams | 16:0 | 18:0 | 18:1 | 18:2 |
| 89B-7503-1 | 2471 | 31.3 | 215 | 4.2 | 1.4 | 7.9 | 85.2 |
| 89B-826-1 | 3180 | 52.8 | 137 | 5.1 | 1.7 | 8.0 | 85.2 |
| 89B-889-1 | 3235 | 50.2 | 220 | 4.9 | 1.5 | 8.4 | 85.2 |
| 89B-890-1 | 3236 | 46.3 | 255 | 5.3 | 1.6 | 7.5 | 85.2 |
| 89B-894-5 | 3806 | N/A | N/A | 5.2 | 1.9 | 6.2 | 85.2 |
| 89B-907-2 | 1164 | 48.5 | 231 | 5.4 | 1.9 | 6.7 | 85.2 |
| 89B-1163-1 | 3471 | 47.3 | 91 | 5.2 | 1.8 | 7.3 | 85.1 |
| 89B-1412-1 | 3687 | 42.8 | 108 | 4.9 | 1.6 | 8.3 | 85.1 |
| 89B-1488-4 | 4264 | 37.5 | 125 | 5.4 | 1.6 | 7.9 | 85.1 |
| 89B-1593-5 | 4534 | 45.0 | 320 | 4.9 | 1.7 | 7.5 | 85.1 |
| 89B-1624-1 | 3949 | 39.5 | 292 | 5.8 | 1.6 | 7.5 | 85.1 |
| 89B-1633-1 | 3958 | 38.4 | 260 | 4.7 | 1.9 | 7.7 | 85.1 |
| 89B-2011-1 | 4475 | 39.8 | 130 | 5.2 | 1.5 | 8.2 | 85.1 |
| 89B-2101-1 | 4373 | 39.2 | 249 | 5.4 | 1.5 | 7.1 | 85.1 |
| 89B-2123-1 | 4393 | 35.0 | 111 | 5.4 | 1.3 | 8.2 | 85.1 |
| 89B-608-2 | 1079 | 46.2 | 137 | 5.1 | 1.5 | 7.7 | 85.1 |
| 89B-633-3 | 3829 | 45.9 | 255 | 5.1 | 1.6 | 8.3 | 85.1 |
| 89B-7123-1 | 2077 | 36.4 | 25 | 4.7 | 1.6 | 7.1 | 85.1 |
| 89B-7188-4 | 2132 | 47.6 | 255 | 5.2 | 2.3 | 5.7 | 85.1 |
| 89B-7311-3 | 2762 | 44.4 | 155 | 4.5 | 1.6 | 7.6 | 85.1 |
| 89B-7558-1 | 2522 | 35.9 | 36 | 4.4 | 1.6 | 7.5 | 85.1 |
| 89B-890-5 | 3802 | 46.3 | 255 | 5.1 | 1.6 | 7.7 | 85.1 |
| 89B-1072-1 | 3393 | 48.3 | 198 | 5.4 | 1.5 | 7.2 | 85.0 |
| 89B-1183-2 | 3490 | 42.0 | 267 | 4.9 | 1.7 | 7.9 | 85.0 |
| 89B-1186-5 | 3984 | 47.1 | 230 | 4.7 | 1.8 | 8.5 | 85.0 |
| 89B-1214-4 | 4037 | 47.0 | 205 | 5.3 | 1.7 | 7.6 | 85.0 |
| 89B-1316-5 | 4046 | 38.2 | 274 | 5.2 | 1.5 | 8.4 | 85.0 |
| 89B-1589-1 | 3922 | 40.3 | 120 | 6.6 | 1.6 | 6.8 | 85.0 |
| 898-1613-1 | 3940 | 39.5 | 207 | 4.7 | 1.6 | 8.6 | 85.0 |
| 89B-1635-5 | 4614 | 40.5 | 186 | 4.9 | 1.9 | 7.1 | 85.0 |
| 89B-1675-1 | 4107 | 46.4 | 200 | 5.5 | 1.8 | 7.3 | 85.0 |
| 89B-1885-1 | 4266 | 39.2 | 374 | 5.2 | 1.8 | 7.5 | 85.0 |
| 89B-1945-1 | 4321 | 46.1 | 299 | 4.8 | 1.7 | 8.6 | 85.0 |
| 89B-1957-1 | 4333 | 49.0 | 300 | 5.0 | 2.0 | 7.8 | 85.0 |
| 89B-2028-1 | 4490 | 36.3 | 216 | 4.7 | 1.8 | 7.7 | 85.0 |
| 89B-2092-1 | 4640 | 41.7 | 26 | 4.8 | 1.8 | 7.6 | 85.0 |
| 89B-2124-1 | 4394 | 44.2 | 74 | 4.3 | 1.6 | 7.6 | 85.0 |
| 89B-2145-1 | 4414 | 45.0 | 84 | 4.8 | 1.9 | 8.3 | 85.0 |
| 89B-7192-1 | 2135 | 43.4 | 167 | 5.1 | 1.5 | 7.3 | 85.0 |
| 89B-7299-1 | 2237 | 34.3 | 58 | 4.6 | 1.8 | 6.9 | 85.0 |
| 89B-7473-1 | 2396 | 45.4 | 193 | 5.0 | 1.9 | 6.3 | 85.0 |
| 89B-7498-1 | 2457 | 38.3 | 271 | 5.2 | 1.5 | 6.8 | 85.0 |
| 89B-7498-1 | 2886 | 38.3 | 271 | 5.2 | 1.5 | 6.8 | 85.0 |
| 89B-7501-2 | 2459 | 35.7 | 150 | 4.8 | 1.8 | 7.4 | 85.0 |
| 89B-834-1 | 3188 | 43.3 | 149 | 5.2 | 1.6 | 8.3 | 85.0 |
| 89B-863-1 | 3211 | 44.8 | 74 | 5.9 | 1.9 | 7.3 | 85.0 |
| 89B-966-1 | 3298 | 47.7 | 23 | 5.4 | 2.1 | 7.5 | 85.0 |

### PROCESSING

The safflower seeds of the present invention may be processed commercially by being ground to a typical size for oil processing. The ground-seed composition is then cooked in a sealed vessel for 1 hour at 130°C. Extraction is then performed in a Butt-extractor, using commercial grade hexane as the solvent for a period of 4 hours. The hexane treated ground composition is then reground and returned to the extractor for an additional 4 hours wherein a second extraction step is conducted using the aforementioned conditions. The solvent is then removed from the miscella, first by distillation to a pot temperature of 75°C and finally into a roto-vac apparatus under reduced pressure of 1.32 KPa (10mm mercury pressure).

The resulting crude extracted oils were each treated with sufficient 16° Baume sodium hydroxide solution to neutralize the free fatty acids plus an additional 1.8% of the sodium hydroxide based on the total oil weight for 5 minutes at 65°C under moderate agitation. The heavy soap phase was separated by centrifugation. Residual soap and impurity removal is accomplished by washing with approximately 15% by volume of water for 5 minutes at 90°C, followed by bleaching the clear oil obtained by centrifugation with 1% by weight acid activated earth at 1.3 to 2.6 KPa (10-20mm) and 95°C for 10 minutes. The activated earth is separated by suction filtration.

The AOM determinations may be conducted pursuant to the American Oil Chemists Society (AOCS) method CD12-57. The AOM content of the high oleic safflower oils described herein is at least about 60 hours, preferably at least 70 hours and most preferably greater than 80 hours in the absence of an antioxidant. If antioxidants are used the AOM values are even greater.

Safflower oil may be identified by a unique sterol. Safflower plants are believed to contain delta-7 stigmasterol which is not believed to be found in other plants. The safflower plants of the present invention are also unique in their ability to fix nitrogen from fertilizer below the level of other plant species. Thus the safflower may be planted on a previously fertilized field and make use of nitrates at lower soil levels. Not only is the fertilizer effectively used but nitrate contamination of ground water is reduced.

While the invention has been disclosed in this patent application by reference to the details of preferred embodiments of the invention, it is to be understood that this disclosure is intended in an illustrative rather than in a limiting sense. The scope of the invention in defined by the appended claims.

## Claims

1. A safflower seed or oil obtained therefrom, said seed or oil containing from 3.7 to 6.2 wt.% saturated fatty acid content, and further containing an oleic acid content of at least 81.7%.

2. A safflower seed or oil according to claim 1, which contains 3.9 to 6.2% of saturated fatty acids.

3. A safflower seed or oil according to claim 1, which contains 4.7 to 5.8% of saturated fatty acids.

4. A safflower seed or oil of claim 1, wherein the oleic acid content of the oil ranges from 83.4% to 90%.

5. A safflower seed or oil according to claim 1, wherein the ratio of the amount of saturated fatty acid in said oil to the amount of oleic acid is less than 0.08.

6. A safflower seed or oil of claim 1, wherein the oleic acid content is at least 81.9 %.

7. A safflower seed or oil of claim 1, wherein the ratio of saturated fatty acids to oleic acid is less than about 0.07.

8. A safflower oil according to claim 1, which has been deodorized.

9. A deodorized safflower oil of claim 1, which is bleached.

10. A safflower oil according to claim 1, wherein the safflower oil has an AOM value of greater than about 60 hours.

11. A safflower oil according to claim 1, said oil containing an oleic acid content of 83.4% to 86.1%, and a linoleic acid content of 7.4% to 10.4%.

12. A safflower plant which produces a seed according to any preceding claim.

## Patentansprüche

1. Saflorsamen oder daraus erhaltenes Öl, wobei dieser Samen oder dieses Öl 3,7 bis 6,2 Gew.-% gesättigte Fettsäure und außerdem einen Ölsäuregehalt von wenigstens 81,7 % aufweisen.

2. Saflorsamen oder Öl nach Anspruch 1, welche 3,9 bis 6,2 % gesättigte Fettsäuren enthalten.

3. Saflorsamen oder Öl nach Anspruch 1, welche 4,7 bis 5,8 % gesättigte Fettsäuren enthalten.

4. Saflorsamen oder Öl nach Anspruch 1, worin der Ölsäuregehalt des Öls im Bereich von 83,4 % bis 90 % liegt.

5. Saflorsamen oder Öl nach Anspruch 1, worin das Verhältnis der Menge an gesättigter Fettsäure in dem Öl zu der Menge an Ölsäure weniger als 0,08 ist.

6. Saflorsamen oder Öl nach Anspruch 1, worin der Ölsäuregehalt mindestens 81,9 % beträgt.

7. Saflorsamen oder Öl nach Anspruch 1, worin das Verhältnis von gesättigten Fettsäuren zu Ölsäure geringer als etwa 0,07 ist.

8. Safloröl nach Anspruch 1, welches deodoriert wurde.

9. Deodoriertes Safloröl nach Anspruch 1, welches gebleicht wurde.

10. Safloröl nach Anspruch 1, welches einen AOM-Wert größer als etwa 60 Stunden hat.

11. Safloröl nach Anspruch 1, wobei das Öl einen Ölsäuregehalt von 83,4 % bis 86,1 % und einen Linolsäuregehalt von 7,4 % bis 10,4 % hat.

12. Saflorpflanze, die einen Samen gemäß einem der vorausgehenden Ansprüche erzeugt.

## Revendications

1. Graine de carthame ou huile qui en est retirée, ladite graine ou huile ayant une teneur en acides gras saturés de 3,7 à 6,2 % en poids et ayant de plus une teneur en acide oléique d'au moins 81,7 %.

2. Graine ou huile de carthame selon la revendication 1, qui contient 3,9 à 6,2 % d'acides gras saturés.

3. Graine ou huile de carthame selon la revendication 1, qui contient 4,7 à 5,8 % d'acides gras saturés.

4. Graine ou huile de carthame selon la revendication 1, dans laquelle la teneur en acide oléique de l'huile est comprise entre 83,4 % et 90 %.

5. Graine ou huile de carthame selon la revendication 1, dans laquelle le rapport de la quantité d'acide gras saturé contenu dans ladite huile à la quantité d'acide oléique est inférieur à 0,08.

6. Graine ou huile de carthame selon la revendication 1, dans laquelle la teneur en acide oléique est d'au moins 81,9 %.

7. Graine ou huile de carthame selon la revendication 1, dans laquelle le rapport des acides gras saturés à l'acide oléique est inférieur à environ 0,07.

8. Huile de carthame selon la revendication 1, qui a été désodorisée.

9. Huile de carthame désodorisée selon la revendication 1, qui est blanchie.

10. Huile de carthame selon la revendication 1, dans laquelle l'huile de carthame a un indice AOM supérieur à environ 60 heures.

11. Huile de carthame selon la revendication 1, ladite huile ayant une teneur en acide oléique de 83,4 % à 86,1 % et une teneur en acide linoléique de 7,4 % à 10,4 %.

12. Plante de carthame qui produit une graine selon l'une quelconque des revendications précédentes.
